(19)

**Europäisches Patentamt**
**European Patent Office**
**Office européen des brevets**

(11) **EP 3 925 971 A1**

(12) **EUROPEAN PATENT APPLICATION**

(43) Date of publication:
**22.12.2021 Bulletin 2021/51**

(51) Int Cl.:
**C07K 14/705** (2006.01)     **C07K 14/005** (2006.01)
**C12N 9/48** (2006.01)     **C12Q 1/70** (2006.01)
**G01N 33/569** (2006.01)

(21) Application number: **20180359.0**

(22) Date of filing: **16.06.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Viravaxx AG**
**1090 Wien (AT)**

(72) Inventors:
• **VALENTA, Rudolf**
  **2604 Theresienfeld (AT)**
• **GATTINGER, Pia**
  **1120 Vienna (AT)**
• **BOROCHOVA, Kristina**
  **1160 Vienna (AT)**
• **DOROFEEVA, Yulia**
  **1050 Vienna (AT)**
• **HENNING, Rainer**
  **3738 Uetliburg (CH)**
• **KISS, Renate**
  **1090 Vienna (AT)**
• **KRATZER, Bernhard**
  **8244 Schäffern (AT)**
• **TULAEVA, Inna**
  **1170 Vienna (AT)**
• **PICKL, Winfried**
  **1080 Vienna (AT)**

(74) Representative: **Redl, Gerda et al**
**REDL Life Science Patent Attorneys**
**Donau-City-Straße 11**
**1220 Wien (AT)**

(54) **METHOD FOR IDENTIFYING COMPOUNDS INFLUENCING VIRUS RECEPTOR BINDING**

(57)     The present invention relates to a method for identifying a compound enhancing, not affecting or inhibiting the binding of a virus to a receptor protein of said virus comprising the steps of

a) incubating a first solid support comprising said receptor protein or a functional fragment thereof immobilized thereon with said virus or a fragment thereof;

b) incubating a second solid support comprising said receptor protein or functional fragment thereof immobilized thereon with said virus or fragment thereof in the presence of said compound;

c) determining the virus-receptor binding levels resulting from step a) and step b);

d) comparing the binding levels determined in step c), and

e) identifying said compound as a compound enhancing virus-receptor binding if the binding level resulting from step b) is higher than the binding level resulting from step a),

f) identifying said compound as a compound not affecting virus-receptor binding if the binding level resulting from step b) is substantially identical to the binding level resulting from step a), and

g) identifying said compound as a compound inhibiting virus-receptor binding if the binding level resulting from step b) is lower than the binding level resulting from step a).

SEQUENCE LISTING

<110> Viravaxx AG

<120> METHOD FOR IDENTIFYING COMPOUNDS INFLUENCING VIRUS RECEPTOR
BINDING

<130> 27092-EP

<160> 32

<170> PatentIn version 3.5

<210> 1
<211> 30
<212> PRT
<213> Artificial Sequence

<220>
<223> Fragment of SARS-CoV-2 S1 protein

<400> 1

Pro Leu Val Ser Ser Gln Cys Val Asn Leu Thr Thr Arg Thr Gln Leu
1               5                   10                  15


Pro Pro Ala Tyr Thr Asn Ser Phe Thr Arg Gly Val Tyr Tyr
            20                  25                  30


<210> 2
<211> 30
<212> PRT
<213> Artificial Sequence

<220>
<223> Fragment of SARS-CoV-2 S1 protein

<400> 2

Arg Gly Val Tyr Tyr Pro Asp Lys Val Phe Arg Ser Ser Val Leu His
1               5                   10                  15


Ser Thr Gln Asp Leu Phe Leu Pro Phe Phe Ser Asn Val Thr
            20                  25                  30


<210> 3
<211> 30
<212> PRT
<213> Artificial Sequence

<220>
<223> Fragment of SARS-CoV-2 S1 protein

<400> 3

Phe Ser Asn Val Thr Trp Phe His Ala Ile His Val Ser Gly Thr Asn
1               5                   10                  15

```
Gly Thr Lys Arg Phe Asp Asn Pro Val Leu Pro Phe Asn Asp
                20                  25                  30
```

<210> 4
<211> 30
<212> PRT
<213> Artificial Sequence

<220>
<223> Fragment of SARS-CoV-2 S1 protein

<400> 4

```
Thr Leu Asp Ser Lys Thr Gln Ser Leu Leu Ile Val Asn Asn Ala Thr
1               5                   10                  15


Asn Val Val Ile Lys Val Cys Glu Phe Gln Phe Cys Asn Asp
                20                  25                  30
```

<210> 5
<211> 30
<212> PRT
<213> Artificial Sequence

<220>
<223> Fragment of SARS-CoV-2 S1 protein

<400> 5

```
Gln Phe Cys Asn Asp Pro Phe Leu Gly Val Tyr Tyr His Lys Asn Asn
1               5                   10                  15


Lys Ser Trp Met Glu Ser Glu Phe Arg Val Tyr Ser Ser Ala
                20                  25                  30
```

<210> 6
<211> 30
<212> PRT
<213> Artificial Sequence

<220>
<223> Fragment of SARS-CoV-2 S1 protein

<400> 6

```
Val Tyr Ser Ser Ala Asn Asn Cys Thr Phe Glu Tyr Val Ser Gln Pro
1               5                   10                  15


Phe Leu Met Asp Leu Glu Gly Lys Gln Gly Asn Phe Lys Asn
                20                  25                  30
```

<210> 7
<211> 30
<212> PRT
<213> Artificial Sequence

16

```
<220>
<223>   Fragment of SARS-CoV-2 S1 protein

<400>   7

Gly Asn Phe Lys Asn Leu Arg Glu Phe Val Phe Lys Asn Ile Asp Gly
1               5                   10                  15


Tyr Phe Lys Ile Tyr Ser Lys His Thr Pro Ile Asn Leu Val
            20                  25                  30


<210>   8
<211>   30
<212>   PRT
<213>   Artificial Sequence

<220>
<223>   Fragment of SARS-CoV-2 S1 protein

<400>   8

Asn Ile Thr Arg Phe Gln Thr Leu Leu Ala Leu His Arg Ser Tyr Leu
1               5                   10                  15


Thr Pro Gly Asp Ser Ser Ser Gly Trp Thr Ala Gly Ala Ala
            20                  25                  30


<210>   9
<211>   30
<212>   PRT
<213>   Artificial Sequence

<220>
<223>   Fragment of SARS-CoV-2 S1 protein

<400>   9

Thr Ala Gly Ala Ala Ala Tyr Tyr Val Gly Tyr Leu Gln Pro Arg Thr
1               5                   10                  15


Phe Leu Leu Lys Tyr Asn Glu Asn Gly Thr Ile Thr Asp Ala
            20                  25                  30


<210>   10
<211>   30
<212>   PRT
<213>   Artificial Sequence

<220>
<223>   Fragment of SARS-CoV-2 S1 protein

<400>   10

Thr Ile Thr Asp Ala Val Asp Cys Ala Leu Asp Pro Leu Ser Glu Thr
1               5                   10                  15
```

Lys Cys Thr Leu Lys Ser Phe Thr Val Glu Lys Gly Ile Tyr
                20                  25                  30


<210> 11
<211> 28
<212> PRT
<213> Artificial Sequence

<220>
<223> Fragment of SARS-CoV-2 S1 protein

<400> 11

Glu Lys Gly Ile Tyr Gln Thr Ser Asn Phe Arg Val Gln Pro Thr Glu
1               5                   10                  15


Ser Ile Val Arg Phe Pro Asn Ile Thr Asn Leu Cys
                20                  25


<210> 12
<211> 25
<212> PRT
<213> Artificial Sequence

<220>
<223> Fragment of SARS-CoV-2 S1 protein

<400> 12

Pro Thr Glu Ser Ile Val Arg Phe Pro Asn Ile Thr Asn Leu Cys Pro
1               5                   10                  15


Phe Gly Glu Val Phe Asn Ala Thr Arg
                20                  25


<210> 13
<211> 25
<212> PRT
<213> Artificial Sequence

<220>
<223> Fragment of SARS-CoV-2 S1 protein

<400> 13

Phe Asn Ala Thr Arg Phe Ala Ser Val Tyr Ala Trp Asn Arg Lys Arg
1               5                   10                  15


Ile Ser Asn Cys Val Ala Asp Tyr Ser
                20                  25


<210> 14
<211> 25
<212> PRT
<213> Artificial Sequence

```
<220>
<223>   Fragment of SARS-CoV-2 S1 protein

<400>   14

Val Ala Asp Tyr Ser Val Leu Tyr Asn Ser Ala Ser Phe Ser Thr Phe
1               5                   10                  15


Lys Cys Tyr Gly Val Ser Pro Thr Lys
            20                  25


<210>   15
<211>   30
<212>   PRT
<213>   Artificial Sequence

<220>
<223>   Fragment of SARS-CoV-2 S1 protein

<400>   15

Val Ser Pro Thr Lys Leu Asn Asp Leu Cys Phe Thr Asn Val Tyr Ala
1               5                   10                  15


Asp Ser Phe Val Ile Arg Gly Asp Glu Val Arg Gln Ile Ala
            20                  25                  30


<210>   16
<211>   30
<212>   PRT
<213>   Artificial Sequence

<220>
<223>   Fragment of SARS-CoV-2 S1 protein

<400>   16

Val Arg Gln Ile Ala Pro Gly Gln Thr Gly Lys Ile Ala Asp Tyr Asn
1               5                   10                  15


Tyr Lys Leu Pro Asp Asp Phe Thr Gly Cys Val Ile Ala Trp
            20                  25                  30


<210>   17
<211>   30
<212>   PRT
<213>   Artificial Sequence

<220>
<223>   Fragment of SARS-CoV-2 S1 protein

<400>   17

Cys Val Ile Ala Trp Asn Ser Asn Asn Leu Asp Ser Lys Val Gly Gly
1               5                   10                  15
```

Asn Tyr Asn Tyr Leu Tyr Arg Leu Phe Arg Lys Ser Asn Leu
                20                      25                      30


<210> 18
<211> 25
<212> PRT
<213> Artificial Sequence

<220>
<223> Fragment of SARS-CoV-2 S1 protein

<400> 18

Asp Ser Lys Val Gly Gly Asn Tyr Asn Tyr Leu Tyr Arg Leu Phe Arg
1               5                   10                  15


Lys Ser Asn Leu Lys Pro Phe Glu Arg
                20                  25


<210> 19
<211> 25
<212> PRT
<213> Artificial Sequence

<220>
<223> Fragment of SARS-CoV-2 S1 protein

<400> 19

Lys Pro Phe Glu Arg Asp Ile Ser Thr Glu Ile Tyr Gln Ala Gly Ser
1               5                   10                  15


Thr Pro Cys Asn Gly Val Glu Gly Phe
                20                  25


<210> 20
<211> 30
<212> PRT
<213> Artificial Sequence

<220>
<223> Fragment of SARS-CoV-2 S1 protein

<400> 20

Gly Val Glu Gly Phe Asn Cys Tyr Phe Pro Leu Gln Ser Tyr Gly Phe
1               5                   10                  15


Gln Pro Thr Asn Gly Val Gly Tyr Gln Pro Tyr Arg Val Val
                20                      25                      30


<210> 21
<211> 30
<212> PRT
<213> Artificial Sequence

<220>
<223>    Fragment of SARS-CoV-2 S1 protein

<400>    21

Pro Tyr Arg Val Val Val Leu Ser Phe Glu Leu Leu His Ala Pro Ala
1               5                   10                  15

Thr Val Cys Gly Pro Lys Lys Ser Thr Asn Leu Val Lys Asn
            20                  25                  30


<210>    22
<211>    28
<212>    PRT
<213>    Artificial Sequence

<220>
<223>    Fragment of SARS-CoV-2 S1 protein

<400>    22

Pro Phe Gln Gln Phe Gly Arg Asp Ile Ala Asp Thr Thr Asp Ala Val
1               5                   10                  15

Arg Asp Pro Gln Thr Leu Glu Ile Leu Asp Ile Thr
            20                  25


<210>    23
<211>    30
<212>    PRT
<213>    Artificial Sequence

<220>
<223>    Fragment of SARS-CoV-2 S1 protein

<400>    23

Ala Val Leu Tyr Gln Asp Val Asn Cys Thr Glu Val Pro Val Ala Ile
1               5                   10                  15

His Ala Asp Gln Leu Thr Pro Thr Trp Arg Val Tyr Ser Thr
            20                  25                  30


<210>    24
<211>    30
<212>    PRT
<213>    Artificial Sequence

<220>
<223>    Fragment of SARS-CoV-2 S1 protein

<400>    24

Arg Val Tyr Ser Thr Gly Ser Asn Val Phe Gln Thr Arg Ala Gly Cys
1               5                   10                  15

```
Leu Ile Gly Ala Glu His Val Asn Asn Ser Tyr Glu Cys Asp
            20                  25                  30
```

<210> 25
<211> 30
<212> PRT
<213> Artificial Sequence

<220>
<223> Fragment of SARS-CoV-2 S1 protein

<400> 25

```
Ser Tyr Glu Cys Asp Ile Pro Ile Gly Ala Gly Ile Cys Ala Ser Tyr
1                   5                   10                  15


Gln Thr Gln Thr Asn Ser Pro Arg Arg Ala Arg Ser Val Ala
            20                  25                  30
```

<210> 26
<211> 684
<212> PRT
<213> SARS-CoV-2

<400> 26

```
Met Phe Val Phe Leu Val Leu Leu Pro Leu Val Ser Ser Gln Cys Val
1                   5                   10                  15


Asn Leu Thr Thr Arg Thr Gln Leu Pro Pro Ala Tyr Thr Asn Ser Phe
            20                  25                  30


Thr Arg Gly Val Tyr Tyr Pro Asp Lys Val Phe Arg Ser Ser Val Leu
            35                  40                  45


His Ser Thr Gln Asp Leu Phe Leu Pro Phe Phe Ser Asn Val Thr Trp
        50                  55                  60


Phe His Ala Ile His Val Ser Gly Thr Asn Gly Thr Lys Arg Phe Asp
65                  70                  75                  80


Asn Pro Val Leu Pro Phe Asn Asp Gly Val Tyr Phe Ala Ser Thr Glu
                85                  90                  95


Lys Ser Asn Ile Ile Arg Gly Trp Ile Phe Gly Thr Thr Leu Asp Ser
            100                 105                 110


Lys Thr Gln Ser Leu Leu Ile Val Asn Asn Ala Thr Asn Val Val Ile
            115                 120                 125


Lys Val Cys Glu Phe Gln Phe Cys Asn Asp Pro Phe Leu Gly Val Tyr
            130                 135                 140
```

Tyr His Lys Asn Asn Lys Ser Trp Met Glu Ser Glu Phe Arg Val Tyr
145                     150                 155                 160

Ser Ser Ala Asn Asn Cys Thr Phe Glu Tyr Val Ser Gln Pro Phe Leu
                    165                 170                 175

Met Asp Leu Glu Gly Lys Gln Gly Asn Phe Lys Asn Leu Arg Glu Phe
            180                 185                 190

Val Phe Lys Asn Ile Asp Gly Tyr Lys Ile Tyr Ser Lys His Thr Pro
        195                 200                 205

Ile Asn Leu Val Arg Asp Leu Pro Gln Gly Phe Ser Ala Leu Glu Pro
        210                 215                 220

Leu Val Asp Leu Pro Ile Gly Ile Asn Ile Thr Arg Phe Gln Thr Leu
225                 230                 235                 240

Leu Ala Leu His Arg Ser Tyr Leu Thr Pro Gly Asp Ser Ser Ser Gly
                245                 250                 255

Trp Thr Ala Gly Ala Ala Ala Tyr Tyr Val Gly Tyr Leu Pro Arg Thr
                260                 265                 270

Phe Leu Leu Lys Tyr Asn Glu Asn Gly Thr Ile Thr Asp Ala Val Asp
            275                 280                 285

Cys Ala Leu Asp Pro Leu Ser Glu Thr Lys Cys Thr Leu Lys Ser Phe
        290                 295                 300

Thr Val Glu Lys Gly Ile Tyr Gln Thr Ser Asn Phe Arg Val Gln Pro
305                 310                 315                 320

Thr Glu Ser Ile Val Arg Phe Pro Asn Ile Thr Asn Leu Cys Pro Phe
                325                 330                 335

Gly Glu Phe Asn Ala Thr Arg Phe Ala Ser Val Tyr Ala Trp Asn Arg
            340                 345                 350

Lys Arg Ile Ser Asn Cys Val Ala Asp Tyr Ser Val Leu Tyr Asn Ser
        355                 360                 365

Ala Ser Phe Ser Thr Phe Lys Cys Tyr Gly Val Ser Pro Thr Lys Leu
        370                 375                 380

Asn Asp Leu Cys Phe Thr Asn Val Tyr Ala Asp Ser Phe Val Ile Arg

385                390                395                400

Gly Asp Glu Val Arg Gln Ile Pro Gly Gln Thr Gly Lys Ile Ala Asp
                405             410             415

Tyr Asn Tyr Lys Leu Pro Asp Asp Phe Thr Gly Cys Val Ile Ala Trp
                420             425             430

Asn Ser Asn Asn Leu Asp Ser Lys Val Gly Gly Asn Tyr Asn Tyr Leu
                435             440             445

Tyr Arg Leu Phe Arg Lys Ser Asn Leu Lys Pro Phe Glu Arg Asp Ile
    450             455             460

Ser Thr Glu Ile Tyr Gln Ala Gly Ser Thr Pro Cys Asn Gly Val Glu
465             470             475             480

Gly Phe Asn Cys Tyr Phe Pro Leu Gln Ser Tyr Gly Phe Gln Pro Thr
                485             490             495

Asn Gly Val Gly Tyr Gln Pro Tyr Arg Val Val Val Leu Ser Phe Glu
                500             505             510

Leu Leu His Ala Pro Ala Thr Val Cys Gly Pro Lys Lys Ser Thr Asn
                515             520             525

Leu Val Lys Asn Lys Cys Val Asn Phe Asn Phe Asn Gly Leu Thr Gly
                530             535             540

Thr Gly Val Leu Thr Glu Ser Asn Lys Lys Phe Leu Pro Phe Gln Gln
545             550             555             560

Phe Gly Arg Asp Ile Ala Asp Thr Thr Asp Ala Val Arg Asp Pro Gln
                565             570             575

Thr Leu Glu Ile Leu Asp Ile Thr Pro Cys Ser Phe Gly Gly Val Ser
                580             585             590

Val Ile Thr Pro Gly Thr Asn Thr Ser Asn Gln Val Ala Val Leu Tyr
                595             600             605

Gln Asp Val Asn Cys Thr Glu Val Pro Val Ala Ile His Ala Asp Gln
    610             615             620

Leu Thr Pro Thr Trp Arg Val Tyr Ser Thr Gly Ser Asn Val Phe Gln
625             630             635             640

```
Thr Arg Ala Gly Cys Leu Ile Gly Ala Glu His Val Asn Asn Ser Tyr
            645             650             655

Glu Cys Asp Ile Pro Ile Gly Ala Gly Ile Cys Ala Ser Tyr Gln Thr
            660             665             670

Gln Thr Asn Ser Pro Arg Arg Ala Arg Ser Val Ala
            675             680
```

<210> 27
<211> 1273
<212> PRT
<213> SARS-CoV-2

<400> 27

```
Met Phe Val Phe Leu Val Leu Leu Pro Leu Val Ser Ser Gln Cys Val
1               5               10              15

Asn Leu Thr Thr Arg Thr Gln Leu Pro Pro Ala Tyr Thr Asn Ser Phe
            20              25              30

Thr Arg Gly Val Tyr Tyr Pro Asp Lys Val Phe Arg Ser Ser Val Leu
            35              40              45

His Ser Thr Gln Asp Leu Phe Leu Pro Phe Phe Ser Asn Val Thr Trp
        50              55              60

Phe His Ala Ile His Val Ser Gly Thr Asn Gly Thr Lys Arg Phe Asp
65              70              75              80

Asn Pro Val Leu Pro Phe Asn Asp Gly Val Tyr Phe Ala Ser Thr Glu
            85              90              95

Lys Ser Asn Ile Ile Arg Gly Trp Ile Phe Gly Thr Thr Leu Asp Ser
            100             105             110

Lys Thr Gln Ser Leu Leu Ile Val Asn Asn Ala Thr Asn Val Val Ile
        115             120             125

Lys Val Cys Glu Phe Gln Phe Cys Asn Asp Pro Phe Leu Gly Val Tyr
        130             135             140

Tyr His Lys Asn Asn Lys Ser Trp Met Glu Ser Glu Phe Arg Val Tyr
145             150             155             160

Ser Ser Ala Asn Asn Cys Thr Phe Glu Tyr Val Ser Gln Pro Phe Leu
            165             170             175
```

```
Met Asp Leu Glu Gly Lys Gln Gly Asn Phe Lys Asn Leu Arg Glu Phe
        180                 185                 190

Val Phe Lys Asn Ile Asp Gly Tyr Phe Lys Ile Tyr Ser Lys His Thr
        195                 200                 205

Pro Ile Asn Leu Val Arg Asp Leu Pro Gln Gly Phe Ser Ala Leu Glu
        210                 215                 220

Pro Leu Val Asp Leu Pro Ile Gly Ile Asn Ile Thr Arg Phe Gln Thr
225                 230                 235                 240

Leu Leu Ala Leu His Arg Ser Tyr Leu Thr Pro Gly Asp Ser Ser Ser
                245                 250                 255

Gly Trp Thr Ala Gly Ala Ala Ala Tyr Tyr Val Gly Tyr Leu Gln Pro
                260                 265                 270

Arg Thr Phe Leu Leu Lys Tyr Asn Glu Asn Gly Thr Ile Thr Asp Ala
        275                 280                 285

Val Asp Cys Ala Leu Asp Pro Leu Ser Glu Thr Lys Cys Thr Leu Lys
        290                 295                 300

Ser Phe Thr Val Glu Lys Gly Ile Tyr Gln Thr Ser Asn Phe Arg Val
305                 310                 315                 320

Gln Pro Thr Glu Ser Ile Val Arg Phe Pro Asn Ile Thr Asn Leu Cys
                325                 330                 335

Pro Phe Gly Glu Val Phe Asn Ala Thr Arg Phe Ala Ser Val Tyr Ala
                340                 345                 350

Trp Asn Arg Lys Arg Ile Ser Asn Cys Val Ala Asp Tyr Ser Val Leu
        355                 360                 365

Tyr Asn Ser Ala Ser Phe Ser Thr Phe Lys Cys Tyr Gly Val Ser Pro
        370                 375                 380

Thr Lys Leu Asn Asp Leu Cys Phe Thr Asn Val Tyr Ala Asp Ser Phe
385                 390                 395                 400

Val Ile Arg Gly Asp Glu Val Arg Gln Ile Ala Pro Gly Gln Thr Gly
                405                 410                 415

Lys Ile Ala Asp Tyr Asn Tyr Lys Leu Pro Asp Asp Phe Thr Gly Cys
                420                 425                 430
```

```
Val Ile Ala Trp Asn Ser Asn Asn Leu Asp Ser Lys Val Gly Gly Asn
        435                 440                 445

Tyr Asn Tyr Leu Tyr Arg Leu Phe Arg Lys Ser Asn Leu Lys Pro Phe
        450                 455                 460

Glu Arg Asp Ile Ser Thr Glu Ile Tyr Gln Ala Gly Ser Thr Pro Cys
465                 470                 475                 480

Asn Gly Val Glu Gly Phe Asn Cys Tyr Phe Pro Leu Gln Ser Tyr Gly
                485                 490                 495

Phe Gln Pro Thr Asn Gly Val Gly Tyr Gln Pro Tyr Arg Val Val Val
            500                 505                 510

Leu Ser Phe Glu Leu Leu His Ala Pro Ala Thr Val Cys Gly Pro Lys
        515                 520                 525

Lys Ser Thr Asn Leu Val Lys Asn Lys Cys Val Asn Phe Asn Phe Asn
        530                 535                 540

Gly Leu Thr Gly Thr Gly Val Leu Thr Glu Ser Asn Lys Lys Phe Leu
545                 550                 555                 560

Pro Phe Gln Gln Phe Gly Arg Asp Ile Ala Asp Thr Thr Asp Ala Val
            565                 570                 575

Arg Asp Pro Gln Thr Leu Glu Ile Leu Asp Ile Thr Pro Cys Ser Phe
            580                 585                 590

Gly Gly Val Ser Val Ile Thr Pro Gly Thr Asn Thr Ser Asn Gln Val
            595                 600                 605

Ala Val Leu Tyr Gln Asp Val Asn Cys Thr Glu Val Pro Val Ala Ile
        610                 615                 620

His Ala Asp Gln Leu Thr Pro Thr Trp Arg Val Tyr Ser Thr Gly Ser
625                 630                 635                 640

Asn Val Phe Gln Thr Arg Ala Gly Cys Leu Ile Gly Ala Glu His Val
            645                 650                 655

Asn Asn Ser Tyr Glu Cys Asp Ile Pro Ile Gly Ala Gly Ile Cys Ala
        660                 665                 670

Ser Tyr Gln Thr Gln Thr Asn Ser Pro Arg Arg Ala Arg Ser Val Ala
        675                 680                 685
```

Ser Gln Ser Ile Ile Ala Tyr Thr Met Ser Leu Gly Ala Glu Asn Ser
    690             695             700

Val Ala Tyr Ser Asn Asn Ser Ile Ala Ile Pro Thr Asn Phe Thr Ile
705             710             715                 720

Ser Val Thr Thr Glu Ile Leu Pro Val Ser Met Thr Lys Thr Ser Val
            725             730             735

Asp Cys Thr Met Tyr Ile Cys Gly Asp Ser Thr Glu Cys Ser Asn Leu
        740             745             750

Leu Leu Gln Tyr Gly Ser Phe Cys Thr Gln Leu Asn Arg Ala Leu Thr
        755             760             765

Gly Ile Ala Val Glu Gln Asp Lys Asn Thr Gln Glu Val Phe Ala Gln
    770             775             780

Val Lys Gln Ile Tyr Lys Thr Pro Pro Ile Lys Asp Phe Gly Gly Phe
785             790             795                 800

Asn Phe Ser Gln Ile Leu Pro Asp Pro Ser Lys Pro Ser Lys Arg Ser
            805             810             815

Phe Ile Glu Asp Leu Leu Phe Asn Lys Val Thr Leu Ala Asp Ala Gly
        820             825             830

Phe Ile Lys Gln Tyr Gly Asp Cys Leu Gly Asp Ile Ala Ala Arg Asp
        835             840             845

Leu Ile Cys Ala Gln Lys Phe Asn Gly Leu Thr Val Leu Pro Pro Leu
    850             855             860

Leu Thr Asp Glu Met Ile Ala Gln Tyr Thr Ser Ala Leu Leu Ala Gly
865             870             875                 880

Thr Ile Thr Ser Gly Trp Thr Phe Gly Ala Gly Ala Ala Leu Gln Ile
            885             890             895

Pro Phe Ala Met Gln Met Ala Tyr Arg Phe Asn Gly Ile Gly Val Thr
        900             905             910

Gln Asn Val Leu Tyr Glu Asn Gln Lys Leu Ile Ala Asn Gln Phe Asn
        915             920             925

Ser Ala Ile Gly Lys Ile Gln Asp Ser Leu Ser Ser Thr Ala Ser Ala

                              930                    935                        940


Leu Gly Lys Leu Gln Asp Val Val Asn Gln Asn Ala Gln Ala Leu Asn
945                    950                    955                        960


Thr Leu Val Lys Gln Leu Ser Ser Asn Phe Gly Ala Ile Ser Ser Val
                965                    970                    975


Leu Asn Asp Ile Leu Ser Arg Leu Asp Lys Val Glu Ala Glu Val Gln
            980                    985                    990


Ile Asp Arg Leu Ile Thr Gly Arg  Leu Gln Ser Leu Gln  Thr Tyr Val
        995                    1000                    1005


Thr Gln  Gln Leu Ile Arg Ala  Ala Glu Ile Arg Ala  Ser Ala Asn
    1010                    1015                    1020


Leu Ala  Ala Thr Lys Met Ser  Glu Cys Val Leu Gly  Gln Ser Lys
    1025                    1030                    1035


Arg Val  Asp Phe Cys Gly Lys  Gly Tyr His Leu Met  Ser Phe Pro
    1040                    1045                    1050


Gln Ser  Ala Pro His Gly Val  Val Phe Leu His Val  Thr Tyr Val
    1055                    1060                    1065


Pro Ala  Gln Glu Lys Asn Phe  Thr Thr Ala Pro Ala  Ile Cys His
    1070                    1075                    1080


Asp Gly  Lys Ala His Phe Pro  Arg Glu Gly Val Phe  Val Ser Asn
    1085                    1090                    1095


Gly Thr  His Trp Phe Val Thr  Gln Arg Asn Phe Tyr  Glu Pro Gln
    1100                    1105                    1110


Ile Ile  Thr Thr Asp Asn Thr  Phe Val Ser Gly Asn  Cys Asp Val
    1115                    1120                    1125


Val Ile  Gly Ile Val Asn Asn  Thr Val Tyr Asp Pro  Leu Gln Pro
    1130                    1135                    1140


Glu Leu  Asp Ser Phe Lys Glu  Glu Leu Asp Lys Tyr  Phe Lys Asn
    1145                    1150                    1155


His Thr  Ser Pro Asp Val Asp  Leu Gly Asp Ile Ser  Gly Ile Asn
    1160                    1165                    1170

```
Ala Ser  Val Val Asn Ile Gln  Lys Glu Ile Asp Arg  Leu Asn Glu
    1175                  1180              1185


Val Ala  Lys Asn Leu Asn Glu  Ser Leu Ile Asp Leu  Gln Glu Leu
    1190                  1195              1200


Gly Lys  Tyr Glu Gln Tyr Ile  Lys Trp Pro Trp Tyr  Ile Trp Leu
    1205                  1210              1215


Gly Phe  Ile Ala Gly Leu Ile  Ala Ile Val Met Val  Thr Ile Met
    1220                  1225              1230


Leu Cys  Cys Met Thr Ser Cys  Cys Ser Cys Leu Lys  Gly Cys Cys
    1235                  1240              1245


Ser Cys  Gly Ser Cys Cys Lys  Phe Asp Glu Asp Asp  Ser Glu Pro
    1250                  1255              1260


Val Leu  Lys Gly Val Lys Leu  His Tyr Thr
    1265                  1270
```

```
<210>  28
<211>  1255
<212>  PRT
<213>  SARS-CoV

<400>  28
```

```
Met Phe Ile Phe Leu Leu Phe Leu Thr Leu Thr Ser Gly Ser Asp Leu
1               5               10              15


Asp Arg Cys Thr Thr Phe Asp Asp Val Gln Ala Pro Asn Tyr Thr Gln
        20              25              30


His Thr Ser Ser Met Arg Gly Val Tyr Tyr Pro Asp Glu Ile Phe Arg
        35              40              45


Ser Asp Thr Leu Tyr Leu Thr Gln Asp Leu Phe Leu Pro Phe Tyr Ser
    50              55              60


Asn Val Thr Gly Phe His Thr Ile Asn His Thr Phe Gly Asn Pro Val
65              70              75              80


Ile Pro Phe Lys Asp Gly Ile Tyr Phe Ala Ala Thr Glu Lys Ser Asn
            85              90              95


Val Val Arg Gly Trp Val Phe Gly Ser Thr Met Asn Asn Lys Ser Gln
            100             105             110
```

```
Ser Val Ile Ile Ile Asn Asn Ser Thr Asn Val Val Ile Arg Ala Cys
        115                 120                 125

Asn Phe Glu Leu Cys Asp Asn Pro Phe Phe Ala Val Ser Lys Pro Met
        130                 135                 140

Gly Thr Gln Thr His Thr Met Ile Phe Asp Asn Ala Phe Asn Cys Thr
145                 150                 155                 160

Phe Glu Tyr Ile Ser Asp Ala Phe Ser Leu Asp Val Ser Glu Lys Ser
                165                 170                 175

Gly Asn Phe Lys His Leu Arg Glu Phe Val Phe Lys Asn Lys Asp Gly
        180                 185                 190

Phe Leu Tyr Val Tyr Lys Gly Tyr Gln Pro Ile Asp Val Val Arg Asp
        195                 200                 205

Leu Pro Ser Gly Phe Asn Thr Leu Lys Pro Ile Phe Lys Leu Pro Leu
210                 215                 220

Gly Ile Asn Ile Thr Asn Phe Arg Ala Ile Leu Thr Ala Phe Ser Pro
225                 230                 235                 240

Ala Gln Asp Ile Trp Gly Thr Ser Ala Ala Ala Tyr Phe Val Gly Tyr
                245                 250                 255

Leu Lys Pro Thr Thr Phe Met Leu Lys Tyr Asp Glu Asn Gly Thr Ile
                260                 265                 270

Thr Asp Ala Val Asp Cys Ser Gln Asn Pro Leu Ala Glu Leu Lys Cys
        275                 280                 285

Ser Val Lys Ser Phe Glu Ile Asp Lys Gly Ile Tyr Gln Thr Ser Asn
        290                 295                 300

Phe Arg Val Val Pro Ser Gly Asp Val Val Arg Phe Pro Asn Ile Thr
305                 310                 315                 320

Asn Leu Cys Pro Phe Gly Glu Val Phe Asn Ala Thr Lys Phe Pro Ser
                325                 330                 335

Val Tyr Ala Trp Glu Arg Lys Lys Ile Ser Asn Cys Val Ala Asp Tyr
                340                 345                 350

Ser Val Leu Tyr Asn Ser Thr Phe Phe Ser Thr Phe Lys Cys Tyr Gly
        355                 360                 365
```

31

Val Ser Ala Thr Lys Leu Asn Asp Leu Cys Phe Ser Asn Val Tyr Ala
370 375 380

Asp Ser Phe Val Val Lys Gly Asp Asp Val Arg Gln Ile Ala Pro Gly
385 390 395 400

Gln Thr Gly Val Ile Ala Asp Tyr Asn Tyr Lys Leu Pro Asp Asp Phe
405 410 415

Met Gly Cys Val Leu Ala Trp Asn Thr Arg Asn Ile Asp Ala Thr Ser
420 425 430

Thr Gly Asn Tyr Asn Tyr Lys Tyr Arg Tyr Leu Arg His Gly Lys Leu
435 440 445

Arg Pro Phe Glu Arg Asp Ile Ser Asn Val Pro Phe Ser Pro Asp Gly
450 455 460

Lys Pro Cys Thr Pro Pro Ala Leu Asn Cys Tyr Trp Pro Leu Asn Asp
465 470 475 480

Tyr Gly Phe Tyr Thr Thr Thr Gly Ile Gly Tyr Gln Pro Tyr Arg Val
485 490 495

Val Val Leu Ser Phe Glu Leu Leu Asn Ala Pro Ala Thr Val Cys Gly
500 505 510

Pro Lys Leu Ser Thr Asp Leu Ile Lys Asn Gln Cys Val Asn Phe Asn
515 520 525

Phe Asn Gly Leu Thr Gly Thr Gly Val Leu Thr Pro Ser Ser Lys Arg
530 535 540

Phe Gln Pro Phe Gln Gln Phe Gly Arg Asp Val Ser Asp Phe Thr Asp
545 550 555 560

Ser Val Arg Asp Pro Lys Thr Ser Glu Ile Leu Asp Ile Ser Pro Cys
565 570 575

Ser Phe Gly Gly Val Ser Val Ile Thr Pro Gly Thr Asn Ala Ser Ser
580 585 590

Glu Val Ala Val Leu Tyr Gln Asp Val Asn Cys Thr Asp Val Ser Thr
595 600 605

Ala Ile His Ala Asp Gln Leu Thr Pro Ala Trp Arg Ile Tyr Ser Thr
610 615 620

Gly Asn Asn Val Phe Gln Thr Gln Ala Gly Cys Leu Ile Gly Ala Glu
625                 630             635                 640

His Val Asp Thr Ser Tyr Glu Cys Asp Ile Pro Ile Gly Ala Gly Ile
                645             650                 655

Cys Ala Ser Tyr His Thr Val Ser Leu Leu Arg Ser Thr Ser Gln Lys
            660             665             670

Ser Ile Val Ala Tyr Thr Met Ser Leu Gly Ala Asp Ser Ser Ile Ala
            675             680             685

Tyr Ser Asn Asn Thr Ile Ala Ile Pro Thr Asn Phe Ser Ile Ser Ile
    690             695             700

Thr Thr Glu Val Met Pro Val Ser Met Ala Lys Thr Ser Val Asp Cys
705             710             715                 720

Asn Met Tyr Ile Cys Gly Asp Ser Thr Glu Cys Ala Asn Leu Leu Leu
            725             730             735

Gln Tyr Gly Ser Phe Cys Thr Gln Leu Asn Arg Ala Leu Ser Gly Ile
            740             745             750

Ala Ala Glu Gln Asp Arg Asn Thr Arg Glu Val Phe Ala Gln Val Lys
    755             760             765

Gln Met Tyr Lys Thr Pro Thr Leu Lys Tyr Phe Gly Gly Phe Asn Phe
    770             775             780

Ser Gln Ile Leu Pro Asp Pro Leu Lys Pro Thr Lys Arg Ser Phe Ile
785             790             795                 800

Glu Asp Leu Leu Phe Asn Lys Val Thr Leu Ala Asp Ala Gly Phe Met
            805             810             815

Lys Gln Tyr Gly Glu Cys Leu Gly Asp Ile Asn Ala Arg Asp Leu Ile
            820             825             830

Cys Ala Gln Lys Phe Asn Gly Leu Thr Val Leu Pro Pro Leu Leu Thr
    835             840             845

Asp Asp Met Ile Ala Ala Tyr Thr Ala Ala Leu Val Ser Gly Thr Ala
    850             855             860

Thr Ala Gly Trp Thr Phe Gly Ala Gly Ala Ala Leu Gln Ile Pro Phe

33

865                   870                 875                880

Ala Met Gln Met Ala Tyr Arg Phe Asn Gly Ile Gly Val Thr Gln Asn
             885                890             895

Val Leu Tyr Glu Asn Gln Lys Gln Ile Ala Asn Gln Phe Asn Lys Ala
      900               905             910

Ile Ser Gln Ile Gln Glu Ser Leu Thr Thr Thr Ser Thr Ala Leu Gly
     915              920            925

Lys Leu Gln Asp Val Val Asn Gln Asn Ala Gln Ala Leu Asn Thr Leu
   930             935           940

Val Lys Gln Leu Ser Ser Asn Phe Gly Ala Ile Ser Ser Val Leu Asn
945            950           955         960

Asp Ile Leu Ser Arg Leu Asp Lys Val Glu Ala Glu Val Gln Ile Asp
         965           970          975

Arg Leu Ile Thr Gly Arg Leu Gln Ser Leu Gln Thr Tyr Val Thr Gln
        980            985          990

Gln Leu Ile Arg Ala Ala Glu Ile Arg Ala Ser Ala Asn Leu Ala Ala
     995             1000          1005

Thr Lys Met Ser Glu Cys Val Leu Gly Gln Ser Lys Arg Val Asp
   1010            1015          1020

Phe Cys Gly Lys Gly Tyr His Leu Met Ser Phe Pro Gln Ala Ala
   1025            1030          1035

Pro His Gly Val Val Phe Leu His Val Thr Tyr Val Pro Ser Gln
   1040            1045          1050

Glu Arg Asn Phe Thr Thr Ala Pro Ala Ile Cys His Glu Gly Lys
   1055            1060          1065

Ala Tyr Phe Pro Arg Glu Gly Val Phe Val Phe Asn Gly Thr Ser
   1070            1075          1080

Trp Phe Ile Thr Gln Arg Asn Phe Phe Ser Pro Gln Ile Ile Thr
   1085            1090          1095

Thr Asp Asn Thr Phe Val Ser Gly Asn Cys Asp Val Val Ile Gly
   1100            1105          1110

```
Ile Ile Asn Asn Thr Val Tyr Asp Pro Leu Gln Pro Glu Leu Asp
    1115                1120                1125

Ser Phe Lys Glu Glu Leu Asp Lys Tyr Phe Lys Asn His Thr Ser
    1130                1135                1140

Pro Asp Val Asp Leu Gly Asp Ile Ser Gly Ile Asn Ala Ser Val
    1145                1150                1155

Val Asn Ile Gln Lys Glu Ile Asp Arg Leu Asn Glu Val Ala Lys
    1160                1165                1170

Asn Leu Asn Glu Ser Leu Ile Asp Leu Gln Glu Leu Gly Lys Tyr
    1175                1180                1185

Glu Gln Tyr Ile Lys Trp Pro Trp Tyr Val Trp Leu Gly Phe Ile
    1190                1195                1200

Ala Gly Leu Ile Ala Ile Val Met Val Thr Ile Leu Leu Cys Cys
    1205                1210                1215

Met Thr Ser Cys Cys Ser Cys Leu Lys Gly Ala Cys Ser Cys Gly
    1220                1225                1230

Ser Cys Cys Lys Phe Asp Glu Asp Asp Ser Glu Pro Val Leu Lys
    1235                1240                1245

Gly Val Lys Leu His Tyr Thr
    1250                1255
```

```
<210>   29
<211>   1356
<212>   PRT
<213>   hCoV-NL63

<400>   29

Met Lys Leu Phe Leu Ile Leu Leu Val Leu Pro Leu Ala Ser Cys Phe
1               5                   10                  15

Phe Thr Cys Asn Ser Asn Ala Asn Leu Ser Met Leu Gln Leu Gly Val
        20                  25                  30

Pro Asp Asn Ser Ser Thr Ile Val Thr Gly Leu Leu Pro Thr His Trp
        35                  40                  45

Phe Cys Ala Asn Gln Ser Thr Ser Val Tyr Ser Ala Asn Gly Phe Phe
        50                  55                  60
```

```
Tyr Ile Asp Val Gly Asn His Arg Ser Ala Phe Ala Leu His Thr Gly
65              70              75                          80

Tyr Tyr Asp Ala Asn Gln Tyr Tyr Ile Tyr Val Thr Asn Glu Ile Gly
                85              90                          95

Leu Asn Ala Ser Val Thr Leu Lys Ile Cys Lys Phe Ser Arg Asn Thr
            100             105             110

Thr Phe Asp Phe Leu Ser Asn Ala Ser Ser Ser Phe Asp Cys Ile Val
        115             120             125

Asn Leu Leu Phe Thr Glu Gln Leu Gly Ala Pro Leu Gly Ile Thr Ile
    130             135             140

Ser Gly Glu Thr Val Arg Leu His Leu Tyr Asn Val Thr Arg Thr Phe
145             150             155             160

Tyr Val Pro Ala Ala Tyr Lys Leu Thr Lys Leu Ser Val Lys Cys Tyr
            165             170             175

Phe Asn Tyr Ser Cys Val Phe Ser Val Val Asn Ala Thr Val Thr Val
            180             185             190

Asn Val Thr Thr His Asn Gly Arg Val Val Asn Tyr Thr Val Cys Asp
        195             200             205

Asp Cys Asn Gly Tyr Thr Asp Asn Ile Phe Ser Val Gln Gln Asp Gly
    210             215             220

Arg Ile Pro Asn Gly Phe Pro Phe Asn Asn Trp Phe Leu Leu Thr Asn
225             230             235             240

Gly Ser Thr Leu Val Asp Gly Val Ser Arg Leu Tyr Gln Pro Leu Arg
            245             250             255

Leu Thr Cys Leu Trp Pro Val Pro Gly Leu Lys Ser Ser Thr Gly Phe
        260             265             270

Val Tyr Phe Asn Ala Thr Gly Ser Asp Val Asn Cys Asn Gly Tyr Gln
        275             280             285

His Asn Ser Val Val Asp Val Met Arg Tyr Asn Leu Asn Phe Ser Ala
    290             295             300

Asn Ser Leu Asp Asn Leu Lys Ser Gly Val Ile Val Phe Lys Thr Leu
305             310             315             320
```

Gln Tyr Asp Val Leu Phe Tyr Cys Ser Asn Ser Ser Ser Gly Val Leu
                325             330             335

Asp Thr Thr Ile Pro Phe Gly Pro Ser Ser Gln Pro Tyr Tyr Cys Phe
                340             345             350

Ile Asn Ser Thr Ile Asn Thr Thr His Val Ser Thr Phe Val Gly Ile
        355             360             365

Leu Pro Pro Thr Val Arg Glu Ile Val Val Ala Arg Thr Gly Gln Phe
    370             375             380

Tyr Ile Asn Gly Phe Lys Tyr Phe Asp Leu Gly Phe Ile Glu Ala Val
385             390             395                 400

Asn Phe Asn Val Thr Thr Ala Ser Ala Thr Asp Phe Trp Thr Val Ala
                405             410             415

Phe Ala Thr Phe Val Asp Val Leu Val Asn Val Ser Ala Thr Asn Ile
            420             425             430

Gln Asn Leu Leu Tyr Cys Asp Ser Pro Phe Glu Lys Leu Gln Cys Glu
        435             440             445

His Leu Gln Phe Gly Leu Gln Asp Gly Phe Tyr Ser Ala Asn Phe Leu
    450             455             460

Asp Asp Asn Val Leu Pro Glu Thr Tyr Val Ala Leu Pro Ile Tyr Tyr
465             470             475                 480

Gln His Thr Asp Ile Asn Phe Thr Ala Thr Ala Ser Phe Gly Gly Ser
                485             490             495

Cys Tyr Val Cys Lys Pro His Gln Val Asn Ile Ser Leu Asn Gly Asn
            500             505             510

Thr Ser Val Cys Val Arg Thr Ser His Phe Ser Ile Arg Tyr Ile Tyr
        515             520             525

Asn Arg Val Lys Ser Gly Ser Pro Gly Asp Ser Ser Trp His Ile Tyr
    530             535             540

Leu Lys Ser Gly Thr Cys Pro Phe Ser Phe Ser Lys Leu Asn Asn Phe
545             550             555                 560

Gln Lys Phe Lys Thr Ile Cys Phe Ser Thr Val Glu Val Pro Gly Ser
                565             570             575

37

Cys Asn Phe Pro Leu Glu Ala Thr Trp His Tyr Thr Ser Tyr Thr Ile
                580                 585                 590

Val Gly Ala Leu Tyr Val Thr Trp Ser Glu Gly Asn Ser Ile Thr Gly
                595                 600                 605

Val Pro Tyr Pro Val Ser Gly Ile Arg Glu Phe Ser Asn Leu Val Leu
        610                 615                 620

Asn Asn Cys Thr Lys Tyr Asn Ile Tyr Asp Tyr Val Gly Thr Gly Ile
625                 630                 635                 640

Ile Arg Ser Ser Asn Gln Ser Leu Ala Gly Gly Ile Thr Tyr Val Ser
                645                 650                 655

Asn Ser Gly Asn Leu Leu Gly Phe Lys Asn Val Ser Thr Gly Asn Ile
                660                 665                 670

Phe Ile Val Thr Pro Cys Asn Gln Pro Asp Gln Val Ala Val Tyr Gln
        675                 680                 685

Gln Ser Ile Ile Gly Ala Met Thr Ala Val Asn Glu Ser Arg Tyr Gly
        690                 695                 700

Leu Gln Asn Leu Leu Gln Leu Pro Asn Phe Tyr Tyr Val Ser Asn Gly
705                 710                 715                 720

Gly Asn Asn Cys Thr Thr Ala Val Met Thr Tyr Ser Asn Phe Gly Ile
                725                 730                 735

Cys Ala Asp Gly Ser Leu Ile Pro Val Arg Pro Arg Asn Ser Ser Asp
                740                 745                 750

Asn Gly Ile Ser Ala Ile Ile Thr Ala Asn Leu Ser Ile Pro Ser Asn
                755                 760                 765

Trp Thr Thr Ser Val Gln Val Glu Tyr Leu Gln Ile Thr Ser Thr Pro
        770                 775                 780

Ile Val Val Asp Cys Ala Thr Tyr Val Cys Asn Gly Asn Pro Arg Cys
785                 790                 795                 800

Lys Asn Leu Leu Lys Gln Tyr Thr Ser Ala Cys Lys Thr Ile Glu Asp
                805                 810                 815

Ala Leu Arg Leu Ser Ala His Leu Glu Thr Asn Asp Val Ser Ser Met

```
                    820                     825                     830

Leu Thr Phe Asp Ser Asn Ala Phe Ser Leu Ala Asn Val Thr Ser Phe
        835                 840                 845

Gly Asp Tyr Asn Leu Ser Ser Val Leu Pro Gln Arg Asn Ile Arg Ser
    850                 855                 860

Ser Arg Ile Ala Gly Arg Ser Ala Leu Glu Asp Leu Leu Phe Ser Lys
865                 870                 875                 880

Val Val Thr Ser Gly Leu Gly Thr Val Asp Val Asp Tyr Lys Ser Cys
            885                 890                 895

Thr Lys Gly Leu Ser Ile Ala Asp Leu Ala Cys Ala Gln Tyr Tyr Asn
            900                 905                 910

Gly Ile Met Val Leu Pro Gly Val Ala Asp Ala Glu Arg Met Ala Met
        915                 920                 925

Tyr Thr Gly Ser Leu Ile Gly Gly Met Val Leu Gly Gly Leu Thr Ser
    930                 935                 940

Ala Ala Ala Ile Pro Phe Ser Leu Ala Leu Gln Ala Arg Leu Asn Tyr
945                 950                 955                 960

Val Ala Leu Gln Thr Asp Val Leu Gln Glu Asn Gln Lys Ile Leu Ala
            965                 970                 975

Ala Ser Phe Asn Lys Ala Ile Asn Asn Ile Val Ala Ser Phe Ser Ser
        980                 985                 990

Val Asn Asp Ala Ile Thr Gln Thr  Ala Glu Ala Ile His  Thr Val Thr
        995                 1000                1005

Ile Ala  Leu Asn Lys Ile Gln  Asp Val Val Asn Gln  Gln Gly Ser
    1010                1015                1020

Ala Leu  Asn His Leu Thr Ser  Gln Leu Arg His Asn  Phe Gln Ala
    1025                1030                1035

Ile Ser  Asn Ser Ile Gln Ala  Ile Tyr Asp Arg Leu  Asp Ser Ile
    1040                1045                1050

Gln Ala  Asp Gln Gln Val Asp  Arg Leu Ile Thr Gly  Arg Leu Ala
    1055                1060                1065
```

```
Ala Leu Asn Ala Phe Val Ser  Gln Val Leu Asn Lys  Tyr Thr Glu
    1070             1075             1080

Val Arg Gly Ser Arg Arg Leu  Ala Gln Gln Lys Ile  Asn Glu Cys
    1085             1090             1095

Val Lys Ser Gln Ser Asn Arg  Tyr Gly Phe Cys Gly  Asn Gly Thr
    1100             1105             1110

His Ile Phe Ser Ile Val Asn  Ser Ala Pro Asp Gly  Leu Leu Phe
    1115             1120             1125

Leu His Thr Val Leu Leu Pro  Thr Asp Tyr Lys Asn  Val Lys Ala
    1130             1135             1140

Trp Ser Gly Ile Cys Val Asp  Gly Ile Tyr Gly Tyr  Val Leu Arg
    1145             1150             1155

Gln Pro Asn Leu Val Leu Tyr  Ser Asp Asn Gly Val  Phe Arg Val
    1160             1165             1170

Thr Ser Arg Val Met Phe Gln  Pro Arg Leu Pro Val  Leu Ser Asp
    1175             1180             1185

Phe Val Gln Ile Tyr Asn Cys  Asn Val Thr Phe Val  Asn Ile Ser
    1190             1195             1200

Arg Val Glu Leu His Thr Val  Ile Pro Asp Tyr Val  Asp Val Asn
    1205             1210             1215

Lys Thr Leu Gln Glu Phe Ala  Gln Asn Leu Pro Lys  Tyr Val Lys
    1220             1225             1230

Pro Asn Phe Asp Leu Thr Pro  Phe Asn Leu Thr Tyr  Leu Asn Leu
    1235             1240             1245

Ser Ser Glu Leu Lys Gln Leu  Glu Ala Lys Thr Ala  Ser Leu Phe
    1250             1255             1260

Gln Thr Thr Val Glu Leu Gln  Gly Leu Ile Asp Gln  Ile Asn Ser
    1265             1270             1275

Thr Tyr Val Asp Leu Lys Leu  Leu Asn Arg Phe Glu  Asn Tyr Ile
    1280             1285             1290

Lys Trp Pro Trp Trp Val Trp  Leu Ile Ile Ser Val  Val Phe Val
    1295             1300             1305
```

40

```
Val Leu  Leu Ser Leu Leu Val  Phe Cys Cys Leu Ser  Thr Gly Cys
    1310             1315             1320

Cys Gly  Cys Cys Asn Cys Leu  Thr Ser Ser Met Arg  Gly Cys Cys
    1325             1330             1335

Asp Cys  Gly Ser Thr Lys Leu  Pro Tyr Tyr Glu Phe  Glu Lys Val
    1340             1345             1350

His Val  Gln
    1355
```

<210>  30
<211>  1361
<212>  PRT
<213>  hCoV-OC43

<400>  30

```
Met Phe Leu Ile Leu Leu Ile Ser Leu Pro Thr Ala Phe Ala Val Ile
1               5               10              15

Gly Asp Leu Lys Cys Thr Ser Asp Thr Ser Tyr Ile Asn Asp Lys Asp
        20              25              30

Thr Gly Pro Pro Pro Ile Ser Thr Asp Thr Val Asp Val Thr Asn Gly
        35              40              45

Leu Gly Thr Tyr Tyr Val Leu Asp Arg Val Tyr Leu Asn Thr Thr Leu
    50              55              60

Phe Leu Asn Gly Tyr Tyr Pro Thr Ser Gly Ser Thr Tyr Arg Asn Met
65              70              75              80

Ala Leu Lys Gly Ser Val Leu Leu Ser Arg Leu Trp Phe Lys Pro Pro
                85              90              95

Phe Leu Ser Asp Phe Ile Asn Gly Ile Phe Ala Lys Val Lys Asn Thr
            100             105             110

Lys Val Ile Lys Asp Arg Val Met Tyr Ser Glu Phe Pro Ala Ile Thr
        115             120             125

Ile Gly Ser Thr Phe Val Asn Thr Ser Tyr Ser Val Val Val Gln Pro
    130             135             140

Arg Thr Ile Asn Ser Thr Gln Asp Gly Tyr Asn Lys Leu Gln Gly Leu
145             150             155             160
```

41

```
Leu Glu Val Ser Val Cys Gln Tyr Asn Met Cys Glu Tyr Pro Gln Thr
            165                 170                 175

Ile Cys His Pro Asn Leu Gly Asn His Arg Lys Glu Leu Trp His Leu
            180                 185                 190

Asp Thr Gly Val Val Ser Cys Leu Tyr Lys Arg Asn Phe Thr Tyr Asp
            195                 200                 205

Val Asn Ala Asp Tyr Leu Tyr Phe His Phe Tyr Gln Glu Gly Gly Thr
            210                 215                 220

Phe Tyr Ala Tyr Phe Thr Asp Thr Gly Val Val Thr Lys Phe Leu Phe
225                 230                 235                 240

Asn Val Tyr Leu Gly Met Ala Leu Ser His Tyr Tyr Val Met Pro Leu
                245                 250                 255

Thr Cys Asn Ser Lys Val Lys Asn Gly Phe Thr Leu Glu Tyr Trp Val
            260                 265                 270

Thr Pro Leu Thr Ser Arg Gln Tyr Leu Leu Ala Phe Asn Gln Asp Gly
            275                 280                 285

Ile Ile Phe Asn Ala Val Asp Cys Met Ser Asp Phe Met Ser Glu Ile
            290                 295                 300

Lys Cys Lys Thr Gln Ser Ile Ala Pro Pro Thr Gly Val Tyr Glu Leu
305                 310                 315                 320

Asn Gly Tyr Thr Val Gln Pro Ile Ala Asp Val Tyr Arg Arg Lys Leu
                325                 330                 335

Asn Leu Pro Asn Cys Asn Ile Glu Ala Trp Leu Asn Asp Lys Ser Val
            340                 345                 350

Pro Ser Pro Leu Asn Trp Glu Arg Lys Thr Phe Ser Asn Cys Asn Phe
            355                 360                 365

Asn Met Ser Ser Leu Met Ser Phe Ile Gln Ala Asp Ser Phe Thr Cys
            370                 375                 380

Asn Asn Ile Asp Ala Ala Lys Ile Tyr Gly Met Cys Phe Ser Ser Ile
385                 390                 395                 400

Thr Ile Asp Lys Phe Ala Ile Pro Asn Gly Arg Lys Val Asp Leu Gln
                405                 410                 415
```

```
Leu Gly Asn Leu Gly Tyr Leu Gln Ser Phe Asn Tyr Arg Ile Asp Thr
        420             425             430

Thr Ala Thr Ser Cys Gln Leu Tyr Tyr Asn Leu Pro Ala Ala Asn Val
        435             440             445

Ser Val Ser Arg Phe Asn Pro Ser Thr Trp Asn Lys Arg Phe Gly Phe
        450             455             460

Ile Glu Asp Ser Val Phe Lys Pro Arg Pro Ala Gly Val Leu Thr Asn
465             470             475             480

His Asp Val Val Tyr Ala Gln His Cys Phe Lys Ala Pro Lys Asn Phe
                485             490             495

Cys Pro Cys Lys Leu Asn Gly Ser Cys Val Gly Ser Gly Pro Gly Lys
        500             505             510

Asn Asn Gly Ile Gly Thr Cys Pro Ala Gly Thr Asn Tyr Leu Thr Cys
        515             520             525

Asp Asn Leu Cys Thr Pro Asp Pro Ile Thr Phe Lys Ala Thr Gly Thr
        530             535             540

Tyr Lys Cys Pro Gln Thr Lys Ser Leu Val Gly Ile Gly Glu His Cys
545             550             555             560

Ser Gly Leu Ala Val Lys Ser Asp Tyr Cys Gly Gly Asn Ser Cys Thr
                565             570             575

Cys Arg Pro Gln Ala Phe Leu Gly Trp Ser Ala Asp Ser Cys Leu Gln
        580             585             590

Gly Asp Lys Cys Asn Ile Phe Ala Asn Phe Ile Leu His Asp Val Asn
        595             600             605

Ser Gly Leu Thr Cys Ser Thr Asp Leu Gln Lys Ala Asn Thr Asp Ile
        610             615             620

Ile Leu Gly Val Cys Val Asn Tyr Asp Leu Tyr Gly Ile Leu Gly Gln
625             630             635             640

Gly Ile Phe Val Glu Val Asn Ala Thr Tyr Tyr Asn Ser Trp Gln Asn
                645             650             655

Leu Leu Tyr Asp Ser Asn Gly Asn Leu Tyr Gly Phe Arg Asp Tyr Ile
```

43

```
                     660                          665                          670

        Thr Asn Arg Thr Phe Met Ile Arg Ser Cys Tyr Ser Gly Arg Val Ser
                675                      680                  685


        Ala Ala Phe His Ala Asn Ser Ser Glu Pro Ala Leu Leu Phe Arg Asn
                690                      695                  700


        Ile Lys Cys Asn Tyr Val Phe Asn Asn Ser Leu Thr Arg Gln Leu Gln
        705                      710                  715                  720


        Pro Ile Asn Tyr Phe Asp Ser Tyr Leu Gly Cys Val Val Asn Ala Tyr
                        725                  730                      735


        Asn Ser Thr Ala Ile Ser Val Gln Thr Cys Asp Leu Thr Val Gly Ser
                    740                  745                  750


        Gly Tyr Cys Val Asp Tyr Ser Lys Asn Arg Arg Ser Arg Gly Ala Ile
                755                  760                  765


        Thr Thr Gly Tyr Arg Phe Thr Asn Phe Glu Pro Phe Thr Val Asn Ser
            770                  775                  780


        Val Asn Asp Ser Leu Glu Pro Val Gly Gly Leu Tyr Glu Ile Gln Ile
        785                  790                  795                  800


        Pro Ser Glu Phe Thr Ile Gly Asn Met Glu Glu Phe Ile Gln Thr Ser
                    805                  810                  815


        Ser Pro Lys Val Thr Ile Asp Cys Ala Ala Phe Val Cys Gly Asp Tyr
                820                  825                  830


        Ala Ala Cys Lys Ser Gln Leu Val Glu Tyr Gly Ser Phe Cys Asp Asn
                835                  840                  845


        Ile Asn Ala Ile Leu Thr Glu Val Asn Glu Leu Leu Asp Thr Thr Gln
            850                  855                  860


        Leu Gln Val Ala Asn Ser Leu Met Asn Gly Val Thr Leu Ser Thr Lys
        865                  870                  875                  880


        Leu Lys Asp Gly Val Asn Phe Asn Val Asp Asp Ile Asn Phe Ser Pro
                        885                  890                      895


        Val Leu Gly Cys Leu Gly Ser Glu Cys Ser Lys Ala Ser Ser Arg Ser
                    900                  905                  910
```

```
Ala Ile Glu Asp Leu Leu Phe Asp Lys Val Lys Leu Ser Asp Val Gly
    915                 920                 925

Phe Val Glu Ala Tyr Asn Asn Cys Thr Gly Gly Ala Glu Ile Arg Asp
    930                 935                 940

Leu Ile Cys Val Gln Ser Tyr Lys Gly Ile Lys Val Leu Pro Pro Leu
945                 950                 955                 960

Leu Ser Glu Asn Gln Ile Ser Gly Tyr Thr Leu Ala Ala Thr Ser Ala
                965                 970                 975

Ser Leu Phe Pro Leu Trp Thr Ala Ala Ala Gly Val Pro Phe Tyr Leu
        980                 985                 990

Asn Val Gln Tyr Arg Ile Asn Gly  Leu Gly Val Thr Met  Asp Val Leu
        995                 1000                1005

Ser Gln  Asn Gln Lys Leu Ile  Ala Asn Ala Phe Asn  Asn Ala Leu
    1010                1015                1020

Tyr Ala  Ile Gln Glu Gly Phe  Asp Ala Thr Asn Ser  Ala Leu Val
    1025                1030                1035

Lys Ile  Gln Ala Val Val Asn  Ala Asn Ala Glu Ala  Leu Asn Asn
    1040                1045                1050

Leu Leu  Gln Gln Leu Ser Asn  Arg Phe Gly Ala Ile  Ser Ala Ser
    1055                1060                1065

Leu Gln  Glu Ile Leu Ser Arg  Leu Asp Ala Leu Glu  Ala Glu Ala
    1070                1075                1080

Gln Ile  Asp Arg Leu Ile Asn  Gly Arg Leu Thr Ala  Leu Asn Ala
    1085                1090                1095

Tyr Val  Ser Gln Gln Leu Ser  Asp Ser Thr Leu Val  Lys Phe Ser
    1100                1105                1110

Ala Ala  Gln Ala Met Glu Lys  Val Asn Glu Cys Val  Lys Ser Gln
    1115                1120                1125

Ser Ser  Arg Ile Asn Phe Cys  Gly Asn Gly Asn His  Ile Ile Ser
    1130                1135                1140

Leu Val  Gln Asn Ala Pro Tyr  Gly Leu Tyr Phe Ile  His Phe Ser
    1145                1150                1155
```

45

```
Tyr Val Pro Thr Lys Tyr Val Thr Ala Arg Val Ser Pro Gly Leu
    1160            1165            1170

Cys Ile Ala Gly Asp Arg Gly Ile Ala Pro Lys Ser Gly Tyr Phe
    1175            1180            1185

Val Asn Val Asn Asn Thr Trp Met Tyr Thr Gly Ser Gly Tyr Tyr
    1190            1195            1200

Tyr Pro Glu Pro Ile Thr Glu Asn Asn Val Val Val Met Ser Thr
    1205            1210            1215

Cys Ala Val Asn Tyr Thr Lys Ala Pro Tyr Val Met Leu Asn Thr
    1220            1225            1230

Ser Ile Pro Asn Leu Pro Asp Phe Lys Glu Glu Leu Asp Gln Trp
    1235            1240            1245

Phe Lys Asn Gln Thr Ser Val Ala Pro Asp Leu Ser Leu Asp Tyr
    1250            1255            1260

Ile Asn Val Thr Phe Leu Asp Leu Gln Val Glu Met Asn Arg Leu
    1265            1270            1275

Gln Glu Ala Ile Lys Val Leu Asn Gln Ser Tyr Ile Asn Leu Lys
    1280            1285            1290

Asp Ile Gly Thr Tyr Glu Tyr Tyr Val Lys Trp Pro Trp Tyr Val
    1295            1300            1305

Trp Leu Leu Ile Cys Leu Ala Gly Val Ala Met Leu Val Leu Leu
    1310            1315            1320

Phe Phe Ile Cys Cys Cys Thr Gly Cys Gly Thr Ser Cys Phe Lys
    1325            1330            1335

Lys Cys Gly Gly Cys Cys Asp Asp Tyr Thr Gly Tyr Gln Glu Leu
    1340            1345            1350

Val Ile Lys Thr Ser His Asp Asp
    1355            1360


<210>  31
<211>  1173
<212>  PRT
<213>  hCoV-229E

<400>  31
```

```
Met Phe Val Leu Leu Val Ala Tyr Ala Leu Leu His Ile Ala Gly Cys
1               5                   10                  15

Gln Thr Thr Asn Gly Leu Asn Thr Ser Tyr Ser Val Cys Asn Gly Cys
            20                  25                  30

Val Gly Tyr Ser Glu Asn Val Phe Ala Val Glu Ser Gly Gly Tyr Ile
        35                  40                  45

Pro Ser Asp Phe Ala Phe Asn Asn Trp Phe Leu Leu Thr Asn Thr Ser
    50                  55                  60

Ser Val Val Asp Gly Val Val Arg Ser Phe Gln Pro Leu Leu Leu Asn
65                  70                  75                  80

Cys Leu Trp Ser Val Ser Gly Leu Arg Phe Thr Thr Gly Phe Val Tyr
            85                  90                  95

Phe Asn Gly Thr Gly Arg Gly Asp Cys Lys Gly Phe Ser Ser Asp Val
            100                 105                 110

Leu Ser Asp Val Ile Arg Tyr Asn Leu Asn Phe Glu Glu Asn Leu Arg
        115                 120                 125

Arg Gly Thr Ile Leu Phe Lys Thr Ser Tyr Gly Val Val Val Phe Tyr
    130                 135                 140

Cys Thr Asn Asn Thr Leu Val Ser Gly Asp Ala His Ile Pro Phe Gly
145                 150                 155                 160

Thr Val Leu Gly Asn Phe Tyr Cys Phe Val Asn Thr Thr Ile Gly Asn
            165                 170                 175

Glu Thr Thr Ser Ala Phe Val Gly Ala Leu Pro Lys Thr Val Arg Glu
            180                 185                 190

Phe Val Ile Ser Arg Thr Gly His Phe Tyr Ile Asn Gly Tyr Arg Tyr
        195                 200                 205

Phe Thr Leu Gly Asn Val Glu Ala Val Asn Phe Asn Val Thr Thr Ala
    210                 215                 220

Glu Thr Thr Asp Phe Cys Thr Val Ala Leu Ala Ser Tyr Ala Asp Val
225                 230                 235                 240

Leu Val Asn Val Ser Gln Thr Ser Ile Ala Asn Ile Ile Tyr Cys Asn
            245                 250                 255
```

47

```
Ser Val Ile Asn Arg Leu Arg Cys Asp Gln Leu Ser Phe Asp Val Pro
        260             265             270

Asp Gly Phe Tyr Ser Thr Ser Pro Ile Gln Ser Val Glu Leu Pro Val
        275             280             285

Ser Ile Val Ser Leu Pro Val Tyr His Lys His Thr Phe Ile Val Leu
    290             295             300

Tyr Val Asp Phe Lys Pro Gln Ser Gly Gly Gly Lys Cys Phe Asn Cys
305             310             315             320

Tyr Pro Ala Gly Val Asn Ile Thr Leu Ala Asn Phe Asn Glu Thr Lys
            325             330             335

Gly Pro Leu Cys Val Asp Thr Ser His Phe Thr Thr Lys Tyr Val Ala
        340             345             350

Val Tyr Ala Asn Val Gly Arg Trp Ser Ala Ser Ile Asn Thr Gly Asn
        355             360             365

Cys Pro Phe Ser Phe Gly Lys Val Asn Asn Phe Val Lys Phe Gly Ser
    370             375             380

Val Cys Phe Ser Leu Lys Asp Ile Pro Gly Gly Cys Ala Met Pro Ile
385             390             395             400

Val Ala Asn Trp Ala Tyr Ser Lys Tyr Tyr Thr Ile Gly Ser Leu Tyr
            405             410             415

Val Ser Trp Ser Asp Gly Asp Gly Ile Thr Gly Val Pro Gln Pro Val
        420             425             430

Glu Gly Val Ser Ser Phe Met Asn Val Thr Leu Asp Lys Cys Thr Lys
        435             440             445

Tyr Asn Ile Tyr Asp Val Ser Gly Val Gly Val Ile Arg Val Ser Asn
    450             455             460

Asp Thr Phe Leu Asn Gly Ile Thr Tyr Thr Ser Thr Ser Gly Asn Leu
465             470             475             480

Leu Gly Phe Lys Asp Val Thr Lys Gly Thr Ile Tyr Ser Ile Thr Pro
            485             490             495

Cys Asn Pro Pro Asp Gln Leu Val Val Tyr Gln Gln Ala Val Val Gly
```

|  |  |  | 500 |  |  |  |  | 505 |  |  |  |  | 510 |  |  |

Ala Met Leu Ser Glu Asn Phe Thr Ser Tyr Gly Phe Ser Asn Val Val
515                     520                     525

Glu Leu Pro Lys Phe Phe Tyr Ala Ser Asn Gly Thr Tyr Asn Cys Thr
530                     535                     540

Asp Ala Val Leu Thr Tyr Ser Ser Phe Gly Val Cys Ala Asp Gly Ser
545                     550                     555                     560

Ile Ile Ala Val Gln Pro Arg Asn Val Ser Tyr Asp Ser Val Ser Ala
565                     570                     575

Ile Val Thr Ala Asn Leu Ser Ile Pro Ser Asn Trp Thr Thr Ser Val
580                     585                     590

Gln Val Glu Tyr Leu Gln Ile Thr Ser Thr Pro Ile Val Val Asp Cys
595                     600                     605

Ser Thr Tyr Val Cys Asn Gly Asn Val Arg Cys Val Glu Leu Leu Lys
610                     615                     620

Gln Tyr Thr Ser Ala Cys Lys Thr Ile Glu Asp Ala Leu Arg Asn Ser
625                     630                     635                     640

Ala Arg Leu Glu Ser Ala Asp Val Ser Glu Met Leu Thr Phe Asp Lys
645                     650                     655

Lys Ala Phe Thr Leu Ala Asn Val Ser Ser Phe Gly Asp Tyr Asn Leu
660                     665                     670

Ser Ser Val Ile Pro Ser Leu Pro Thr Ser Gly Ser Arg Val Ala Gly
675                     680                     685

Arg Ser Ala Ile Glu Asp Ile Leu Phe Ser Lys Leu Val Thr Ser Gly
690                     695                     700

Leu Gly Thr Val Asp Ala Asp Tyr Lys Lys Cys Thr Lys Gly Leu Ser
705                     710                     715                     720

Ile Ala Asp Leu Ala Cys Ala Gln Tyr Tyr Asn Gly Ile Met Val Leu
725                     730                     735

Pro Gly Val Ala Asp Ala Glu Arg Met Ala Met Tyr Thr Gly Ser Leu
740                     745                     750

Ile Gly Gly Ile Ala Leu Gly Gly Leu Thr Ser Ala Val Ser Ile Pro
        755             760             765

Phe Ser Leu Ala Ile Gln Ala Arg Leu Asn Tyr Val Ala Leu Gln Thr
        770             775             780

Asp Val Leu Gln Glu Asn Gln Lys Ile Leu Ala Ala Ser Phe Asn Lys
785             790             795             800

Ala Met Thr Asn Ile Val Asp Ala Phe Thr Gly Val Asn Asp Ala Ile
                805             810             815

Thr Gln Thr Ser Gln Ala Leu Gln Thr Val Ala Thr Ala Leu Asn Lys
        820             825             830

Ile Gln Asp Val Val Asn Gln Gln Gly Asn Ser Leu Asn His Leu Thr
        835             840             845

Ser Gln Leu Arg Gln Asn Phe Gln Ala Ile Ser Ser Ser Ile Gln Ala
    850             855             860

Ile Tyr Asp Arg Leu Asp Thr Ile Gln Ala Asp Gln Gln Val Asp Arg
865             870             875             880

Leu Ile Thr Gly Arg Leu Ala Ala Leu Asn Val Phe Val Ser His Thr
            885             890             895

Leu Thr Lys Tyr Thr Glu Val Arg Ala Ser Arg Gln Leu Ala Gln Gln
        900             905             910

Lys Val Asn Glu Cys Val Lys Ser Gln Ser Lys Arg Tyr Gly Phe Cys
        915             920             925

Gly Asn Gly Thr His Ile Phe Ser Ile Val Asn Ala Ala Pro Glu Gly
    930             935             940

Leu Val Phe Leu His Thr Val Leu Leu Pro Thr Gln Tyr Lys Asp Val
945             950             955             960

Glu Ala Trp Ser Gly Leu Cys Val Asp Gly Thr Asn Gly Tyr Val Leu
            965             970             975

Arg Gln Pro Asn Leu Ala Leu Tyr Lys Glu Gly Asn Tyr Tyr Arg Ile
        980             985             990

Thr Ser Arg Ile Met Phe Glu Pro  Arg Ile Pro Thr Met  Ala Asp Phe
        995             1000             1005

50

```
Val Gln  Ile Glu Asn Cys Asn  Val Thr Phe Val Asn  Ile Ser Arg
    1010                1015                1020

Ser Glu  Leu Gln Thr Ile Val  Pro Glu Tyr Ile Asp  Val Asn Lys
    1025                1030                1035

Thr Leu  Gln Glu Leu Ser Tyr  Lys Leu Pro Asn Tyr  Thr Val Pro
    1040                1045                1050

Asp Leu  Val Val Glu Gln Tyr  Asn Gln Thr Ile Leu  Asn Leu Thr
    1055                1060                1065

Ser Glu  Ile Ser Thr Leu Glu  Asn Lys Ser Ala Glu  Leu Asn Tyr
    1070                1075                1080

Thr Val  Gln Lys Leu Gln Thr  Leu Ile Asp Asn Ile  Asn Ser Thr
    1085                1090                1095

Leu Val  Asp Leu Lys Trp Leu  Asn Arg Val Glu Thr  Tyr Ile Lys
    1100                1105                1110

Trp Pro  Trp Trp Val Trp Leu  Cys Ile Ser Val Val  Leu Ile Phe
    1115                1120                1125

Val Val  Ser Met Leu Leu Leu  Cys Cys Cys Ser Thr  Gly Cys Cys
    1130                1135                1140

Gly Phe  Phe Ser Cys Phe Ala  Ser Ser Ile Arg Gly  Cys Cys Glu
    1145                1150                1155

Ser Thr  Lys Leu Pro Tyr Tyr  Asp Val Glu Lys Ile  His Ile Gln
    1160                1165                1170
```

```
<210>   32
<211>   1356
<212>   PRT
<213>   huCoV-HKU1

<400>   32

Met Leu Leu Ile Ile Phe Ile Leu Pro Thr Thr Leu Ala Val Ile Gly
1               5                   10                  15

Asp Phe Asn Cys Thr Asn Phe Ala Ile Asn Asp Leu Asn Thr Thr Val
            20                  25                  30

Pro Arg Ile Ser Glu Tyr Val Val Asp Val Ser Tyr Gly Leu Gly Thr
        35                  40                  45
```

Tyr Tyr Ile Leu Asp Arg Val Tyr Leu Asn Thr Thr Ile Leu Phe Thr
50              55              60

Gly Tyr Phe Pro Lys Ser Gly Ala Asn Phe Arg Asp Leu Ser Leu Lys
65              70              75              80

Gly Thr Thr Tyr Leu Ser Thr Leu Trp Tyr Gln Lys Pro Phe Leu Ser
85              90              95

Asp Phe Asn Asn Gly Ile Phe Ser Arg Val Lys Asn Thr Lys Leu Tyr
100             105             110

Val Asn Lys Thr Leu Tyr Ser Glu Phe Ser Thr Ile Val Ile Gly Ser
115             120             125

Val Phe Ile Asn Asn Ser Tyr Thr Ile Val Val Gln Pro His Asn Gly
130             135             140

Val Leu Glu Ile Thr Ala Cys Gln Tyr Thr Met Cys Glu Tyr Pro His
145             150             155             160

Thr Ile Cys Lys Ser Lys Gly Ser Ser Arg Asn Glu Ser Trp His Phe
165             170             175

Asp Lys Ser Glu Pro Leu Cys Leu Phe Lys Lys Asn Phe Thr Tyr Asn
180             185             190

Val Ser Thr Asp Trp Leu Tyr Phe His Phe Tyr Gln Glu Arg Gly Thr
195             200             205

Phe Tyr Ala Tyr Tyr Ala Asp Ser Gly Met Pro Thr Thr Phe Leu Phe
210             215             220

Ser Leu Tyr Leu Gly Thr Leu Leu Ser His Tyr Tyr Val Leu Pro Leu
225             230             235             240

Thr Cys Asn Ala Ile Ser Ser Asn Thr Asp Asn Glu Thr Leu Gln Tyr
245             250             255

Trp Val Thr Pro Leu Ser Lys Arg Gln Tyr Leu Leu Lys Phe Asp Asn
260             265             270

Arg Gly Val Ile Thr Asn Ala Val Asp Cys Ser Ser Ser Phe Phe Ser
275             280             285

Glu Ile Gln Cys Lys Thr Lys Ser Leu Leu Pro Asn Thr Gly Val Tyr
290             295             300

Asp Leu Ser Gly Phe Thr Val Lys Pro Val Ala Thr Val His Arg Arg
305             310         315                 320

Ile Pro Asp Leu Pro Asp Cys Asp Ile Asp Lys Trp Leu Asn Asn Phe
            325             330                 335

Asn Val Pro Ser Pro Leu Asn Trp Glu Arg Lys Ile Phe Ser Asn Cys
            340             345                 350

Asn Phe Asn Leu Ser Thr Leu Leu Arg Leu Val His Thr Asp Ser Phe
            355             360                 365

Ser Cys Asn Asn Phe Asp Glu Ser Lys Ile Tyr Gly Ser Cys Phe Lys
    370             375             380

Ser Ile Val Leu Asp Lys Phe Ala Ile Pro Asn Ser Arg Arg Ser Asp
385             390             395                 400

Leu Gln Leu Gly Ser Ser Gly Phe Leu Gln Ser Ser Asn Tyr Lys Ile
            405             410                 415

Asp Thr Thr Ser Ser Ser Cys Gln Leu Tyr Tyr Ser Leu Pro Ala Ile
            420             425             430

Asn Val Thr Ile Asn Asn Tyr Asn Pro Ser Ser Trp Asn Arg Arg Tyr
            435             440             445

Gly Phe Asn Asn Phe Asn Leu Ser Ser His Ser Val Val Tyr Ser Arg
    450             455             460

Tyr Cys Phe Ser Val Asn Asn Thr Phe Cys Pro Cys Ala Lys Pro Ser
465             470             475                 480

Phe Ala Ser Ser Cys Lys Ser His Lys Pro Pro Ser Ala Ser Cys Pro
            485             490             495

Ile Gly Thr Asn Tyr Arg Ser Cys Glu Ser Thr Thr Val Leu Asp His
            500             505             510

Thr Asp Trp Cys Arg Cys Ser Cys Leu Pro Asp Pro Ile Thr Ala Tyr
            515             520             525

Asp Pro Arg Ser Cys Ser Gln Lys Lys Ser Leu Val Gly Val Gly Glu
    530             535             540

His Cys Ala Gly Phe Gly Val Asp Glu Glu Lys Cys Gly Val Leu Asp

53

545 550 555 560

Gly Ser Tyr Asn Val Ser Cys Leu Cys Ser Thr Asp Ala Phe Leu Gly
565 570 575

Trp Ser Tyr Asp Thr Cys Val Ser Asn Asn Arg Cys Asn Ile Phe Ser
580 585 590

Asn Phe Ile Leu Asn Gly Ile Asn Ser Gly Thr Thr Cys Ser Asn Asp
595 600 605

Leu Leu Gln Pro Asn Thr Glu Val Phe Thr Asp Val Cys Val Asp Tyr
610 615 620

Asp Leu Tyr Gly Ile Thr Gly Gln Gly Ile Phe Lys Glu Val Ser Ala
625 630 635 640

Val Tyr Tyr Asn Ser Trp Gln Asn Leu Leu Tyr Asp Ser Asn Gly Asn
645 650 655

Ile Ile Gly Phe Lys Asp Phe Val Thr Asn Lys Thr Tyr Asn Ile Phe
660 665 670

Pro Cys Tyr Ala Gly Arg Val Ser Ala Ala Phe His Gln Asn Ala Ser
675 680 685

Ser Leu Ala Leu Leu Tyr Arg Asn Leu Lys Cys Ser Tyr Val Leu Asn
690 695 700

Asn Ile Ser Leu Thr Thr Gln Pro Tyr Phe Asp Ser Tyr Leu Gly Cys
705 710 715 720

Val Phe Asn Ala Asp Asn Leu Thr Asp Tyr Ser Val Ser Ser Cys Ala
725 730 735

Leu Arg Met Gly Ser Gly Phe Cys Val Asp Tyr Asn Ser Pro Ser Ser
740 745 750

Ser Ser Ser Arg Arg Lys Arg Arg Ser Ile Ser Ala Ser Tyr Arg Phe
755 760 765

Val Thr Phe Glu Pro Phe Asn Val Ser Phe Val Asn Asp Ser Ile Glu
770 775 780

Ser Val Gly Gly Leu Tyr Glu Ile Lys Ile Pro Thr Asn Phe Thr Ile
785 790 795 800

Val Gly Gln Glu Glu Phe Ile Gln Thr Asn Ser Pro Lys Val Thr Ile
                805                     810                     815

Asp Cys Ser Leu Phe Val Cys Ser Asn Tyr Ala Ala Cys His Asp Leu
                820                     825                     830

Leu Ser Glu Tyr Gly Thr Phe Cys Asp Asn Ile Asn Ser Ile Leu Asp
                835                     840                     845

Glu Val Asn Gly Leu Leu Asp Thr Thr Gln Leu His Val Ala Asp Thr
    850                     855                     860

Leu Met Gln Gly Val Thr Leu Ser Ser Asn Leu Asn Thr Asn Leu His
865                     870                     875                     880

Phe Asp Val Asp Asn Ile Asn Phe Lys Ser Leu Val Gly Cys Leu Gly
                885                     890                     895

Pro His Cys Gly Ser Ser Ser Arg Ser Phe Phe Glu Asp Leu Leu Phe
                900                     905                     910

Asp Lys Val Lys Leu Ser Asp Val Gly Phe Val Glu Ala Tyr Asn Asn
                915                     920                     925

Cys Thr Gly Gly Ser Glu Ile Arg Asp Leu Leu Cys Val Gln Ser Phe
    930                     935                     940

Asn Gly Ile Lys Val Leu Pro Pro Ile Leu Ser Glu Ser Gln Ile Ser
945                     950                     955                     960

Gly Tyr Thr Thr Ala Ala Thr Val Ala Ala Met Phe Pro Pro Trp Ser
                965                     970                     975

Ala Ala Ala Gly Ile Pro Phe Ser Leu Asn Val Gln Tyr Arg Ile Asn
                980                     985                     990

Gly Leu Gly Val Thr Met Asp Val Leu Asn Lys Asn Gln Lys Leu Ile
        995                     1000                    1005

Ala Thr Ala Phe Asn Asn Ala Leu Leu Ser Ile Gln Asn Gly Phe
    1010                    1015                    1020

Ser Ala Thr Asn Ser Ala Leu Ala Lys Ile Gln Ser Val Val Asn
    1025                    1030                    1035

Ser Asn Ala Gln Ala Leu Asn Ser Leu Leu Gln Gln Leu Phe Asn
    1040                    1045                    1050

```
Lys Phe Gly Ala Ile Ser Ser  Ser Leu Gln Glu Ile  Leu Ser Arg
    1055            1060           1065

Leu Asp Ala Leu Glu Ala Gln  Val Gln Ile Asp Arg  Leu Ile Asn
    1070            1075           1080

Gly Arg Leu Thr Ala Leu Asn  Ala Tyr Val Ser Gln  Gln Leu Ser
    1085            1090           1095

Asp Ile Ser Leu Val Lys Phe  Gly Ala Ala Leu Ala  Met Glu Lys
    1100            1105           1110

Val Asn Glu Cys Val Lys Ser  Gln Ser Pro Arg Ile  Asn Phe Cys
    1115            1120           1125

Gly Asn Gly Asn His Ile Leu  Ser Leu Val Gln Asn  Ala Pro Tyr
    1130            1135           1140

Gly Leu Leu Phe Met His Phe  Ser Tyr Lys Pro Ile  Ser Phe Lys
    1145            1150           1155

Thr Val Leu Val Ser Pro Gly  Leu Cys Ile Ser Gly  Asp Val Gly
    1160            1165           1170

Ile Ala Pro Lys Gln Gly Tyr  Phe Ile Lys His Asn  Asp His Trp
    1175            1180           1185

Met Phe Thr Gly Ser Ser Tyr  Tyr Tyr Pro Glu Pro  Ile Ser Asp
    1190            1195           1200

Lys Asn Val Val Phe Met Asn  Thr Cys Ser Val Asn  Phe Thr Lys
    1205            1210           1215

Ala Pro Leu Val Tyr Leu Asn  His Ser Val Pro Lys  Leu Ser Asp
    1220            1225           1230

Phe Glu Ser Glu Leu Ser His  Trp Phe Lys Asn Gln  Thr Ser Ile
    1235            1240           1245

Ala Pro Asn Leu Thr Leu Asn  Leu His Thr Ile Asn  Ala Thr Phe
    1250            1255           1260

Leu Asp Leu Tyr Tyr Glu Met  Asn Leu Ile Gln Glu  Ser Ile Lys
    1265            1270           1275

Ser Leu Asn Asn Ser Tyr Ile  Asn Leu Lys Asp Ile  Gly Thr Tyr
    1280            1285           1290
```

56

```
Glu Met  Tyr Val Lys Trp Pro  Trp Tyr Val Trp Leu  Leu Ile Ser
    1295              1300              1305


Phe Ser  Phe Ile Ile Phe Leu  Val Leu Leu Phe Phe  Ile Cys Cys
    1310              1315              1320


Cys Thr  Gly Cys Gly Ser Ala  Cys Phe Ser Lys Cys  His Asn Cys
    1325              1330              1335


Cys Asp  Glu Tyr Gly Gly His  His Asp Phe Val Ile  Lys Thr Ser
    1340              1345              1350


His Asp  Asp
    1355
```

**Description**

TECHNICAL FIELD

The present invention relates to the field of the treatment or prevention of viral diseases, in particular to the identification of compounds enhancing said treatment or prevention.

BACKGROUND ART

After the appearance of first cases in Wuhan, China in December 2019, the novel human coronavirus disease, COVID-19, has become the first coronavirus pandemic in history. In mid May 2020 more than 5 million patients world-wide have been infected with the novel corona virus, SARS-CoV-2, and more than 330.000 global deaths related to COVID-19 have been reported.

The first descriptions of corona viruses date back to the 1930ties when they were isolated from chicken. Originally, coronaviruses were associated with important diseases in cattle, poultry, pigs and cats. They are large, enveloped, positive-stranded RNA viruses with a round structure and long, petal-shaped spikes protruding from their surface.

Coronaviruses can be divided in three serogroups of which groups I and II have been isolated from mammals and group III from birds. Members from group I and II (Group I: HCo-229E, HCoV-NL63; Group II: HCoV-OC43, HCoV-HKU1) have been known for decades as causes for relatively mild common colds in humans.

However, in 2003 Middle East respiratory syndrome (MERS) and in 2012 severe acute respiratory syndrome (SARS) were shown to be caused by novel corona viruses (SARS-CoV) and MERS-CoV, respectively which caused high death rates in up to 10% of infected people.

MERS-CoV uses dipeptidyl peptidase IV (DPP IV) as receptor.

SARS-CoV and SARS-CoV-2 use angiotensin-converting enzyme 2 (ACE2) on human cells as receptor and binds to it with its receptor binding domain (RBD). The RBD is located in the spike protein S within S1, the receptor-binding subunit close to the C-terminal S2 membrane fusion subunit. The clinical course of COVID-19 has a tri-phasic pattern with fever, cough, fatigue in week 1, dyspnoea, lymphopenia and pneumonia in week 2 and resolution in week 3. However, in severe cases thrombocytopenia, coagulopathy, acute kidney injury, myocardial injury, respiratory distress syndrome as well as deteriorating multi-organ dysfunction can occur.

Acute infection can be diagnosed by demonstrating the presence of virus-derived nucleic acid by RT-PCR in nasopharyngeal swabs in patients. However, there is currently no specific and effective treatment for COVID-19. Accordingly, quarantine, social distancing and enhanced hygiene precautions are the only measures to prevent virus spread.

It has been shown that COVID-19 patients develop SARS-CoV-2-specific antibodies but it is not known if and in how many infected subjects the virus-induced antibodies are protective. Indeed, it was reported that patients who had recovered from COVID-19 again presented with detectable positive SARS-CoV-2 RNA (Fu et al., J. Med. Virol., doi: 10.1002/jmv.25968 (2020)).

For detecting antibodies against SARS-CoV-2 in a patient's blood, virus neutralization tests (VNT) exist. However, such VNT require enhanced biosafety standards since they use live virus. Thus, VNT are cumbersome and time consuming. Due to drawbacks of virus neutralization tests, pseudovirus neutralization assay have been developed, which however still require cell cultures (Nie et al., Emerg. Microbes Infect. 9 (1): 680-686 (2020)).

An enzyme-linked immunosorbent assay (ELISA) for detecting neutralizing antibodies against SARS-CoV-2 has been described in Tan et al. (Natureresearch, doi: 10.21203/rs.3.rs-24574/v1 (2020)). The test described by Tan et al. seems to be useful for identifying subjects producing antibodies, which inhibit the binding of the receptor binding domain (RBD) of SARS-CoV-2 to its receptor ACE2 on human cells. However, there is a need also for tests which may identify antibodies leading to immune enhancement. In this context it has been reported that symptoms of COVID-19 may be increased by antibody-mediated enhancement and that this can be a serious problem for treatment with SARS-CoV-2-specific antibodies and for the development of vaccines which should not induce immune enhancing antibodies (de Alwis et al., EBioMedicine, 55:102768, doi: 10.1016/j.ebiom.2020.102768 (2020)).

There is thus a need for a reliable and simple method for studying compounds, which influence the binding of SARS-CoV, SARS-CoV-2 or MERS-CoV to its receptor. It is one object of the present invention to provide a method for identifying a compound which inhibits virus-receptor binding. It is also an object to provide a method for identifying a compound which enhances virus-receptor binding. In particular, one object of the present invention is to provide a method for identifying antibodies enhancing the binding of SARS-CoV, SARS-CoV-2 or MERS-CoV to its receptor. A further object of the present invention is to provide a method for assessing the suitability of a compound as a vaccine candidate against a virus-induced disease.

SUMMARY OF THE INVENTION

Thus, the present invention relates to a method for identifying a compound enhancing, not affecting or inhibiting the binding of a virus to a receptor protein of said virus comprising the steps of

a) incubating a first solid support comprising said receptor protein or a functional fragment thereof immobilized thereon with said virus or a fragment thereof;
b) incubating a second solid support comprising said receptor protein or functional fragment thereof immobilized thereon with said virus or fragment thereof in the presence of said compound;
c) determining the virus-receptor binding levels resulting from step a) and step b);
d) comparing the binding levels determined in step c),
e) identifying said compound as a compound enhancing virus-receptor binding if the binding level resulting from step b) is higher than the binding level resulting from step a),
f) identifying said compound as a compound not affecting virus-receptor binding if the binding level resulting from step b) is substantially identical to the binding level resulting from step a), and
g) identifying said compound as a compound inhibiting virus-receptor binding if the binding level resulting from step b) is lower than the binding level resulting from step a).

It turned surprisingly out that the method of the present invention allows to determine whether a compound enhances, does not affect/influence or inhibits the binding of a virus to a receptor protein of said virus. This information about a compound is useful to determine whether a compound can be used in the treatment of a disease caused by viruses. Typically used neutralisation assays are not able to recognize whether a compound has one of the aforementioned properties.

The virus used in the inventive method can be any virus, whereby a coronavirus, preferably SARS-CoV, SARS-CoV-2, MERS-CoV or a fragment thereof, are most preferred. The inventive method is useful for identifying a compound, which influences the binding of a virus to a receptor protein of said virus. Said influence may be an enhancement or inhibition of the binding of a virus to a receptor protein of said virus. Additionally, the inventive method can be used to identify a compound, which does not influence the virus-receptor binding.

The inventive method thus allows to specifically study virus-receptor binding and the influence of a compound on said binding.

Surprisingly, it turned out that the inventive method is particularly useful for identifying a compound enhancing the binding of a virus to a receptor protein of said virus. Due to the enhancement of the virus-receptor binding, the attachment of a virus particle on the surface of a cell can be promoted. In consequence, a significantly increased amount of virus particles is able to pass the cell barrier, which can intensify a virus-induced disease.

It is essential to identify compounds enhancing the binding of a virus to a receptor protein of said virus in order to exclude such compounds as a potential vaccine candidate or active agent in a medicament against a virus-induced disease. Such compound may be an antibody which enhances virus-receptor interaction. Said antibody may be present in the serum of a patient.

The present invention therefore further provides a method for discriminating between compounds enhancing the binding of a virus to a receptor protein of said virus and compounds inhibiting or not influencing said binding from a set of compounds, comprising the steps of

a) incubating a first solid support comprising said receptor protein or a functional fragment thereof immobilized thereon with said virus or a fragment thereof;
b) incubating a further solid support comprising said receptor protein or functional fragment thereof immobilized thereon with said virus or fragment thereof in the presence of a compound from said set of compounds;
c) repeating step b) for further compounds from said set of compounds;
d) determining the virus-receptor binding levels resulting from steps a) to c);
e) comparing the binding levels determined in step d), and
f) discriminating a compound enhancing virus-receptor binding from a compound inhibiting virus-receptor binding by evaluating if the binding level determined in the presence of said compound is higher compared to the binding level determined in the absence of one of said compounds.

Preferably, said compounds are antibodies. The inventive method thus allows discriminating an antibody enhancing virus-receptor binding from an antibody which inhibits or does substantially not affect the virus-receptor binding. An antibody does not influence the virus-receptor binding if the binding level determined in the presence of said antibody is substantially identical to the binding level determined in the absence of said antibody.

The present invention further provides a method for assessing the suitability of a compound as vaccine candidate against a virus-induced disease comprising the steps of

> a) performing the method for identifying a compound enhancing or inhibiting the binding of a virus to a receptor protein of said virus as described herein;
> b) identifying said compound as a suitable vaccine candidate against a disease induced by said virus if said compound inhibits virus-receptor binding, and
> c) identifying said compound as an unsuitable vaccine candidate against a disease induced by said virus if said compound enhances virus-receptor binding.

The present invention thus provides a surprisingly robust and efficient method for testing a large number of vaccine candidates and is particularly useful to identify compounds, which enhance virus-receptor binding and are therefore unsuitable as vaccination candidates.

The present invention further provides a kit for identifying a compound enhancing or inhibiting the binding of a virus to a receptor protein of said virus comprising

> a) a solid support coated with said receptor protein or a functional fragment thereof, and
> b) said virus or a fragment thereof
> c) at least one detection molecule or labelling molecule.

This kit is particularly useful to identify a compound enhancing or inhibiting virus-receptor binding. The inventive kit can also be used to assess the suitability of a compound as vaccine candidate against a virus-induced disease.

DESCRIPTION OF EMBODIMENTS

As used herein, the phrase "binding of a virus or fragment thereof to a receptor protein of said virus or a functional fragment thereof" or "binding of a virus to a receptor protein of said virus" is also referred to as "virus-receptor binding".

As used herein, a "fragment of a virus" comprises or consists of a stretch of a viral protein, preferably a capsid protein, which contains less amino acid residues than the full length of said viral protein and which comprises a domain binding to the viral receptor ("receptor binding domain") .

As used herein, a "virus receptor protein" can be any protein or polypeptide naturally present on the surface of a cell which is able to bind to a virus (particle) or a fragment thereof. The term "functional fragment of a virus receptor protein" refers to a fragment of less than the full length of the receptor protein, which maintains at least the virus specific binding function of the virus receptor protein and, thus, comprises at least the binding domain of the virus receptor protein to a virus or fragment thereof.

A "binding level" is "substantially identical" to another binding level, when the respective differences of the binding levels are within 10%, preferably within 5%.

According to the present invention, a solid support comprising a receptor protein or a functional fragment thereof immobilized thereon is incubated with a virus, which can bind to said receptor protein. The solid support may be any solid carrier suitable for immobilizing a receptor protein. The solid support may be of any suitable shape, e.g. flat or spherical, or material, e.g. polymer or glass. Preferably, the solid support is a plate for performing an enzyme-linked immunosorbent assay (ELISA). The solid support may also be sepharose beads or nitrocellulose. On said solid support, more than one type of receptor protein may be immobilized.

In one aspect, the virus or a fragment thereof can be immobilized instead of the receptor protein on the solid support. In this aspect, a virus or fragment thereof immobilized on a solid support is incubated with soluble receptor protein or a functional fragment thereof. Said incubation may be performed in the presence of a compound influencing virus-receptor binding.

The phrase "determining the binding level" or "determining the binding levels" as used herein refers to quantifying the amount of virus or fragment thereof that binds to its receptor protein or functional fragment thereof.

A compound inhibits virus-receptor binding when a reduced binding level is determined in the presence of said compound when compared to the binding level determined in the presence of a lower amount of said compound, or in the absence of said compound. A compound enhances virus-receptor binding when an increased binding level is determined in the presence of said compound when compared to the binding level determined in the presence of a lower amount of said compound, or in the absence of said compound. A compound does not affect virus-receptor binding when a substantially identical binding level is determined in the presence of said compound when compared to the binding level determined in the presence of a lower amount of said compound, or in the absence of said compound.

For determining the virus-receptor binding level, a detection molecule or labelling molecule may be used according to the present invention. In a preferred embodiment of the present invention, an enzyme-linked immunosorbent assay (ELISA) interaction assay is performed.

As used herein, the term "detection molecule" refers to a molecule which binds to receptor-bound virus and allows quantifying the amount of virus or fragment thereof bound to its receptor protein or functional fragment thereof. Said detection molecule may be called a "first detection molecule" and may be combined with one or more further detection molecules, e.g. a secondary detection molecule. Said first and further detection molecules may be antibodies. For example, virus-bound receptor may be detected using a first detection antibody binding to virus-bound receptor protein, preferably in combination with a secondary detection molecule. Said secondary detection molecule may be an antibody binding to said first detection molecule and may be labelled with an enzyme, wherein said enzyme converts a substrate and thereby induces a colour reaction. Such colour reaction may be analysed spectroscopically to quantify the amount of virus or fragment thereof binding to its receptor.

Preferably, said first detection antibody binds to the virus outside the receptor binding domain of said virus. In other words, the detection molecule is preferably an antibody or fragment thereof, which does not inhibit the binding of a virus or fragment thereof to its receptor protein or functional fragment thereof. The technical effect of using as a detection molecule an antibody binding to the virus outside the receptor binding domain of said virus is an increased detection sensitivity, since any interference of the detection molecule with virus-receptor binding is avoided. In other words, the detection molecule does not itself inhibit the virus-receptor binding, which is advantageous since such inhibition would distort the measurement accuracy of the binding level.

Alternatively, detection may be performed by using a labelling molecule. As used herein, a "labelling molecule" refers to a molecule which is covalently linked to said virus or fragment thereof. In a specific embodiment, the labelling molecule is biotin and the binding level is determined by quantifying the amount of biotin-labelled receptor-bound virus. Said quantification may for example be performed by using Streptavidin-horseradish peroxidase (HRP) or Avidin-HRP protein conjugates, which specifically react with biotin and induce a colour reaction. The technical effect of using a labelling molecule for the detection is that virus-receptor binding can be monitored directly without the need of a secondary detection molecule. According to the present invention, the covalent bond between the labelling molecule and the virus strain is located outside the receptor binding domain to avoid any interference of the labelling molecule with virus-receptor binding. Thus, high measurement sensitivity and accuracy of the binding level is achieved.

According to a preferred embodiment of the present invention, said virus is a coronavirus strain or fragment thereof and said receptor protein is a coronavirus receptor protein or functional fragment thereof.

Preferably, the virus is SARS-CoV, SARS-CoV-2 or MERS-CoV or a fragment thereof, even more preferably the virus is SARS-CoV-2 or the receptor binding domain thereof.

In a preferred embodiment, the virus is SARS-CoV, SARS-CoV-2, or a fragment thereof, and the receptor protein is human angiotensin-converting enzyme 2 (ACE2) or a functional fragment thereof.

According to another embodiment, the virus is MERS-CoV or a fragment thereof, the receptor protein is human dipeptidyl peptidase IV (DPP IV) or a functional fragment thereof.

According to the present invention, the fragment of SARS-CoV, SARS-CoV-2 or MERS-CoV comprises the receptor binding domain (RBD) of SARS-CoV, SARS-CoV-2 or MERS-CoV. For example, the fragment is the S protein or the RBD of SARS-CoV-2.

In a preferred embodiment according to the present invention, the virus or fragment thereof is RBD of SARS-CoV or SARS-CoV-2 comprising a tag, preferably a histidine (His)-tag. The technical effect of applying His-tagged RBD of SARS-CoV or SARS-CoV-2 in the inventive method is that the His-tag can be used for detecting the virus-receptor binding level in a straightforward and simple way. ACE2-bound His-tagged RBD of SARS-CoV-2 or SARS-CoV can be quantified by using an anti-His detection antibody and an enzyme-labelled secondary detection antibody. In a specific embodiment described herein, said anti-His detection antibody is mouse monoclonal anti-His antibody and said enzyme-labelled secondary detection antibody is anti-mouse IgG$_1$ antibody labelled with horseradish peroxidase (HRP).

Apart from a His-tag, the RBD may comprise any other tag suitable for detecting the virus-receptor binding level.

According to the present invention, determination of virus-receptor binding levels via the His-tag of the RBD of SARS-CoV or SARS-CoV-2 is superior over using directly labelled RBD with a labelling molecule, since such labelling molecule may occupy parts of the RBD important for virus-receptor binding.

In one embodiment of the present invention, the virus or fragment thereof is pre-incubated with a compound enhancing or inhibiting the virus-receptor binding prior to the incubating step b) of the inventive method.

A compound which enhances the binding of a virus to a receptor protein of said virus is herein also referred to as an "immune-enhancing compound".

According to the present invention, the compound enhancing or inhibiting the virus-receptor binding may be a molecule binding to the virus or a fragment thereof or it may be a molecule binding to the receptor protein or a functional fragment thereof.

If said compound is a molecule binding to the receptor protein or a functional fragment thereof, it is particularly preferred that the solid support comprising said receptor protein or functional fragment thereof immobilized thereon is pre-incubated with said compound prior to incubation with the virus or fragment thereof.

If said compound is a molecule binding to the virus or a fragment thereof it is particularly preferred that said virus is pre-incubated with said compound prior to incubating the solid support comprising the virus receptor protein or functional fragment thereof immobilized thereon with said virus or fragment thereof. If said compound binds to said virus or fragment thereof in the pre-incubation step, the virus-receptor binding level will be influenced. For example, the binding level may be reduced or increased compared to the binding level detected in the absence of said compound.

According to the present invention, a compound inhibiting virus-receptor binding may be a neutralizing antibody. Preferably, said compound is a neutralizing antibody directed against SARS-CoV, SARS-CoV-2, MERS-CoV, or a fragment thereof.

In a preferred embodiment, said compound is an immune-enhancing antibody. Herein, the term "immune-enhancing antibody" refers to an antibody which enhances virus-receptor binding. Preferably, said immune-enhancing antibody is directed to SARS-CoV, SARS-CoV-2 or MERS-CoV, even more preferably directed to SARS-CoV-2.

Preferably, said immune-enhancing antibody is an immunoglobulin G (IgG), an immunoglobulin M (IgM) or an immunoglobulin A (IgA). Most, preferably, said immune-enhancing antibody is IgG. The immune-enhancing antibody may be present in a serum sample of a patient. The present invention allows to reliably identify immune-enhancing antibodies.

The present invention further provides a method for assessing the suitability of a compound as vaccine candidate against a virus-induced disease comprising the steps of

a) performing the method for identifying a compound enhancing or inhibiting the binding of a virus to a receptor protein of said virus as described herein;
b) identifying said compound as a suitable vaccine candidate against a disease induced by said virus if said compound inhibits virus-receptor binding, and
c) identifying said compound as an unsuitable vaccine candidate against a disease induced by said virus if said compound enhances virus-receptor binding.

In a preferred embodiment, said vaccine candidate is against a coronavirus-induced disease, preferably against Covid-19.

The present invention further provides a kit for identifying a compound enhancing or inhibiting the binding of a virus to a receptor protein of said virus comprising

a) a solid support coated with said receptor protein or a functional fragment thereof;
b) said virus or a fragment thereof, and
c) at least one detection molecule or labelling molecule as described herein.

In one aspect of the inventive kit, said virus is a coronavirus strain or fragment thereof and said receptor protein is a coronavirus receptor protein or functional fragment thereof.

In a preferred aspect of the inventive kit, said virus is SARS-CoV, SARS-CoV-2, or a fragment thereof, and said receptor protein is angiotensin-converting enzyme 2 (ACE2) or a functional fragment thereof.

In another aspect, said virus is MERS-CoV or a fragment thereof, and said receptor protein is dipeptidyl peptidase IV (DPP IV) or a functional fragment thereof.

According to one aspect of the inventive kit said fragment of said virus comprises the receptor binding domain (RBD) of SARS-CoV, SARS-CoV-2 or MERS-CoV.

The present invention further allows for the use of a kit as described herein for identifying a compound neutralizing the binding of a virus to a receptor protein of said virus.

The present invention further allows for the use of a kit as described herein for identifying a compound enhancing the binding of a virus to a receptor protein of said virus.

The present invention moreover allows for the use a kit as described herein for assessing the suitability of a compound as vaccine candidate against a virus-induced disease.

The present invention is further illustrated by the following embodiments, figures and examples, however, without being restricted thereto.

Preferred embodiments:

1. A method for identifying a compound enhancing, not affecting or inhibiting the binding of a virus to a receptor protein of said virus comprising the steps of

a) incubating a first solid support comprising said receptor protein or a functional fragment thereof immobilized thereon with said virus or a fragment thereof;

b) incubating a second solid support comprising said receptor protein or functional fragment thereof immobilized thereon with said virus or fragment thereof in the presence of said compound;

c) determining the virus-receptor binding levels resulting from step a) and step b);

d) comparing the binding levels determined in step c),

e) identifying said compound as a compound enhancing virus-receptor binding if the binding level resulting from step b) is higher than the binding level resulting from step a),

f) identifying said compound as a compound not affecting virus-receptor binding if the binding level resulting from step b) is substantially identical to the binding level resulting from step a), and

g) identifying said compound as a compound inhibiting virus-receptor binding if the binding level resulting from step b) is lower than the binding level resulting from step a).

2. A method for discriminating between compounds enhancing the binding of a virus to a receptor protein of said virus and compounds inhibiting or not influencing said binding from a set of compounds, comprising the steps of

a) incubating a first solid support comprising said receptor protein or a functional fragment thereof immobilized thereon with said virus or a fragment thereof;

b) incubating a further solid support comprising said receptor protein or functional fragment thereof immobilized thereon with said virus or fragment thereof in the presence of a compound from said set of compounds;

c) repeating step b) for further compounds from said set of compounds;

d) determining the virus-receptor binding levels resulting from steps a) to c);

e) comparing the binding levels determined in step d), and

f) discriminating a compound enhancing virus-receptor binding from a compound inhibiting virus-receptor binding by evaluating if the binding level determined in the presence of said compound is higher compared to the binding level determined in the absence of one of said compounds.

3. The method of embodiment 1 or 2, wherein said virus is a coronavirus strain or fragment thereof and said receptor protein is a coronavirus receptor protein or functional fragment thereof.

4. The method of any one of embodiments 1 to 3, wherein said virus is SARS-CoV, SARS-CoV-2, or a fragment thereof, and wherein said receptor protein is angiotensin-converting enzyme 2 (ACE2) or a functional fragment thereof.

5. The method of any one of embodiments 1 to 3, wherein said virus is MERS-CoV or a fragment thereof, and wherein said receptor protein is dipeptidyl peptidase IV (DPP IV) or a functional fragment thereof.

6. The method of any one of embodiments 1 to 5, wherein said fragment of said virus comprises the receptor binding domain (RBD) of SARS-CoV, SARS-CoV-2 or MERS-CoV.

7. The method of any one of embodiments 1 to 6, wherein said virus or fragment thereof is histidine (His)-tagged RBD of SARS-CoV or SARS-CoV-2 and said His-tagged RBD is detected by using an anti-His detection antibody and an enzyme-labelled secondary detection antibody.

8. The method of embodiment 7, wherein said anti-His detection antibody is mouse monoclonal anti-His antibody and said enzyme-labelled secondary detection antibody is anti-mouse IgG1 antibody labelled with horseradish peroxidase (HRP).

9. The method of any one of embodiments 1 or 3 to 8, wherein said virus or fragment thereof is pre-incubated with said compound prior to step b).

10. The method of any one of embodiments 1 or 3 to 9, wherein said compound is a neutralizing antibody.

11. The method of any one of embodiments 1 or 3 to 9, wherein said compound is an immune-enhancing antibody.

12. The method of any one of embodiments 1 or 3 to 11, wherein said compound is an antibody specific for the RBD of SARS-CoV or SARS-CoV-2.

13. The method of embodiment 12, wherein said antibody is an IgG antibody.

14. A method for assessing the suitability of a compound as vaccine candidate against a virus-induced disease comprising the steps of

a) performing the method of any one of embodiments 1 to 13,

b) identifying said compound as a suitable vaccine candidate against a disease induced by said virus if said compound inhibits virus-receptor binding, and

c) identifying said compound as an unsuitable vaccine candidate against a disease induced by said virus if said compound enhances virus-receptor binding.

15. The method of embodiment 14, wherein said vaccine candidate is against a coronavirus-induced disease, preferably against Covid-19.

16. A kit for identifying a compound enhancing or inhibiting the binding of a virus to a receptor protein of said virus comprising

    a) a solid support coated with said receptor protein or a functional fragment thereof;
    b) said virus or a fragment thereof, and
    c) at least one detection molecule or labelling molecule.

17. The kit of embodiment 16, wherein said virus is a coronavirus strain or fragment thereof and said receptor protein is a coronavirus receptor protein or functional fragment thereof.

18. The kit of embodiment 16 or 17, wherein said virus is SARS-CoV, SARS-CoV-2, or a fragment thereof, and wherein said receptor protein is angiotensin-converting enzyme 2 (ACE2) or a functional fragment thereof.

19. The kit of embodiment 17 to 18, wherein said virus is MERS-CoV or a fragment thereof, and wherein said receptor protein is dipeptidyl peptidase IV (DPP IV) or a functional fragment thereof.

20. The kit of any one of embodiments 16 to 19, wherein said fragment of said virus comprises the receptor binding domain (RBD) of SARS-CoV, SARS-CoV-2 or MERS-CoV.

21. Use of a kit according to any of embodiments 16 to 20 for identifying a compound neutralizing the binding of a virus to a receptor protein of said virus.

22. Use of a kit according to any one of embodiments 17 to 20 for identifying a compound enhancing the binding of a virus to a receptor protein of said virus.

23. Use of a kit according to any one of embodiments 17 to 22 for assessing the suitability of a compound as vaccine candidate against a virus-induced disease.

BRIEF DESCRIPTION OF THE FIGURES

**Fig. 1:** IgG (upper panel) and IgM (lower panel) reactivity (y-axes: OD values corresponding to bound immunoglobulin) to S and RBD determined for COVID-19 convalescent patients (group B: B001-B032, right) and for individuals from a historic control group before the pandemic (group P: P001-P023, left).

**Fig. 2:** IgA reactivity (y-axes: OD values correspond to bound immunoglobulins) to S and RBD determined for COVID-19 convalescent patients (group B: B001-B00X) and for individuals from the historic control group P (P001, P002, P003, P00X).

**Fig. 3:** Amino acid sequence and scheme of the S1 protein of SARS-CoV-2 (SEQ ID No. 26). The hydrophobic leader peptide is indicated in italics, S1 peptides (SEQ ID No. 1-25) are underlined and numbered according to Table 2 and the RBD is printed in bold. Glycosylation sites are boxed.

**Fig. 4:** Correlations between S-specific IgM- (y-axis: optical density levels) and IgG antibody levels (x-axes: optical density OD levels) (left) and RBD-specific IgM- (y-axis: optical density levels) and IgG antibody levels (x-axes: optical density OD levels) (right) in COVID-19 convalescent subjects. r and p levels are indicated.

**Fig. 5:** Correlations between S- and RBD-specific antibody levels and age of COVID-19-convalescent subjects. Shown are the correlations between age (x-axes) and S-specific IgG levels (y-axis, OD levels) (left upper part), S-specific IgM levels (left lower part), RBD-specific IgG levels (right upper part) and RBD-specific IgM levels (right lower part). r and p levels are indicated.

**Fig. 6:** Correlations between (A) RBD-specific IgG antibody levels (y-axis) and duration of symptoms (x-axis: days) and (B) between RBD-specific IgM antibody levels (y-axis) and duration of symptoms (x-axis: days). r and p levels are indicated.

**Fig. 7:** IgG, IgM and IgA reactivity (y-axes: OD values correspond to bound antibodies) to S, RBD and spike protein-derived synthetic peptides (1-25) (x-axes) determined for COVID-19 convalescent subjects (group B: B001, B002, B003, B00X).

**Fig. 8:** IgG, IgM and IgA reactivity (y-axes: OD values correspond to bound antibodies) to S, RBD and spike protein-derived synthetic peptides (1-25) (x-axes) determined for sera from subjects obtained before the COVID-19 pandemic (group P: P001, P002, P003, P00X).

**Fig. 9:** Alignment of the amino acid sequence of the S protein from SARS-CoV-2 with that of SARS-CoV. Identical amino acids are indicated by asterisks, dashes indicate gaps. Synthetic overlapping peptides spanning S1 are boxed and numbered (1-25) and the RBD is indicated in bold letters.

**Fig. 10:** Alignment of the amino acid sequence of the S protein from SARS-CoV-2 with that of HCoV-OC43. Identical amino acids are indicated by asterisks, dashes indicate gaps. Synthetic overlapping peptides spanning S1 are boxed and numbered (1-25) and the RBD is indicated in bold letters.

**Fig. 11:** Alignment of the amino acid sequence of the S protein from SARS-CoV-2 with that of HCoV-NL63. Identical amino acids are indicated by asterisks, dashes indicate gaps. Synthetic overlapping peptides spanning S1 are boxed and numbered (1-25) and the RBD is indicated in bold letters.

**Fig. 12:** Alignment of the amino acid sequence of the S protein from SARS-CoV-2 with that of HCoV-HKU1. Identical amino acids are indicated by asterisks, dashes indicate gaps. Synthetic overlapping peptides spanning S1 are boxed and numbered (1-25) and the RBD is indicated in bold letters.

**Fig. 13:** Alignment of the amino acid sequence of the S protein from SARS-CoV-2 with that of HCo-229-E. Identical amino acids are indicated by asterisks, dashes indicate gaps. Synthetic overlapping peptides spanning S1 are boxed and numbered (1-25) and the RBD is indicated in bold letters.

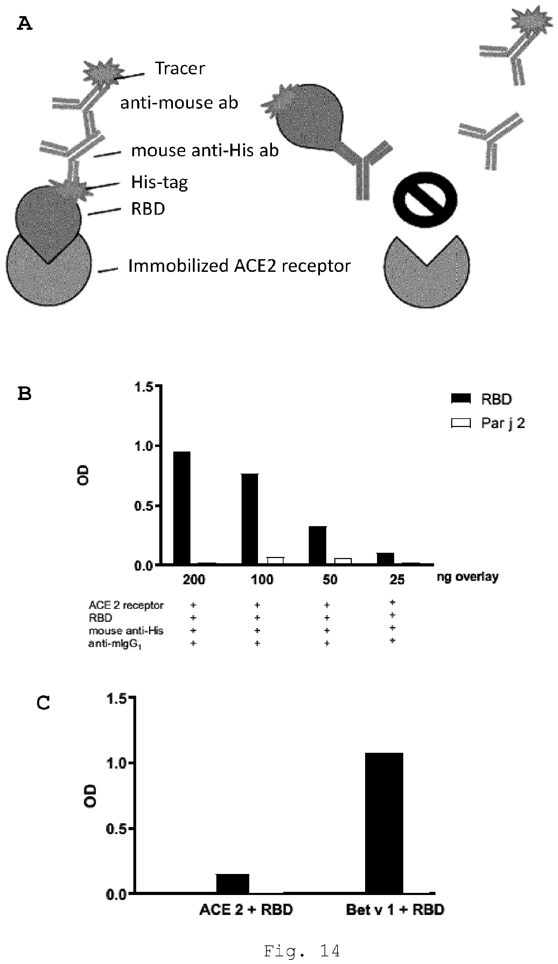

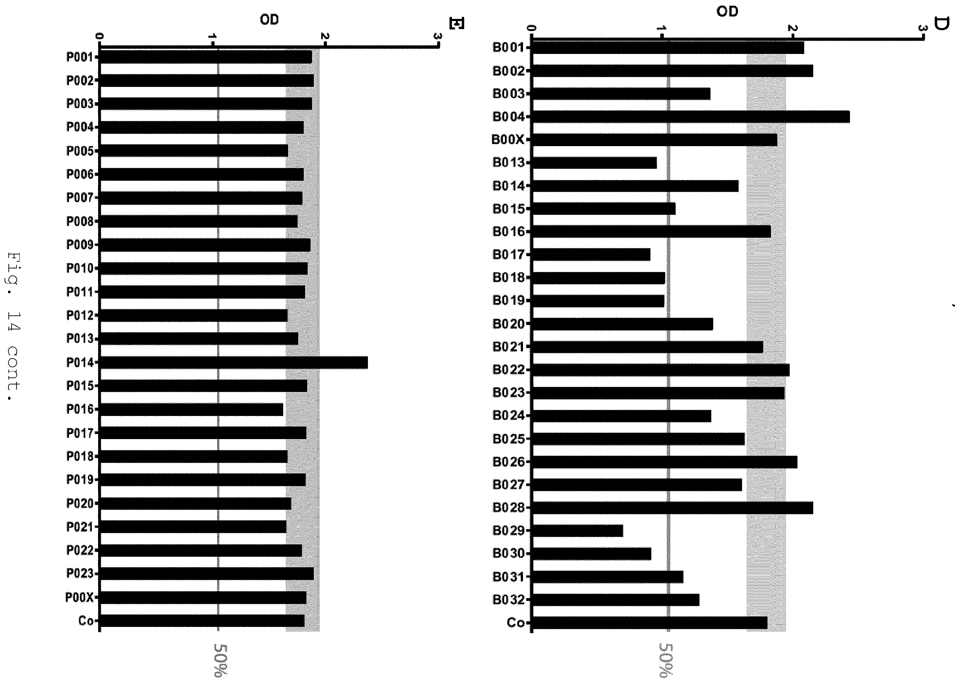

**Fig. 14:** Molecular interaction assay based on ACE2 and SARS-CoV-2 RBD. **(A)** Scheme of the molecular interaction assay. ELISA plate-bound recombinant ACE2 is incubated with His-tagged recombinant SARS-CoV-2 RBD which is detected with a mouse monoclonal anti-His-tag antibody followed by HRP-labelled anti-mouse antibodies. **(B)** Specific binding of three different concentrations of RBD versus a control protein (Par j 2) (y-axis: optical density OD values correspond to bound RBD) to ACE2. Reactants and concentrations in ng/ml are summarized below the x-axis. **(C)** Inhibition of RBD binding (y-axis: OD values) to plate-bound ACE2 by soluble ACE2 (ACE2 + RBD) versus a control protein (Bet v 1 + RBD). **(D)** Effects of serum antibodies from COVID-19 convalescent subjects (group B) and (**E**) from subjects obtained before the COVID pandemic (group P, historic controls) on the ACE2-RBD interaction. Shown is the binding of RBD to ACE2 (y-axis: OD values correspond to amounts of ACE2-bound RBD) which had been pre-incubated with sera or buffer without serum (Co) (x-axis). Results are shown as mean values with a deviation of <5% of duplicate determinations. The grey horizontal threshold area represents the 10% variability of the assay as determined in three independent runs. The horizontal bar below indicates a 50% inhibition of binding.

**Fig. 15:** Correlations between S- and RBD-specific IgG antibody levels (y-axes: optical density OD levels) and percentages of inhibition of RBD binding to ACE2 (x-axes) (upper panels) and S- and RBD-specific IgM (y-axes: OD levels) and percentages of inhibition of RBD binding to ACE2 (x-axes) (lower panels) in COVID-convalescent subjects. r and p levels are indicated.

**Fig. 16:** Correlations between percentages of inhibition of RBD binding to ACE2 (y-axes) and duration of symptoms (x-axis) (left panel) and age (x-axis) (right panel) in COVID-convalescent subjects. r and p levels are indicated.

EXAMPLE

**Materials and Methods**

Recombinant proteins and peptides

Recombinant insect cell-expressed SARS-CoV-2 spike protein (S1+S2, His tagged) was purchased from Sino Biological Inc., Beijing, P.R.China. SARS-CoV-2 spike protein receptor binding domain (RBD, His tagged) and human ACE2 Receptor (Fc-tagged) were purchased from GenScript, New Jersey, U.S.

Overlapping 25-35mer peptides covering the amino acid sequence of SARS-CoV-2 spike protein S1 domain were produced by solid-phase-synthesis. Recombinant His-tagged *Parietaria* allergen Par j 2 was expressed and purified as described (Dorofeeva et al., Sci Rep. 9: 15043 (2019)), and served as a control protein for RBD because it contains also cysteine residues. Recombinant major birch pollen allergen, Bet v 1 was purchased from Biomay, Austria and served as control protein for ACE2 because it also assumes a mixed alpha helical and beta sheet structure.

Sera from COVID-19 convalescent subjects and control subjects

Sera from COVID-19 convalescent subjects were obtained 10 weeks after confirmation of a SARS-CoV-2 infection by PCR when the subjects had recovered completely (Table 1, group B: n=25). Sera from subjects who had COVID-like symptoms but no PCR testing had been performed were obtained at least 10 weeks after convalescence. The study was approved by the Ethics committee of the Medical University of Vienna, Austria (EK 1302/2020) and signed informed consent was obtained from the study subjects. Anonymized sera from subjects which had been obtained before the COVID-19 pandemic were from the serum bank of the Division of Immunopathology (Table 1, group P: n=24), Department of Pathophysiology and Allergy Research, Medical University of Vienna which had been collected with permission of the Ethics committee of the Medical University of Vienna (EK1641/2014).

Table 1. Characterization of subjects

| Subject | gender[1] (f 11/m 14) | age (range 18-70) median 52.2 | Duration of symptoms[2] [days] | Subject | gender[1] (f 13/ m 11) | age (range 18-68) median 43.2 | Duration of symptoms[2] [days] |
|---|---|---|---|---|---|---|---|
| B001 | f | 43 | 19 | P001 | m | 18 | n.a. |
| B002 | f | 49 | 17 | P002 | m | 36 | n.a. |
| B003 | m | 57 | 10 | P003 | m | 29 | n.a. |
| B004 | m | 37 | 10 | P004 | m | 38 | n.a. |
| B00X | f | 56 | 11 | P005 | f | 28 | n.a. |
| B013 | m | 48 | 14 | P006 | f | 28 | n.a. |
| B014 | f | 42 | 5 | P007 | f | 39 | n.a. |
| B015 | m | 41 | 14 | P008 | m | 56 | n.a. |
| B016 | m | 63 | 5 | P009 | m | 69 | n.a. |
| B017 | m | 60 | 0 | P010 | f | 60 | n.a. |
| B018 | f | 70 | 23 | P011 | f | 43 | n.a. |
| B019 | m | 70 | 5 | P012 | m | 24 | n.a. |
| B020 | f | 58 | 21 | P013 | m | 47 | n.a. |
| B021 | m | 61 | 13 | P014 | m | 49 | n.a. |
| B022 | m | 60 | 21 | P015 | f | 43 | n.a. |
| B023 | f | 57 | 20 | P016 | f | 33 | n.a. |
| B024 | f | 59 | 19 | P017 | m | 61 | n.a. |
| B025 | f | 18 | 14 | P018 | m | 59 | n.a. |
| B026 | m | 33 | 15 | P019 | f | 42 | n.a. |
| B027 | m | 28 | 4 | P020 | f | 50 | n.a. |
| B028 | m | 78 | 10 | P021 | f | 45 | n.a. |
| B029 | m | 67 | 8 | P022 | f | 51 | n.a. |

| B030 | m | 58 | 14 | P023 | f | 40 | n.a. |
|---|---|---|---|---|---|---|---|
| B031 | f | 56 | 18 | P00X | f | 50 | n.a. |
| B031 | f | 38 | 10 | | | | |

[1]f = female, m = male

[2]Duration of self-reported COVID-19 symptoms, n.a.=not applicable

Direct ELISA

Immunoglobulin response to SARS-CoV-2 S protein and peptides were measured by ELISA. S protein (Sino Biological), RBD (GenScript) and peptides (2 μg/ml) in bicarbonate buffer were coated overnight onto NUNC Maxisorb 96 well plates (Thermofisher, Thermo-Fisher Scientific, Waltham, 354 MA, USA). Plates were washed 3 times with washing buffer (PBS, 0.05% Tween 20) and subsequently blocked for 2 hours with blocking buffer (PBS, 0.05% Tween 20, 3% BSA). Serum samples 1:20 diluted were applied and incubated for 2 hours.

To determine human IgG reactivity, plates were washed 3 times and incubated with 1: 1000 diluted HRP-conjugated anti-human IgG (BD, San Jose, CA, USA) for 2 hours. After washing 3 times, the plates were developed with ABTS salt (Sigma-Aldrich, St. Louis, MO, USA) optical density was measured at 405/490 nm with Infinite F50 ELISA reader (Tecan, Männedorf, Switzerland).

For detection of human IgM and IgA, the plates were washed 3 times after incubation with serum samples (1:20) and incubated over night with either 1:1000 diluted purified mouse anti-human IgM (BD) or 1:1000 diluted purified mouse anti-human IgA (BD). After washing three times, plates were incubated for 2 hours with 1:1000 diluted HRP-linked anti mouse $IgG_1$ antibody (GE Healthcare, Marlborough, MA, USA), developed with ABTS and optical density was measured. The mean optical density (O.D.) values corresponding to the amount of bound immunoglobulins were measured at 405 nm and 492 nm (reference) in a TECAN Infinite F5 ELISA reader with the integrated software i-control 2.0 (Tecan Group Ltd., Männedorf, Switzerland). For each protein or peptide the corresponding buffer control plus three times standard deviation was subtracted. All determinations were performed in duplicates and results are shown as mean values with a variation of <5%.

Interaction assay

ACE2 receptor (GenScript) was coated (2 μg/ml) in bicarbonate buffer overnight onto NUNC 375 Maxisorb 96 well plates (Thermofisher). Plates were washed 3 times with washing buffer and subsequently blocked for 3 hours at RT with blocking buffer. Meanwhile serum samples were diluted 1:2 in PBS, 0.05% Tween 20, 1% BSA and incubated for 2 hours with 100 ng His-tagged RBD (GenScript). For control purposes, 10 μg/ml ACE2 receptor and 10 μg/ml Bet v 1 were pre-incubated with 100 ng His-tagged RBD.

The overlay was performed by adding the pre-incubated RBD samples to the coated and blocked ACE2 receptor followed by incubation for 3 hours. The plates were washed and incubated overnight with 1:1000 diluted mouse anti-His tag antibody (Dianova, Hamburg, Germany). After washing three times, 1:1000 diluted HRP-linked anti-mouse $IgG_1$ antibody (GE Healthcare) was incubated 2 hours and detected by ABTS. The mean optical density (O.D.) values corresponding to the amount of bound RBD were measured at 405 nm and 492 nm (reference) in a TECAN Infinite F5 ELISA reader with the integrated software i-control 2.0 (Tecan Group Ltd., Männedorf, Switzerland). From each measurement the buffer control (overlay without RBD) was subtracted. All determinations were performed in duplicates and results are shown as mean values with a variation of <5%. The percentages of inhibition were calculated as follows:

$$\text{Percentage inhibition}(\%) = (\text{OD}_{Bet\ v1} - \text{OD}_{Serum}) / (\text{OD}_{Bet\ v1}\ \text{OD}_{ACE2}) \times 100$$

Statistical evaluation

Correlations between specific antibody levels, percentages of inhibition and duration of symptoms were assessed by Spearman's correlation coefficient (r) using GraphPad Prism software, version 7.00 for Windows, GraphPad Software, La Jolla, Calif. P values < 0.05 were considered as significant.

Sequence alignment

Sequence alignment of SARS-CoV-2 spike protein (Genbank accession: QHD43416.1; SEQ ID No. 27) with SARS-CoV spike protein (UniProtKB accession: P59594.1; SEQ ID No. 28) and spike proteins from human coronavirus strains hCoV-NL63 (Genbank accession: AAS58177.1; SEQ ID No. 29), hCoV-OC43 (Genbank accession: AAR01015.1; SEQ ID No. 30), hCoV-229E (Genbank accession: AAG48592.1; SEQ ID No. 31) and huCoV-HKU1 (NCBI Reference Sequence: YP_173238.1; SEQ ID No. 32) was obtained with the multiple sequence alignment program T-coffee and adapted manually from ExPASy tool Boxshade. N-glycosylation sites in S1, RBD and S which are represented by the following amino acid motifs N-X-S/T, where X is any amino acid except P were highlighted in the sequences and the analysis of the SARS-CoV-2 spike has been recently reported (Watanabe et al., Science (2020), doi: 10.1126/science.abb9983) .

Results

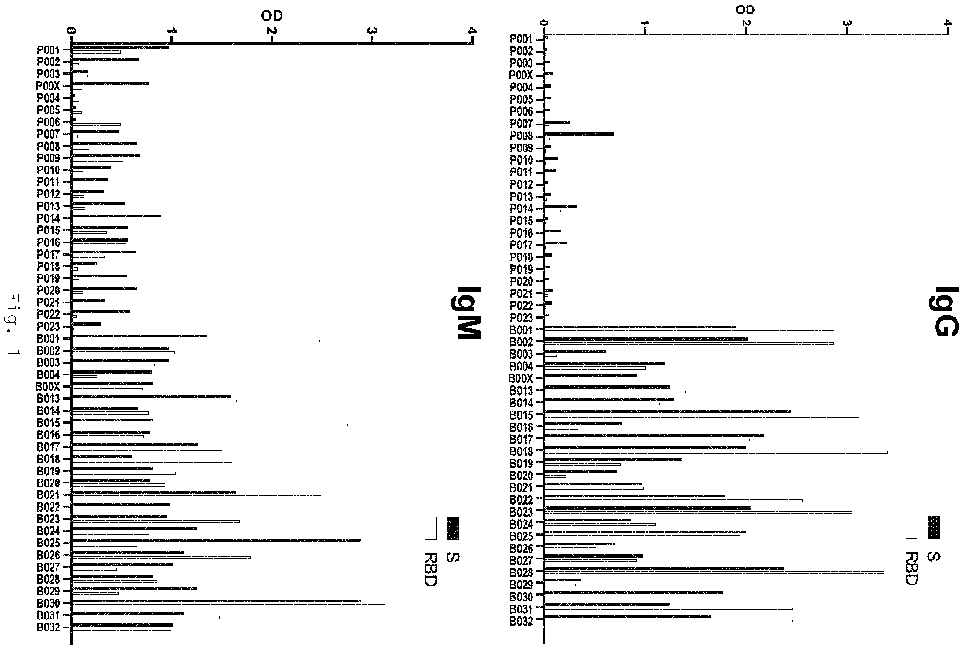

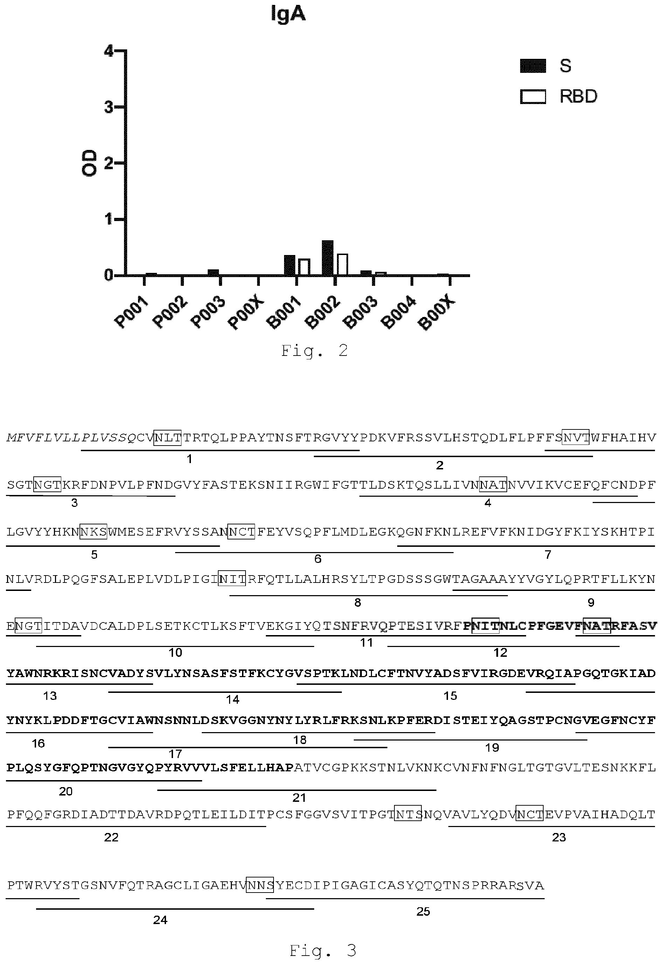

In order to investigate whether COVID-19 convalescent patients have developed antibodies which may protect from reinfection, sera were collected from COVID-19 convalescent patients approximately 10 weeks after confirmation of COVID-19 by PCR (Table 1) (group B, n=25, 11 females, 14 males, age range: 18-70 years, median age 52.2) and for control purposes sera from subjects obtained before the COVID-19 pandemic were included (historic control group P, n=24, 13 females, 11 males, age range: 18-68 years, median age 43.2) (Table 1). The course of COVID-19 in the PCR-confirmed convalescent subjects (group B) was relatively mild and did not require hospitalization but the duration of COVID-19-related symptoms varied considerably among patients (i.e., from 0-23 days) (Table 1). COVID-19 convalescent patients showed a quite strong and distinct IgG reactivity to S and RBD whereas no relevant RBD-specific IgG was found in the historic control sera (group P) of whom few showed some S-specific IgG (Fig. 1). IgA anti-RBD and anti-S responses measured in a subset of COVID-19 convalescent patients were low and not detectable in a subset of historic controls (Fig. 2). Strong S- and RBD-specific IgM responses were found in convalescent patients, but also frequent and distinct IgM responses were found in the historic controls (Fig. 1). In this context, it must be mentioned that S and RBD contain several glycosylation sites (see also Fig. 3 for glycosylation sites of S1 and RBD). S and RBD used in the ELISA described herein were expressed in eukaryotic cells and hence were glycosylated which would explain the occasional and weak recognition by IgG and the more frequent recognition by IgM, an isotype frequently reacting with glycan moieties, by the presence of anti-carbohydrate antibodies in the sera.

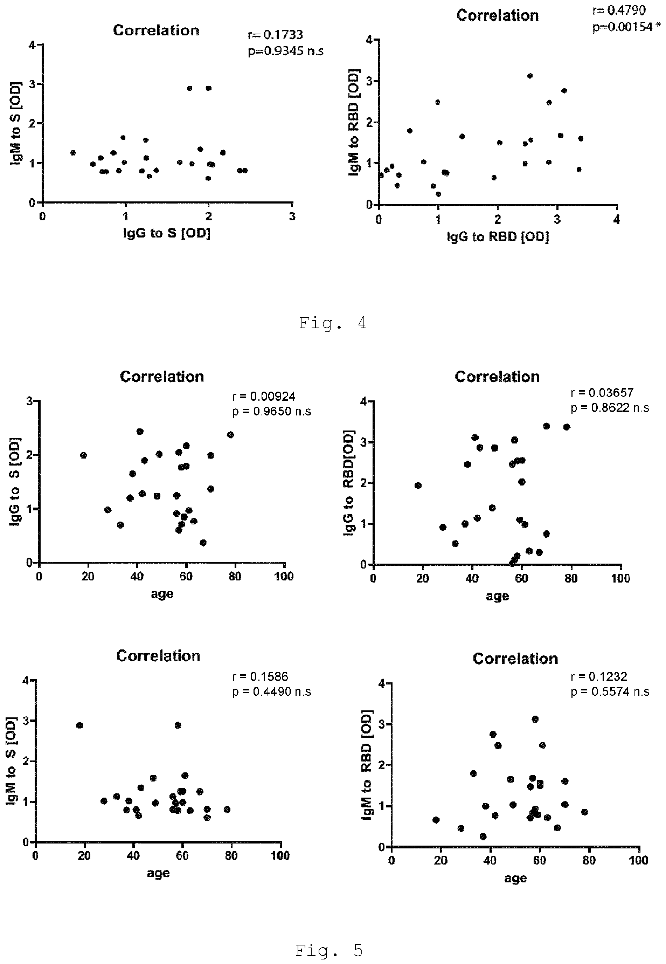

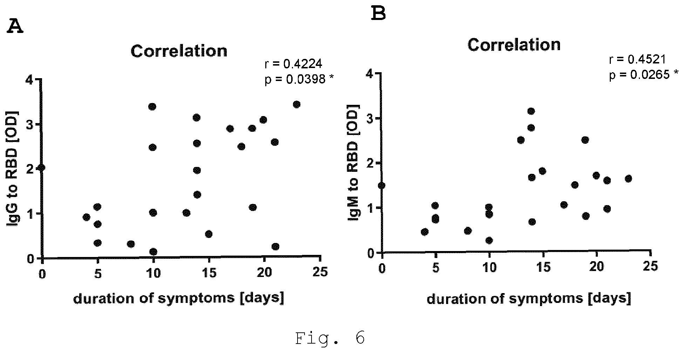

RBD-specific IgM responses in COVID-19 convalescent patients were not always associated with corresponding IgG responses (Fig. 1). For example, subjects B003 and B00X showed RBD-specific IgM reactivity whereas they mounted almost no RBD-specific IgG and subject B004 contained S- and RBD-specific IgG but no specific IgM was detected (Fig. 1). No correlation was found between S-specific IgM and IgG responses and a significant correlation was found between RBD-specific IgM and IgG responses (Fig. 4). While no correlation between age and S- and RBD-specific IgM or IgG levels could be found (Fig. 5), it was interesting to note that RBD-specific IgG and IgM levels were significantly correlated with the duration of COVID-19 symptoms suggesting that prolonged disease and thus virus-exposure may lead to increased virus-specific antibody production (Fig. 6).

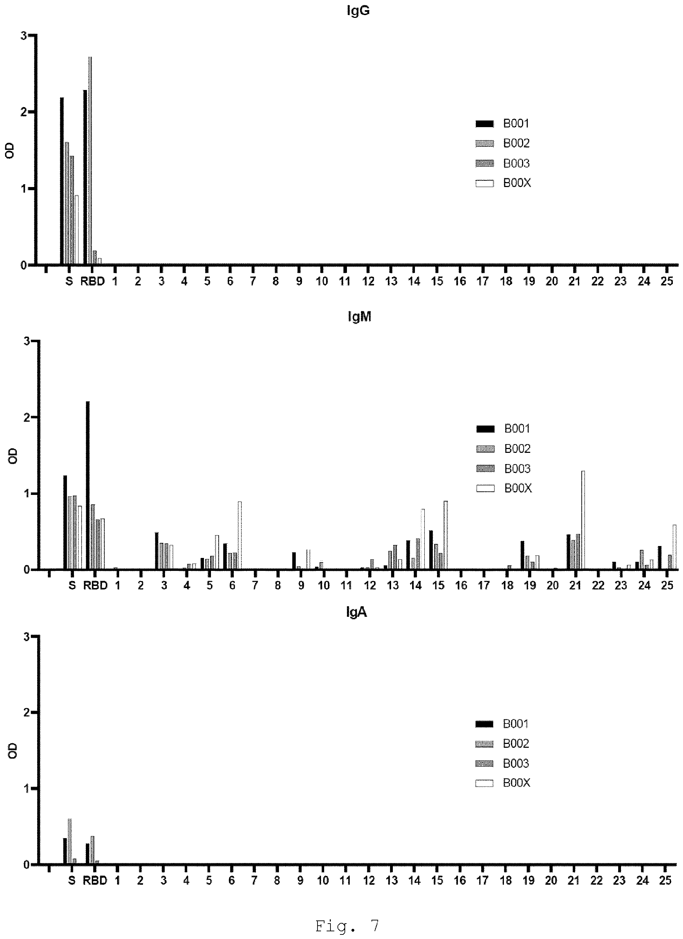

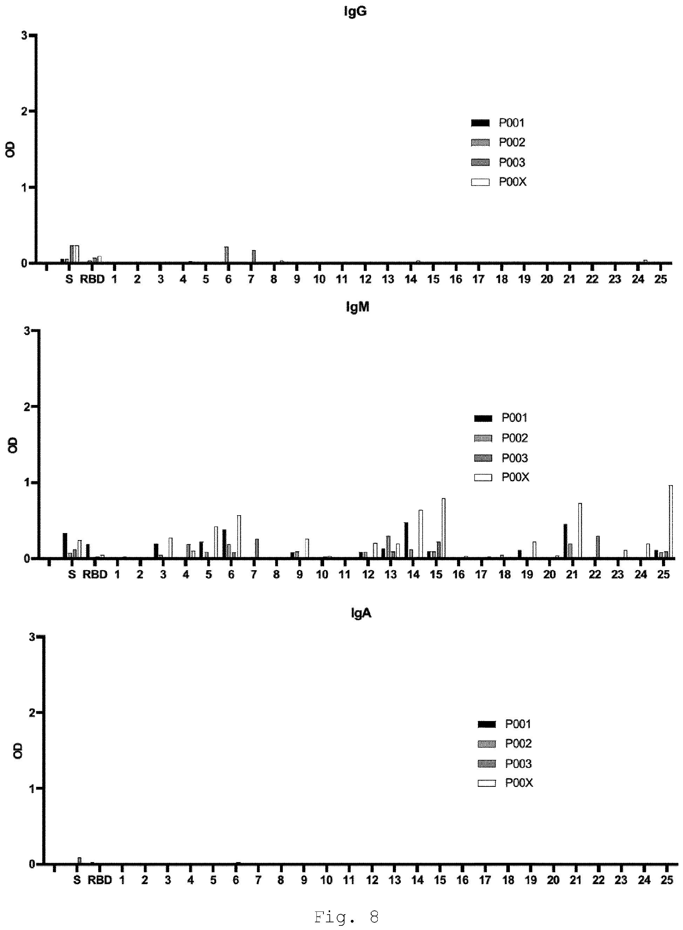

In a subset of sera antibody reactivity could be analysed to 25 synthetic overlapping 25-30 amino acids long peptides spanning the complete receptor-binding subunit S1 (SEQ ID No. 26), including RBD (Table 2, Fig. 3) indicating that there is no relevant peptide-specific IgG or IgA reactivity detectable (Figs. 7, 8).

Table 2. Characterization of SARS-CoV-2-derived synthetic peptides. Peptides are numbered, amino acid sequences, length of the peptides and isoelectric points (pI) are indicated.

| Peptide | aa sequence[1] | Length [aa] | pI[2] | SEQ ID No. |
|---|---|---|---|---|
| 1 | PLVSSQCVNLTTRTQLPPAYTNSFTRGVYY | 30 | 9,26 | 1 |
| 2 | RGVYYPDKVFRSSVLHSTQDLFLPFFSNVT | 30 | 8,5 | 2 |
| 3 | FSNVTWFHAIHVSGTNGTKRFDNPVLPFND | 30 | 6,92 | 3 |
| 4 | TLDSKTQSLLIVNNATNVVIKVCEFQFCND | 30 | 4,56 | 4 |
| 5 | QFCNDPFLGVYYHKNNKSWMESEFRVYSSA | 30 | 6,75 | 5 |
| 6 | VYSSANNCTFEYVSQPFLMDLEGKQGNFKN | 30 | 4,68 | 6 |
| 7 | GNFKNLREFVFKNIDGYFKIYSKHTPINLV | 30 | 9,7 | 7 |
| 8 | NITRFQTLLALHRSYLTPGDSSSGWTAGAA | 30 | 8,75 | 8 |
| 9 | TAGAAAYYVGYLQPRTFLLKYNENGTITDA | 30 | 5,73 | 9 |
| 10 | TITDAVDCALDPLSETKCTLKSFTVEKGIY | 30 | 4,44 | 10 |
| 11 | EKGIYQTSNFRVQPTESIVRFPNITNLC | 28 | 8,29 | 11 |
| 12 | PTESIVRFPNITNLCPFGEVFNATR | 25 | 6,52 | 12 |
| 13 | FNATRFASVYAWNRKRISNCVADYS | 25 | 9,78 | 13 |
| 14 | VADYSVLYNSASFSTFKCYGVSPTK | 25 | 8,11 | 14 |
| 15 | VSPTKLNDLCFTNVYADSFVIRGDEVRQIA | 30 | 4,68 | 15 |
| 16 | VRQIAPGQTGKIADYNYKLPDDFTGCVIAW | 30 | 6 | 16 |
| 17 | CVIAWNSNNLDSKVGGNYNYLYRLFRKSNL | 30 | 9,52 | 17 |
| 18 | DSKVGGNYNYLYRLFRKSNLKPFER | 25 | 9,99 | 18 |

(continued)

| Peptide | aa sequence[1] | Length [aa] | pI[2] | SEQ ID No. |
|---|---|---|---|---|
| 19 | KPFERDISTEIYQAGSTPCNGVEGF | 25 | 4,41 | 19 |
| 20 | GVEGFNCYFPLQSYGFQPTNGVGYQPYRVV | 30 | 5,99 | 20 |
| 21 | PYRVVVVLSFELLHAPATVCGPKKSTNLVKN | 30 | 9,65 | 21 |
| 22 | PFQQFGRDIADTTDAVRDPQTLEILDIT | 28 | 3,9 | 22 |
| 23 | AVLYQDVNCTEVPVAIHADQLTPTWRVYST | 30 | 4,54 | 23 |
| 24 | RVYSTGSNVFQTRAGCLIGAEHVNNSYECD | 30 | 5,44 | 24 |
| 25 | SYECDIPIGAGICASYQTQTNSPRRARSVA | 30 | 7,78 | 25 |

[1]Amino acid sequence of synthesized peptides, SARS-CoV-2 RBD is highlighted in bold.
[2]pI was predicted by ExPASy ProtParam tool.

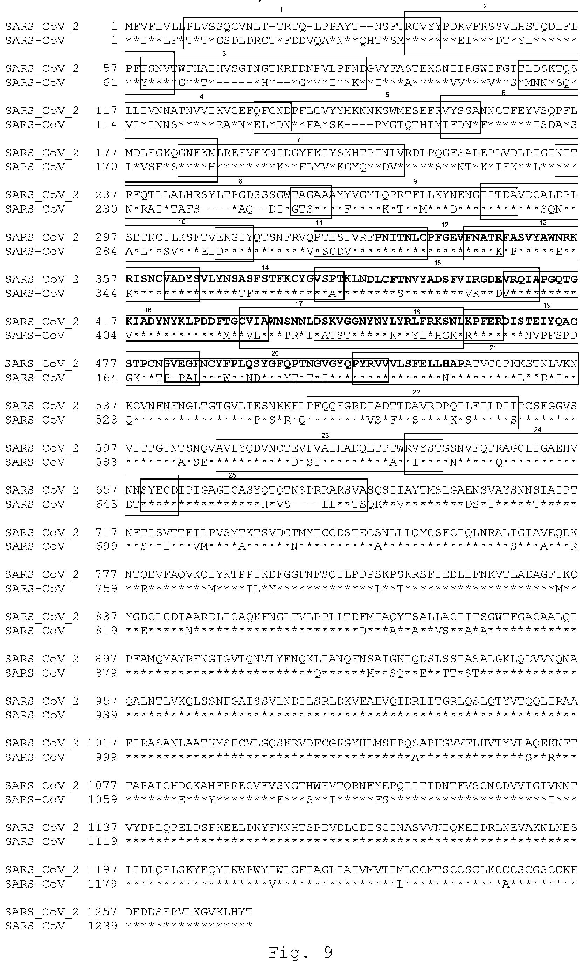

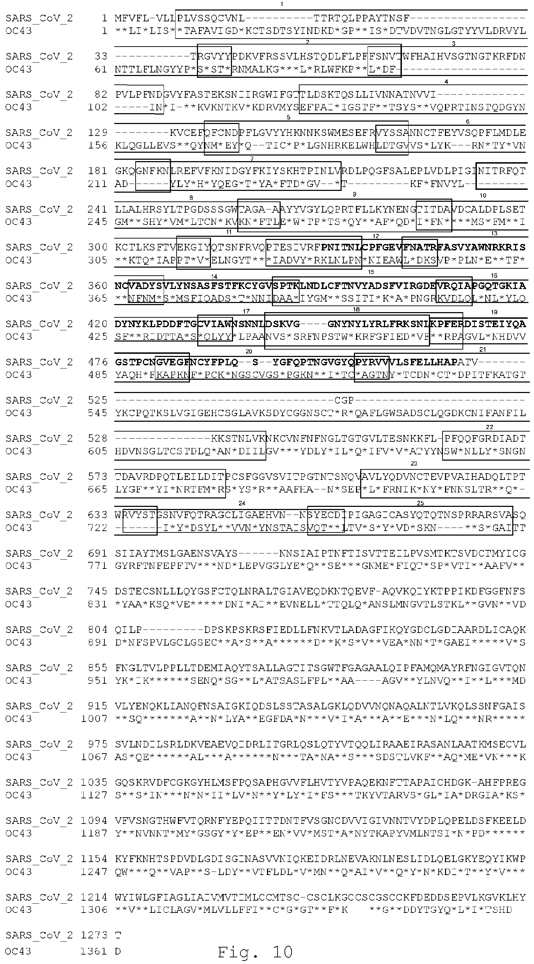

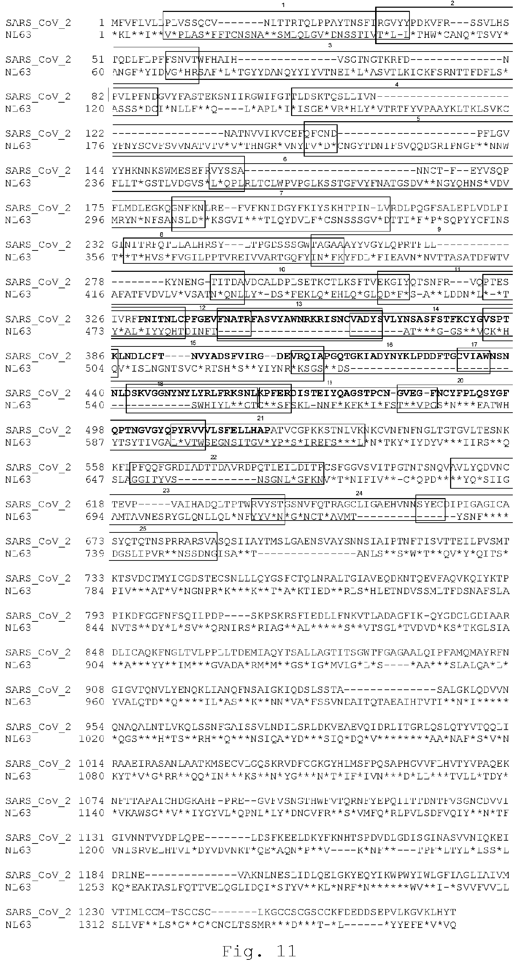

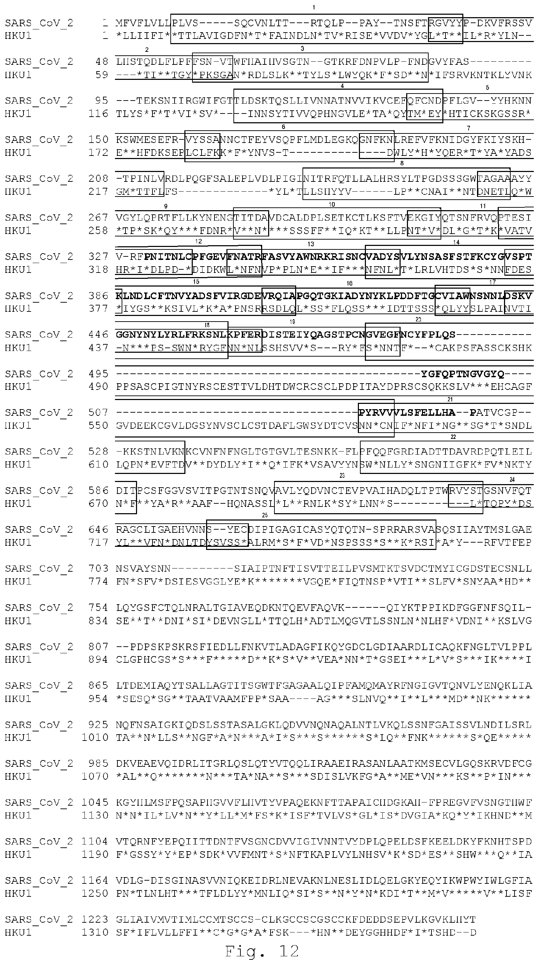

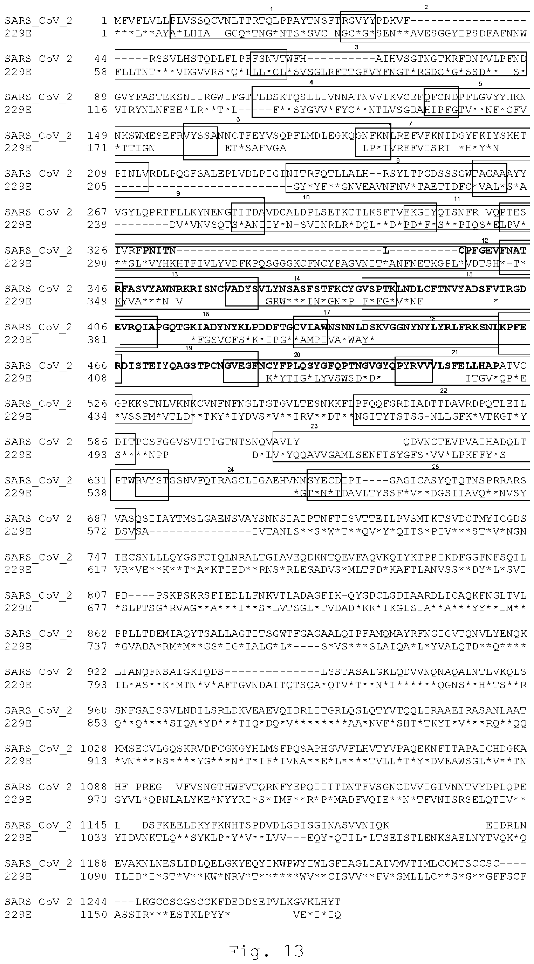

Sera from five tested convalescent COVID-19 subjects and, to a lower degree, sera from subjects of control group P showed some IgM reactivity to peptides from the N- and C-terminus of S1 and to distinct RBD-derived peptides (Figs. 7, 8). The amino acid sequences of the larger part of S1-derived peptides from SARS-CoV-2 are highly conserved in SARS-CoV but not in the other corona viruses known to cause common colds in humans (Figs. 9-13) indicating that the latter had not induced the peptide-specific IgM responses.

It was studied if and how many COVID-19 convalescent patients develop antibodies which can inhibit the binding of the virus via RBD to the corresponding receptor ACE2 which would protect them from a recurrent infection. In order to study this question a molecular interaction assay mimicking SARS-CoV-2 binding to its receptor ACE2 was developed. The ELISA assay is based on plate-bound recombinant ACE2 which is allowed to bind to recombinant His-tagged RBD (Fig 14A) . Bound RBD is then detected with a mouse monoclonal anti-His antibody followed by a secondary HRP-labelled anti-mouse IgG1 antibody (Fig. 14A, see also "Materials and Methods" Section).

Fig. 14B shows that RBD binds to ACE2 in a dose-dependent and specific manner whereas a negative control protein, the cysteine-containing, His-tagged recombinant Parietaria allergen, Par j 2, did not bind to ACE2. Next, it was investigated if binding of RBD to ACE2 can be blocked specifically by pre-incubation with soluble ACE2 (Fig. 14C, see also "Materials and Methods" Section). It was found that pre-incubation of RBD with ACE2 almost completely inhibited RBD binding to plate-bound ACE2 whereas pre-incubation with a negative control protein, recombinant major birch pollen allergen, Bet v 1, did not affect binding of RBD to ACE2 (Fig. 14C).

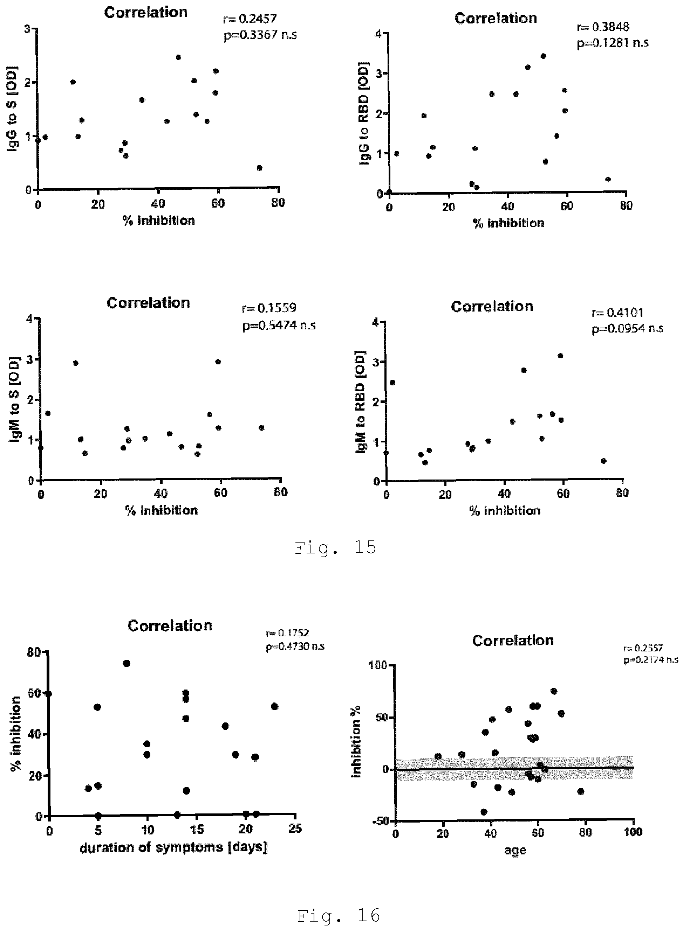

Then, the effects of antibodies in serum of COVID-19 convalescent patients on the binding of RBD to ACE2 were studied. Fig. 14D shows the optical density (OD) values corresponding to the binding of RBD after pre-incubation with sera from the 25 COVID-19 convalescent patients to ACE2. A more than 50% inhibition was found for six sera (B013, B017, B018, B019, B029, B030), an up to 50% inhibition was found for nine sera (B003, B014, B015, B020, B024, B025, B027, B031, B032), no inhibition was found for five sera (B00X, B016, B021, B022, B023) and for five sera (B001, B002, B004, B026, B028) even an enhancement of RBD binding to ACE2 was noted (Fig. 14D). No relevant inhibition was observed for 23 out of the 24 historic control sera obtained before the COVID-19 pandemic (Fig. 14E). One serum (i.e., P0014) which contained elevated S- and RBD-specific IgM antibodies caused an enhancement of RBD binding to ACE2 (Fig. 14E) pointing to the existence of "immune-enhancing", eventually natural anti-glycan antibodies. Interestingly, neither the levels of S- nor RBD-specific IgG or IgM antibodies were correlated with the inhibition of the binding of RBD to ACE2 in the inhibition assay (Fig. 15). There were also no significant correlations between the percentages of inhibition of RBD binding to ACE2 and the duration of COVID-19 symptoms and the age of the subjects, respectively (Fig. 16).

This example contains some very important findings. First of all, a simple and robust ELISA-based molecular interaction assay was established, which allows testing for antibodies and compounds capable of inhibiting the binding of SARS-CoV-2 RBD to its receptor. This is important because certain molecules such as ACE2-derivatives or recombinant antibodies are being considered for treatment of COVID-19 infections and there is a need to identify more and distil out the most efficient compounds for treatment. The molecular interaction assay is also useful to characterize and identify COVID-19 convalescent subjects producing antibodies capable of inhibiting the virus-receptor interaction for obtaining therapeutic convalescent plasma and validating polyclonal immunoglobulin and monoclonal antibody preparations. Furthermore, the assay is suitable for a mass screening of COVID-19 convalescent subjects regarding the presence of protective antibodies to inform who may be protected against SARS-CoV-2 infections considering the possibility of future outbreaks of the virus. The present data would rather indicate that the natural SARS-CoV-2 infection does not establish a protective antibody response in all infected subjects which can prevent the receptor-virus interaction. Only 60% of CVID-19-convalescent patients had produced antibodies which inhibited the binding of RBD to ACE2. Interestingly, evidence was found for an increase in RBD binding to ACE2 caused by sera from patients who produced RBD-specific IgG antibodies. This could be explained by the formation of immune complexes consisting of RBD and antibodies that bind to RBD without blocking the receptor interaction and eventually may be directed to carbohydrate epitopes of the virus. Such a mechanism of immune complex-enhanced SARS-CoV-2 receptor binding could potentially lead to more severe disease and/or aggravate secondary infections in subjects who after the first infection produced antibodies capable of cross-linking the RBDs of viruses and would explain earlier findings of immune enhancement in COVID-19.6 It is also conceivable that such an immune complex-mediated cross-linking of infected cells or cells containing ACE2-bound virus could be responsible for the inexplicably high incidence of thromboembolic events as observed in patients suffering from severe COVID-19 patients despite massive anticoagulation.

In summary, these findings suggest that a natural SARS-CoV-2 infection, eventually does not induce a protective antibody response inhibiting the virus-receptor interaction in all infected patients and therefore underline the urgent need for the development of a SARS-CoV-2 vaccine. The present assay is useful for identifying subjects having developed protective antibodies and for screening candidate vaccines to induce antibodies that inhibit the RBD-ACE2 interaction.

**Claims**

1. A method for identifying a compound enhancing, not affecting or inhibiting the binding of a virus to a receptor protein of said virus comprising the steps of

   a) incubating a first solid support comprising said receptor protein or a functional fragment thereof immobilized thereon with said virus or a fragment thereof;
   b) incubating a second solid support comprising said receptor protein or functional fragment thereof immobilized thereon with said virus or fragment thereof in the presence of said compound;
   c) determining the virus-receptor binding levels resulting from step a) and step b);
   d) comparing the binding levels determined in step c),
   e) identifying said compound as a compound enhancing virus-receptor binding if the binding level resulting from step b) is higher than the binding level resulting from step a),
   f) identifying said compound as a compound not affecting virus-receptor binding if the binding level resulting from step b) is substantially identical to the binding level resulting from step a), and
   g) identifying said compound as a compound inhibiting virus-receptor binding if the binding level resulting from step b) is lower than the binding level resulting from step a).

2. A method for discriminating between compounds enhancing the binding of a virus to a receptor protein of said virus and compounds inhibiting or not influencing said binding from a set of compounds, comprising the steps of

   a) incubating a first solid support comprising said receptor protein or a functional fragment thereof immobilized thereon with said virus or a fragment thereof;
   b) incubating a further solid support comprising said receptor protein or functional fragment thereof immobilized thereon with said virus or fragment thereof in the presence of a compound from said set of compounds;
   c) repeating step b) for further compounds from said set of compounds;
   d) determining the virus-receptor binding levels resulting from steps a) to c);
   e) comparing the binding levels determined in step d), and
   f) discriminating a compound enhancing virus-receptor binding from a compound inhibiting virus-receptor binding by evaluating if the binding level determined in the presence of said compound is higher compared to the binding level determined in the absence of one of said compounds.

3. The method of claim 1 or 2, wherein said virus is a coronavirus strain or fragment thereof and said receptor protein is a coronavirus receptor protein or functional fragment thereof.

4. The method of any one of claims 1 to 3, wherein said virus is SARS-CoV, SARS-CoV-2, or a fragment thereof, and wherein said receptor protein is angiotensin-converting enzyme 2 (ACE2) or a functional fragment thereof.

**EP 3 925 971 A1**

5. The method of any one of claims 1 to 3, wherein said virus is MERS-CoV or a fragment thereof, and wherein said receptor protein is dipeptidyl peptidase IV (DPP IV) or a functional fragment thereof.

6. The method of any one of claims 1 to 5, wherein said fragment of said virus comprises the receptor binding domain (RBD) of SARS-CoV, SARS-CoV-2 or MERS-CoV.

7. The method of any one of claims 1 to 6, wherein said virus or fragment thereof is histidine (His)-tagged RBD of SARS-CoV or SARS-CoV-2 and said His-tagged RBD is detected by using an anti-His detection antibody and an enzyme-labelled secondary detection antibody.

8. The method of any one of claims 1 or 3 to 7, wherein said virus or fragment thereof is pre-incubated with said compound prior to step b).

9. A method for assessing the suitability of a compound as vaccine candidate against a virus-induced disease comprising the steps of

   a) performing the method of any one of claims 1 to 8,
   b) identifying said compound as a suitable vaccine candidate against a disease induced by said virus if said compound inhibits virus-receptor binding, and
   c) identifying said compound as an unsuitable vaccine candidate against a disease induced by said virus if said compound enhances virus-receptor binding.

10. The method of claim 9, wherein said vaccine candidate is against a coronavirus-induced disease, preferably against Covid-19.

11. A kit for identifying a compound enhancing or inhibiting the binding of a virus to a receptor protein of said virus comprising

   a) a solid support coated with said receptor protein or a functional fragment thereof;
   b) said virus or a fragment thereof, and
   c) at least one detection molecule or labelling molecule.

12. The kit of claim 11, wherein said virus is a coronavirus strain or fragment thereof and said receptor protein is a coronavirus receptor protein or functional fragment thereof.

13. The kit of claim 11 or 12, wherein said virus is SARS-CoV, SARS-CoV-2, or a fragment thereof, and wherein said receptor protein is angiotensin-converting enzyme 2 (ACE2) or a functional fragment thereof and/or said virus is MERS-CoV or a fragment thereof, and wherein said receptor protein is dipeptidyl peptidase IV (DPP IV) or a functional fragment thereof.

14. The kit of any one of claims 11 to 13, wherein said fragment of said virus comprises the receptor binding domain (RBD) of SARS-CoV, SARS-CoV-2 or MERS-CoV.

15. Use of a kit according to any of claims 11 to 14 for identifying a compound neutralizing the binding of a virus to a receptor protein of said virus, for identifying a compound enhancing the binding of a virus to a receptor protein of said virus or for assessing the suitability of a compound as vaccine candidate against a virus-induced disease.

58

Fig. 1

## IgA

Fig. 2

*MFVFLVLLPLVSSQ*CV NLT TRTQLPPAYTNSFTRGVYYPDKVFRSSVLHSTQDLFLPFFS NVT WFHAIHV

SGT NGT KRFDNPVLPFNDGVYFASTEKSNIIRGWIFGTTLDSKTQSLLIVN NAT NVVIKVCEFQFCNDPF

LGVYYHKN NKS WMESEFRVYSSAN NCT FEYVSQPFLMDLEGKQGNFKNLREFVFKNIDGYFKIYSKHTPI

NLVRDLPQGFSALEPLVDLPIGI NIT RFQTLLALHRSYLTPGDSSSGWTAGAAAYYVGYLQPRTFLLKYN

E NGT ITDAVDCALDPLSETKCTLKSFTVEKGIYQTSNFRVQPTESIVRF **PNI TNLC PFGEVF NAT RFASV**

**YAWNRKRISNCVADYSVLYNSASFSTFKCYGVSPTKLNDLCFTNVYADSFVIRGDEVRQIAPGQTGKIAD**

**YNYKLPDDFTGCVIAWNSNNLDSKVGGNYNYLYRLFRKSNLKPFERDISTEIYQAGSTPCNGVEGFNCYF**

**PLQSYGFQPTNGVGYQPYRVVVLSFELLHAP**ATVCGPKKSTNLVKNKCVNFNFNGLTGTGVLTESNKKFL

PFQQFGRDIADTTDAVRDPQTLEILDITPCSFGGVSVITPGT NTS NQVAVLYQDV NCT EVPVAIHADQLT

PTWRVYSTGSNVFQTRAGCLIGAEHV NNS YECDIPIGAGICASYQTQTNSPRRAR SVA

Fig. 3

Fig. 4

Fig. 5

Fig. 6

IgG

IgM

IgA

Fig. 7

IgG

IgM

IgA

Fig. 8

```
SARS_CoV_2    1  MFVFLVLLPLVSSQCVNLT-TRTQ-LPPAYT--NSFTRGVYYPDKVFRSSVLHSTQDLFL
SARS-CoV      1  **I**LF*T*T*GSDLDRCT*FDDVQA*N**QHT*SM*****I**EI***DT*YL******

SARS_CoV_2   57  PFFSNVTWFHAIHVSGTNGTKRFDNPVLPFNDGVYFASTEKSNIIRGWIFGTTLDSKTQS
SARS-CoV     61  **Y****G**T*-----*H*--*G***I**K**I***A****VV***V**S*MNN*SQ*

SARS_CoV_2  117  LLIVNNATNVVIKVCEFQFCNDPFLGVYYHKNNKSWMESEFRVYSSANNCTFEYVSQPFL
SARS-CoV    114  VI*INNS*****RA*N*EL*DN**FA*SK----PMGTQTHTMIFDN*F******ISDA*S

SARS_CoV_2  177  MDLEGKQGNFKNLREFVFKNIDGYFKIYSKHTPINLVRDLPQGFSALEPLVDLPIGINIT
SARS-CoV    170  L*VSE*S****H********K**FLYV*KGYQ**DV****S**NT*K*IFK**L**I***

SARS_CoV_2  237  RFQTLLALHRSYLTPGDSSSGWTAGAAAYYVGYLQPRTFLLKYNENGTITDAVDCALDPL
SARS-CoV    230  N*RAI*TAFS----*AQ--DI*GTS***F****K*T**M***D***I*****I**SQN**

SARS_CoV_2  297  SETKCTLKSFTVEKGIYQTSNFRVQPTESIVRFPNITNLCPFGEVFNATRFASVYAWNRK
SARS-CoV    284  A*L**SV***ID****I*****V*SGDV**********K*P****E**

SARS_CoV_2  357  RISNCVADYSVLYNSASFSTFKCYGVSPTKLNDLCFTNVYADSFVIRGDEVRQIAPGQTG
SARS-CoV    344  K***I****I**VF********A*I*******S*****VK**DV*******

SARS_CoV_2  417  KIADYNYKLPDDFTGCVIAWNSNNLDSKVGGNYNYLYRLFRKSNLKPFERDISTEIYQAG
SARS-CoV    404  V***********M**VL**TR*IATST****K**YL*HGK*R*****NVPFSPD

SARS_CoV_2  477  STPCNGVEGFNCYFPLQSYGFQPTNGVGYQPYRVVVLSFELLHAPATVCGPKKSTNLVKN
SARS-CoV    464  GK**TP-PAI***W**ND***YT*T*I*******N*********L**D*I**

SARS_CoV_2  537  KCVNFNFNGLTGTGVLTESNKKFLPFQQFGRDIADTTDAVRDPQTLEILDITPCSFGGVS
SARS-CoV    523  Q***********P*S*R*Q********VS*F**S****K*S*****

SARS_CoV_2  597  VITPGTNTSNQVAVLYQDVNCTEVPVAIHADQLTPTWRVYSTGSNVFQTRAGCLIGAEHV
SARS-CoV    583  ******A*SE*********D*ST********A**I***N*****Q*********

SARS_CoV_2  657  NNSYECDIPIGAGICASYQTQTNSPRRARSVASQSIIAYTMSLGAENSVAYSNNSIAIPT
SARS-CoV    643  DT****************H*VS----LL**TSQK**V********DS*I*****T*****

SARS_CoV_2  717  NFTISVTTEILPVSMTKTSVDCTMYICGDSTECSNLLLQYGSFCTQLNRALTGIAVEQDK
SARS-CoV    699  **S**I**~*VM****A*****N********A*******************S***A***R

SARS_CoV_2  777  NTQEVFAQVKQIYKTPPIKDFGGFNFSQILPDPSKPSKRSFIEDLLFNKVTLADAGFIKQ
SARS-CoV    759  **R********M****TL*Y************L**T******************M**

SARS_CoV_2  837  YGDCLGDIAARDLICAQKFNGLTVLPPLLTDEMIAQYTSALLAGTITSGWTFGAGAALQI
SARS-CoV    819  **E*****N******************D***A**A**VS**A*A*********

SARS_CoV_2  897  PFAMQMAYRFNGIGVTQNVLYENQKLIANQFNSAIGKIQDSLSSTASALGKLQDVVNQNA
SARS-CoV    879  ***************************Q*****K**SQ**E**TT*ST**********

SARS_CoV_2  957  QALNTLVKQLSSNFGAISSVLNDILSRLDKVEAEVQIDRLITGRLQSLQTYVTQQLIRAA
SARS-CoV    939  ***********************************************************

SARS_CoV_2 1017  EIRASANLAATKMSECVLGQSKRVDFCGKGYHLMSFPQSAPHGVVFLHVTYVPAQEKNFT
SARS-CoV    999  *****************************************A*************S**R***

SARS_CoV_2 1077  TAPAICHDGKAHFPREGVFVSNGTHWFVTQRNFYEPQIITTDNTFVSGNCDVVIGIVNNT
SARS-CoV   1059  *******E***Y*******F***S**I*****FS****************I***

SARS_CoV_2 1137  VYDPLQPELDSFKEELDKYFKNHTSPDVDLGDISGINASVVNIQKEIDRLNEVAKNLNES
SARS-CoV   1119  ***********************************************************

SARS_CoV_2 1197  LIDLQELGKYEQYIKWPWYIWLGFIAGLIAIVMVTIMLCCMTSCCSCLKGCCSCGSCCKF
SARS-CoV   1179  ******************V****************L****************A********

SARS_CoV_2 1257  DEDDSEPVLKGVKLHYT
SARS_CoV   1239  *****************
```

Fig. 9

```
SARS_CoV_2    1 MFVFL-VLIPLVSSQCVNL----------TTRTQLPPAYTNSF-----------------
OC43          1 **LI*LIS**TAFAVIGD*KCTSDTSYINDKD*GP**IS*D*VDVTNGLGTYYVLDRVYL

SARS_CoV_2   33 -----------TRGVYYPDKVFRSSVLHSTQDLFLPFFSNV*WFHAIHVSGTNGTKRFDN
OC43         61 NTTLFLNGYYP*S*ST*RNMALKG***L*RLWFKP**L*DF-------------------

SARS_CoV_2   82 PVLPFNDGVYFASTEKSNIIRGWIFGTTLDSKTQSLLIVNNATNVVI------------
OC43        102 -----IN*I-**KVKNTKV*KDRVMYSEFPAI*IGSTF**TSYS**VQPRTINSTQDGYN

SARS_CoV_2  129 --------KVCEFQFCNDPFLGVYHKNNKSWMESEFRVYSSANNCTFEYVSQPFLMDLE
OC43        156 KLQGLLEVS**QYNM*EY*Q--TIC*P*LGNHRKELWHLDTGVVS*LYK---RN*TY*VN

SARS_CoV_2  181 GKQGNFKNLREFVFKNIDGYFKIYSKHTPINLVRDLPQGFSALEPLVDLPIGINITRFQT
OC43        211 AD------YLY*H*YQEG*T*YA*FTD*GV--*T---------KF*FNVYL-*-------

SARS_CoV_2  241 LLALHRSYLTPGDSSSGWTAGA-AYYVGYLQPRTFLLKYNENGTITDAVDCALDPLSET
OC43        245 GM**SHY*VM*LTCN-KVKN*FTLE*W*TP*TS*QY**AF*QD*I*FN****MS*FM**I

SARS_CoV_2  300 KCTLKSFTVEKGIYQTSNFRVQPTESIVRFPNITNLCPFGEVFNATRFASVYAWNRKRIS
OC43        305 **KTQ*IAPPT*V*ELNGYT**IADVY*RKLKLPN*NIEAWL*DKS*VP*PLN*E**TF*

SARS_CoV_2  360 NCVADYSVLYNSASFSTFKCYGVSPTKLNDLCFTNVYADSFVIRGDEVRQIAPGQTGKIA
OC43        365 **NFNM*S*MSFIQADS*T*NNIDAA*IYGM**SSITI*K*A*PNGRKVDLQL*NL*YLQ

SARS_CoV_2  420 DYNYKLPDDFTGCVIAWNSNNLDSKVG----GNYNYLYRLFRKSNLKPFERDISTEIYQA
OC43        425 SF**RIDTTA*S*QLYY*LPAANVS*SRFNPSTW*KRFGFIED*VF**RPAGVL*NHDVV

SARS_CoV_2  476 GSTPCNGVEGFNCYFPLQ--S--YGFQPTNGVGYQPYRVVVLSFELLHAPATV-------
OC43        485 YAQH*F*KAPKNF*PCK*NGSCVGS*PGKN**I*TQ*AGTNY*TCDN*CT*DPITFKATGT

SARS_CoV_2  525 --------------------------------------CGP-------------------
OC43        545 YKCPQTKSLVGIGEHCSGLAVKSDYCGGNSCT*R*QAFLGWSADSCLQGDKCNIFANFIL

SARS_CoV_2  528 ---------------KKSTNLVKNKCVNFNFNGLTGTGVLTESNKKFL-PFQQFGRDIADT
OC43        605 HDVNSGLTCSTDLQ*AN*DIILGV***YDLY*IL*Q*IFV*V*AT*YYNSW*NLLY*SNGN

SARS_CoV_2  573 TDAVRDPQTLEILDITPCSFGGVSVITPGTNTSNQVAVLYQDVNCTEVPVAIHADQLTPT
OC43        665 LYGF**YI*NRTFM*RS*YS*R**AAFHA--N*SEF*L*FRNIK*NY*FNKSLTR**Q*-

SARS_CoV_2  633 WRVYSTGSNVFQTRAGCLIGAEHVN--NSYECDIPIGAGICASYQTQTNSPRRARSVASQ
OC43        722 -*----*I*Y*DSYL**VVN*YNSTAI*SVQT**ILTV*S*Y*VD*SKN----**S*GAITT

SARS_CoV_2  691 SIIAYTMSLGAENSVAYS------NNSIAIPTNFTISVTTEILPVSMTKTSVDCTMYICG
OC43        771 GYRFTNFEPFTV***ND*LEPVGGLYE*Q**SE***GNME*FIQ*SP*VTI**AAFV**

SARS_CoV_2  745 DSTECSNLLLQYGSFCTQLNRALTGIAVEQDKNTQEVF-AQVKQIYKTPPIKDFGGFNFS
OC43        831 *YAA*KSQ*VE*****DNI*AI**EVNELL*TTQLQ*ANSLMNGVTLSTKL**GVN**VD

SARS_CoV_2  804 QILP---------DPSKPSKRSFIEDLLFNKVTLADAGFIKQYGDCLGDIAARDLICAQK
OC43        891 D*NFSPVLGCLGSEC**A*S**A*****D**K*S*V**VEA*NN*T*GAEI*****V*S

SARS_CoV_2  855 FNGLTVLPPLLTDEMIAQYTSALLAGTITSGWTFGAGAALQIPFAMQMAYRFNGIGVTQN
OC43        951 YK*IK*****SENQ*SG**L*ATSASLFPL**AA----AGV**YLNVQ**I**L**MD

SARS_CoV_2  915 VLYENQKLIANQFNSAIGKIQDSLSSTASALGKLQDVVNQNAQALNTLVKQLSSNFGAIS
OC43       1007 **SQ*******A**N*LYA**EGFDA*N***V*I*A***A**E***N*LQ***NR*****

SARS_CoV_2  975 SVLNDILSRLDKVEAEVQIDRLITGRLQSLQTYVTQQLIRAAEIRASANLAATKMSECVL
OC43       1067 AS*QE*****AL***A******N***TA*NA**S***SDST*LVKF**AQ*ME*VK***K

SARS_CoV_2 1035 GQSKRVDFCGKGYHLMSFPQSAPHGVVFLHVTYVPAQEKNFTTAPAICHDGK-AHFPREG
OC43       1127 S**S*IK***N*N*II*LV*N**Y*LY*I*FS***TKYVTARVS*GL*IA*DRGIA*KS*

SARS_CoV_2 1094 VFVSNGTHWFVTQRNFYEPQIITTDNTFVSGNCDVVIGIVNNTVYDPLQPELDSFKEELD
OC43       1187 Y**NVNKT*MY*GSGY*Y*EP**EK**VV*MST*A*NYTKAPYVMLNTSI*K*PD*****

SARS_CoV_2 1154 KYFKNHTSPDVDLGDISGINASVVNIQKEIDRLNEVAKNLNESLIDLQELGKYEQYIKWP
OC43       1247 QW***Q**VAP**S-LDY**VTFLDL*V*MN**Q*AI*V**Q*Y*N*KDI*T**Y*V***

SARS_CoV_2 1214 WYIWLGFIAGLIAIVMVTIMLCCMTSC-CSCLKGCCSCGSCCKFDEDDSEPVLKGVKLHY
OC43       1306 **V**LICLAGV*MLVLLFFI**C*G*GT**F*K   **G**DDYTGYQ*L*I*TSHD

SARS_CoV_2 1273 T
OC43       1361 D
```

Fig. 10

```
SARS_CoV_2    1 MEVFLVLLPLVSSQCV-------NLTTRTQLPPAYTNSFLRGVYYPDKVFR---SSVLHS
NL63          1 *KL**I**V*PLAS*FFTCNSNA**SMLQLGV*DNSSTIVT*L-L*THW*CANQ*TSVY*

SARS_CoV_2   51 TQDLFLPFFSNVTWFHAIH---------------VSGTNGTKRFD-------------N
NL63         60 ANGF*YIDVG*HRSAF*L*TGYYDANQYYIYVTNEI*L*ASVTLKICKFSRNTTFDFLS*

SARS_CoV_2   82 TVLPFNDGVYFASTEKSNIIRGWIFGTTLDSKTQSLLIVN--------------------
NL63        120 ASSS*DCI*NLLF**Q----L*APL*I*ISGE*VR*HLY*VTRTFYVPAAYKLTKLSVKC

SARS_CoV_2  122 -----------------NATNVVIKVCEFQFCND--------------------PFLGV
NL63        176 YFNYSCVFSVVNATVIV*V*THNGR*VNYIV*D*CNGYTDNIFSVQQDGRIPNGF**NNW

SARS_CoV_2  144 YYHKNNKSWMESEFRVYSSA----------------------NNCT-F--EYVSQP
NL63        236 FLLT*GSTLVDGVS*L*QPLRLTCLWPVPGLKSSTGFVYFNATGSDV**NGYQHNS*VDV

SARS_CoV_2  175 FLMDLEGKQGNFKNLRE--FVFKNIDGYFKIYSKHTPIN-LVRDLPQGFSALEPLVDLPI
NL63        296 MRYN*NFSANSLD**KSGVI***TLQYDVLF*CSNSSSGV*DTTI*F*P*SQPYYCFINS

SARS_CoV_2  232 GINITRFQTLLALHRSY---LTPGDSSSGWTAGAAAYYVGYLQPRTFLL-----------
NL63        356 T**T*HVS*FVGILPPTVREIVVARTGQFYIN*FKYFDL*FIEAVN*NVTTASATDFWTV

SARS_CoV_2  278 --------KYNENG-TITDAVDCALDPLSETKCTLKSFTVEKGIYQTSNFR---VQPTES
NL63        416 AFATFVDVLV*VSATN*QNLLY*-DS*FEKLQ*EHLQ*GLQD*F*S-A**LDDN*L*-*T

SARS_CoV_2  326 IVRFPNITNLCPFGEVFNATRFASVYAWNRKRISNCVADYSVLYNSASFSTFKCYGVSPT
NL63        473 Y*AL*IYYQHTDINFI----------------------------AT***G-GS**VCK*H

SARS_CoV_2  386 KLNDLCFT----NVYADSFVIRG--DEVRQIAPGQTGKIADYNYKLPDDFTGCVIAWNSN
NL63        504 QV*ISLNGNTSVC*RTSH*S**YIYNR*KSGS**DS----------------------

SARS_CoV_2  440 NLDSKVGGNYNYLYRLFRKSNLKPFERDISTEIYQAGSTPCN-GVEG-FNCYFPLQSYGF
NL63        540 --L-----------SWHIYL**CTC**SFSKL-NNF*KFK*I*FST**VPGS*N***FATWH

SARS_CoV_2  498 QPTNGVGYQPYRVVVLSFELLHAPATVCGPKKSTNLVKNKCVNFNFNGLTGTGVLTESNK
NL63        587 YTSYTIVGAL*VTWSEGNSITGV*YP*S*IREFS***L*N*TKY*IYDYV***IIRS**Q

SARS_CoV_2  558 KFLPFQQFGRDIADTTDAVRDPQTLEILDITPCSFGGVSVITPGTNTSNQVAVLYQDVNC
NL63        647 SLAGGITYVS-----------NSGNL*GFKNV*T*NIFIV**-C*QPD**Y*YQ*SIIG

SARS_CoV_2  618 TEVP-----VAIHADQLTPTWRVYSTGSNVFQTRAGCLIGAEHVNNSYECDIPIGAGICA
NL63        694 AMTAVNESRYGLQNLLQL*NFYYV*N*G*NCT*AVMT---------------YSNF****

SARS_CoV_2  673 SYQTQTNSPRRARSVASQSIIAYTMSLGAENSVAYSNNSIAIPTNFTISVTTEILPVSMT
NL63        739 DGSLIPVR**NSSDNGISA**T---------------ANLS**S*W*T**QV*Y*QITS*

SARS_CoV_2  733 KTSVDCTMYICGDSTECSNLLLCYGSFCTQLNRALTGIAVEQDKNTQEVFAQVKQIYKTP
NL63        784 PIV***AT*V*NGNPR*K***K**T*A*KTIED**RLS*HLETNDVSSMLTFDSNAFSLA

SARS_CoV_2  793 PIKDFGGFNFSQILPDP----SKPSKRSFIEDLLFNKVTLADAGFIK-QYGDCLGDIAAR
NL63        844 NVTS**DY*L*SV**QRNIRS*RIAG**AL****S**VTSGL*TVDVD*KS*TKGLSIA

SARS_CoV_2  848 DLICAQKFNGLTVLPPLLTDEMIAQYTSALLAGTITSGWTFGAGAALQIPFAMQMAYRFN
NL63        904 **A***YY**IM***GVADA*RM*M**GS*IG*MVLG*L*S----*AA***SLALQA*L*

SARS_CoV_2  908 GIGVTQNVLYENQKLIANQFNSAIGKIQDSLSSTA--------------SALGKLQDVVN
NL63        960 YVALQTD**Q****IL*AS**K**NN*VA*FSSVNDAITQTAEAIHTVTI**N*I*****

SARS_CoV_2  954 QNAQALNTLVKQLSSNFGAISSVLNDILSRLDKVEAEVQIDRLITGRLQSLQTYVTQQLI
NL63       1020 *QGS***H*TS**RH**Q***NSIQA*YD***SIQ*DQ*V*******AA*NAF*S*V*N

SARS_CoV_2 1014 RAAEIRASANLAATKMSECVLGQSKRVDFCGKGYHLMSFPQSAPHGVVFLHVTYVPAQEK
NL63       1080 KYT*V*G*RR**QQ*IN***KS**N*YG***N*T*IF*IVN***D*LL***TVLL*TDY*

SARS_CoV_2 1074 NFTTAPAICHDGKAHF-PRE--GVFVSNGTHWFVTQRNFYEPQILTTDNTFVSGNCDVVI
NL63       1140 *VKAWSG**V**IYGYVL*QPNL*LY*DNCVFR**S*VMFQ*RLPVLSDFVQIY**N*TF

SARS_CoV_2 1131 GIVNNTVYDPLQPE-------LDSFKEELDKYFKNHTSPDVDLGDISGINASVVNIQKEI
NL63       1200 VNLSRVELHTVL*DYVDVNKT*QE*AQN*P**V----K*NF**---TPF*LTYL*LSS*L

SARS_CoV_2 1184 DRLNE-------------VAKNLNESLIDLQELGKYEQYIKWPWYIWLGFIAGLIAIVM
NL63       1253 KQ*EAKTASLFQTTVELQGLIDCI*STYV**KL*NRF*N******WV**I-*SVVFVVLL

SARS_CoV_2 1230 VTIMLCCM-TSCCSC-------LKGCCSCGSCCKFDEDDSEPVLKGVKLHYT
NL63       1312 SLLVF**LS*G**G*CNCLTSSMR***D***T-*L------YYEFE*V*VQ
```

Fig. 11

```
                          1
SARS_CoV_2    1 MFVFLVLL PLVS-----SQCVNLTT---RTQLP--PAY--TNSFT RGVYY P-DKVFRSSV
HKU1          1 *LLIIFI* *TTLAVIGDFN*T*FAINDLN*TV*RISE*VVDV*YG L*T** IL*R*YLN--
                            2                3
SARS_CoV_2   48 LIISTQDLFLPF FSN-VT WFHAIIVSGTN--GTKRFDNPVLP-FND GVYFAS---------
HKU1         59 ---*TI**TGY* PKSGA N*RDLSLK**TYLS*LWYQK*F*SD**N *IFSRVKNTKLYVNK
                                    4                              5
SARS_CoV_2   95 ---TEKSNIIRGWIFGT TLDSKTQSLLIVNNATNVVIKVCEF QFCND PFLGV--YYHKNN
HKU1        116 TLYS*F*T*VI*SV*-- -INNSYTIVVQPHNGVLE*TA*QY TM*EY *HTICKSKGSSR*
                              6                            7
SARS_CoV_2  150 KSWMESEFR VYSSA NNCTFEYVSQPFLMDLEGKQ GNFKN REFVFKNIDGYFKIYSKH-
HKU1        172 E**HFDKSEP LCLFK K*F*YNVS-T--------- ----DWLY *H*YQER*T*YA*YADS
                                                     8
SARS_CoV_2  208 -TPINLV RDLPQGFSALEPLVDLPIGI NITRFQTLLALHRSYLTPGDSSSGW TAGAAAYY
HKU1        217 GM*TTFL FS------------*YL*T LLSHYYV------LP**CNAI**NT DNETLQ*W
                            9          10                          11
SARS_CoV_2  267 VGYLQPRT FLLKYNENG TITDA VDCALDPLSETKCTLKSFTV EKGIY QTSNFRVQ PTESI
HKU1        258 *TP*SK*QY*** FDNR *V**N* ***SSSFF**IQ*KT**LLP NT*V* DL*G*T*K VATV
                         12             13                   14
SARS_CoV_2  327 V-RF PNITNLC PFGEV FNATR FASVYAWNRKRISNC VADYS VLYNSASFSTFKCYG VSPT
HKU1        318 HR*I* DLPD-* DIDKW L*NFN VP*PLN*E**IF** NFNL* T*LRLVHTDS*S*NN FDES
                           15               16                        17
SARS_CoV_2  386 KLNDLCFTNVYADSFVIRGDE VRQIA PGQTGKIADYNYKLPDDFTG CVIAWNSNNL DSKV
HKU1        377 *IYGS**KSIVL*K*A* PNSRR SDLQL*SS*FLQSS*** IDTTSSS*QLYY SLPAI NVTI
                                  18            19                      20
SARS_CoV_2  446 GGNYNYLYRLFRKSNL KPFER DISTEIYQAGSTPCN GVEGF NCYFPLQS----------
HKU1        437 -N***PS-SWN*RYGF NN*NL SSHSVV*S---RY*F S*NNT F*--*CAKPSFASSCKSHK
SARS_CoV_2  495 -------------------------------------YGFQPTNGVGYQ----
HKU1        490 PPSASCPIGTNYRSCESTTVLDHTDWCRCSCLPDPITAYDPRSCSQKKSLV***EHCAGF
                                                             21
SARS_CoV_2  507 ------------------------------------PYRVV LSFELLHA--P ATVCGP--
HKU1        550 GVDEEKCGVLDGSYNVSCLCSTDAFLGWSYDTCVS NN*CN LF*NFI*NG**SG*T*SNDL
                                                        22
SARS_CoV_2  528 -KKSTNLVKN KCVNFNFNGLTGTGVLTESNKK-FL PFQQFGRDIADTTDAVRDPQTIEIL
HKU1        610 LQPN*EVFTD V**DYDLY*I**Q*IFK*VSAVYYN SW*NLLY*SNGNIIGFK*FV*NKTY
                                             23                         24
SARS_CoV_2  586 DIT PCSFGGVSVITPGTNTSNQV AVLYQDVNCTEVPVAIHADQLTPTW RVYS TGSNVFQT
HKU1        670 N*F**YA*R**AAF--HQNASSL *L**RNLK*SY*LNN*S-------- ---L* TQPY*DS
                                  25
SARS_CoV_2  646 RAGCLIGAEHVNN S--YEC DIPIGAGICASYQTQTN-SPRRARSVA SQSIIAYTMSLGAE
HKU1        717 YL**VFN*DNLTD YSVSS*ALRM*S*F*VD*NSPSSS*S**K*RSI *A*Y---RFVTFEP
SARS_CoV_2  703 NSVAYSNN---------SIAIPTNFTISVTTEILPVSMTKTSVDCTMYICGDSTECSNLL
HKU1        774 FN*SFV*DSIESVGGLYE*K******VGQE*FIQTNSP*VTI**SLFV*SNYAA*HD**
SARS_CoV_2  754 LQYGSFCTQLNRALTGIAVEQDKNTQEVFAQVK------QIYKTPPIKDFGGFNFSQIL-
HKU1        834 SE**T**DNI*SI*DEVNGLL*TTQLH*ADTLMQGVTLSSNLN*NLHF*VDNI**KSLVG
SARS_CoV_2  807 --PDPSKPSKRSFIEDLLFNKVTLADAGFIKQYGDCLGDIAARDLICAQKFNGLTVLPPL
HKU1        894 CLGPHCGS*S***F*****D**K*S*V**VEA*NN*T*GSEI***L*V*S***IK****I
SARS_CoV_2  865 LTDEMIAQYTSALLAGTITSGWTFGAGAALQIPFAMQMAYRFNGIGVTQNVLYENQKLIA
HKU1        954 *SESQ*SG**TAATVAAMFPP*SAA----AG***SLNVQ**I**L***MD**NK******
SARS_CoV_2  925 NQFNSAIGKIQDSLSSTASALGKLQDVVNQNAQALNTLVKQLSSNFGAISSVLNDILSRL
HKU1       1010 TA**N*LLS**NGF*A*N***A*I*S***S******S*LQ**FNK******S*QE*****
SARS_CoV_2  985 DKVEAEVQIDRLITGRLQSLQTYVTQQLIRAAEIRASANLAATKMSECVLGQSKRVDFCG
HKU1       1070 *AL**Q*******N***TA*NA**S***SDISLVKFG*A**ME*VN***KS**P*IN***
SARS_CoV_2 1045 KGYHLMSFPQSAPHGVVFLHVTYVPAQEKNFTTAPAICHDGKAH-FPREGVFVSNGTHWF
HKU1       1130 N*N*IL*LV*N**Y*LL*M*FS*K*ISF*TVLVS*GL*IS*DVGIA*KQ*Y*IKHND**M
SARS_CoV_2 1104 VTQRNFYEPQIITTDNTFVSGNCDVVIGIVNNTVYDPLQPELDSFKEELDKYFKNHTSPD
HKU1       1190 F*GSSY*Y*EP*SDK*VVFMNT*S*NFTKAPLVYLNHSV*K*SD*ES**SHW***Q**IA
SARS_CoV_2 1164 VDLG-DISGINASVVNIQKEIDRLNEVAKNLNESLIDLQELGKYEQYIKWPWYIWLGFIA
HKU1       1250 PN*TLNLHT***TFLDLYY*MNLIQ*SI*S**N*Y*N*KDI*T**M*V****V**LISF
SARS_CoV_2 1223 GLIAIVMVTIMLCCMTSCCS-CLKGCCSCGSCCKFDEDDSEPVLKGVKLHYT
HKU1       1310 SF*IFLVLLFFI**C*G*G*A*FSK---*HN**DEYGGHHDF*I*TSHD--D
```

Fig. 12

```
SARS_CoV_2    1  MFVFLVLLPLVSSQCVNLTTRTQLPPAYTNSFTRGVYYPDKVF-----------------
229E          1  ***L**AYA*LHIA   GCQ*TNG*NTS*SVC NGC*G*SEN**AVESGGYIPSDFAFNNW
                                     1                              2
SARS_CoV_2   44  -----RSSVLHSTQDLFLPFFSNVTWFH----------AIHVSGTNGTKRFDNPVLPFND
229E         58  FLLTNT***VDGVVRS*Q*ILL*CLKSVSGLRFTTGFVYFKGT*RGDC*G*SSD**--S*
                                        3
SARS_CoV_2   89  GVYFASTEKSNIIRGWIFGTTLDSKTQSLLIVNNATNVVIKVCEFQFCNDPFLGVYYHKN
229E        116  VIRYNLNFEE*LR**T*L----F**SYGVV*FYC**NTLVSGDAHIPFGTV**NF*CFV
                                       4                    5
SARS_CoV_2  149  NKSWMESEFRVYSSANNCTFEYVSQPFLMDLEGKQGNFKNLREFVFKNIDGYFKIYSKHT
229E        171  *TIGN----  ----ET*SAFVGA---------LP*TVREFVISRT-*E*Y*N----
                            6                          7
SARS_CoV_2  209  PINLVRDLPQGFSALEPLVDLPIGINITRFQTLLALH--RSYLTPGDSSSGWTAGAAAYY
229E        205  ----- ------------------GY*YF**GNVEAVNFNV*TAETTDFC*VAL*S*A
                     8                    9              10
SARS_CoV_2  267  VGYLQPRTFLLKYNENGTITDAVDCALDPLSETKCTLKSFTVEKGIYQTSNFR-VQPTES
229E        239  --------DV*VNVSQTS*ANIIY*N-SVINRLR*DQL**D*PD*F*S**PIQS*ELPV*
                                    9            10          11
SARS_CoV_2  326  IVRFPNITN-----------------------------L---------CPFGEVFNAT
229E        290  **SL*VYHKETFIVLYVDFKPQSGGGKCFNCYPAGVNIT*ANFNETKGPL*VDTSH*-T*
                                           12                    12
SARS_CoV_2  346  RFASVYAWNRKRISNCVADYSVLYNSASFSTFKCYGVSPTKLNDLCFTNVYADSFVIRGD
229E        349  KYVA***N V         GRW***IN*GN*P  F*FG*V*NF          *
                          13         14                    15
SARS_CoV_2  406  EVRQIAPGQTGKIADYNYKLPDDFTGCVIAWNSNNLDSKVGGNYNYLYRLFRKSNLKPFE
229E        381        *FGSVCFS*K*IPG**AMPIVA*WAY*
                      16          17                     18
SARS_CoV_2  466  RDISTEIYQAGSTPCNGVEGFNCYFPLQSYGFQPTNGVGYQPYRVVLSFELLHAPATVC
229E        408  --------------  ----K*YTIG*LYVSWSD*D*----- ----ITGV*QP*E
                       19             20                   21
SARS_CoV_2  526  GPKKSTNLVKNKCVNFNFNGLTGTGVLTESNKKFIPFQQFGRDIADTTDAVRDPQTLEIL
229E        434  *VSSFM*VILD**TKY*IYDVS*V**IRV**DT**NGITYTSTSG-NLLGFK*VTKGT*Y
                                                     22
SARS_CoV_2  586  DITPCSFGGVSVITPGTNTSNQVAVLY---------------QDVNCTEVPVAIHADQLT
229E        493  S**\**NPP------------D*LV*YQQAVVGAMLSENFTSYGFS*VV*LPKFFY*S---
                            23
SARS_CoV_2  631  PTWRVYSTGSNVFQTRAGCLIGAEHVNNSYECDIPI----GAGICASYQTQTNSPRRARS
229E        538  ---- ------------------*GT*N*TDAVLTYSSF*V**DGSIIAVQ**NVSY
                     24                          25
SARS_CoV_2  687  VASQSIIAYTMSLGAENSVAYSNNSIAIPTNFTISVTTEILPVSMTKTSVDCTMYICGDS
229E        572  DSVSA---------------IVTANLS**S*W*T**QV*Y*QITS*PIV***ST*V*NGN
SARS_CoV_2  747  TECSNLLLQYGSFCTQLNRALTGIAVEQDKNTQEVFAQVKQIYKTPPIKDFGGFNFSQIL
229E        617  VR*VE**K**T*A*KTIED**RNS*RLESADVS*MLTFD*KAFTLANVSS**DY*L*SVI
SARS_CoV_2  807  PD----PSKPSKRSFIEDLLFNKVTLADAGFIK-QYGDCLGDIAARDLICAQKFNGLTVL
229E        677  *SLPTSG*RVAG**A***I**S*LVTSGL*TVDAD*KK*TKGLSIA**A***YY**IM**
SARS_CoV_2  862  PPLLTDEMIAQYTSALLAGTITSGWTFGAGAALQIPFAMQMAYRFNGIGVTQNVLYENQK
229E        737  *GVADA*RM*M**GS*IG*IALG*L*----S*VS***SLAIQA*L*YVALQTD**Q****
SARS_CoV_2  922  LIANQFNSAIGKIQDS--------------LSSTASALGKLQDVVNQNAQALNTLVKQLS
229E        793  IL*AS**K*MTN*V*AFTGVNDAITQTSQA*QTV*T**N*I*****QGNS**H*TS**R
SARS_CoV_2  968  SNFGAISSVLNDILSRLDKVEAEVQIDRLITGRLQSLQTYVTQQLIRAAEIRASANLAAT
229E        853  Q**Q****SIQA*YD***TIQ*DQ*V********AA*NVF*SHT*TKYT*V***RQ**QQ
SARS_CoV_2 1028  KMSECVLGQSKRVDFCGKGYHLMSFPQSAPHGVVFLHVTYVPAQEKNFTTAPAICHDGKA
229E        913  *VN***KS****YG***N*T*IF*IVNA**E*L****TVLL*T*Y*DVEAWSGL*V**TN
SARS_CoV_2 1088  HF-PREG--VFVSNGTHWFVTQRNFYEPQIITTDNTFVSGNCDVVIGIVNNTVYDPLQPE
229E        973  GYVL*QPNLALYKE*NYYRI*S*IMF**R*P*MADFVQIE**N*TFVNISRSELQTIV**
SARS_CoV_2 1145  L---DSFKEELDKYFKNHTSPDVDLGDISGINASVVNIQK--------------EIDRLN
229E       1033  YIDVNKTLQ**SYKLP*Y*V**LVV---EQY*QTIL*LTSEISTLENKSAELNYTVQK*Q
SARS_CoV_2 1188  EVAKNLNESLIDLQELGKYEQYIKWPWYIWLGFIAGLIAIVMVTIMLCCMTSCCSC----
229E       1090  TLID*I*ST*V**KW*NRV*T*****WV**CISVV**FV*SMLLLC**S*G**GFFSCF
SARS_CoV_2 1244  ---LKGCCSCGSCCKFDEDDSEPVLKGVKLHYT
229E       1150  ASSIR***ESTKLPYY*         VE*I*IQ
```

Fig. 13

**A**

Tracer

anti-mouse ab

mouse anti-His ab

His-tag

RBD

Immobilized ACE2 receptor

**B**

| | 200 | 100 | 50 | 25 | ng overlay |
|---|---|---|---|---|---|
| ACE 2 receptor | + | + | + | + | |
| RBD | + | + | + | + | |
| mouse anti-His | + | + | + | + | |
| anti-mIgG₁ | + | + | + | + | |

**C**

Fig. 14

Fig. 14 cont.

Fig. 15

Fig. 16

Europäisches
Patentamt

European
Patent Office

Office européen
des brevets

## EUROPEAN SEARCH REPORT

Application Number

EP 20 18 0359

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/282154 A1 (FARZAN MICHAEL R [US] ET AL) 22 December 2005 (2005-12-22) * paragraph [0016] * ----- | 1-15 | INV. C07K14/705 C07K14/005 C12N9/48 C12Q1/70 G01N33/569 |
| X | HE YUXIAN ET AL: "Receptor-binding domain of severe acute respiratory syndrome coronavirus spike protein contains multiple conformation-dependent epitopes that induce highly potent neutralizing antibodies", THE JOURNAL OF IMMUNOLOGY, AMERICAN ASSOCIATION OF IMMUNOLOGISTS, US, vol. 174, no. 8, 1 April 2005 (2005-04-01) , pages 4908-4915, XP002439744, ISSN: 0022-1767 * page 4909, right-hand column, last paragraph; table 1 * ----- | 1-15 | |
| X | WO 2005/120565 A2 (NEW YORK BLOOD CT INC [US]; JIANG SHIBO [US] ET AL.) 22 December 2005 (2005-12-22) * page 22, last paragraph * * page 26, paragraph f * ----- | 1-15 | |
| X | WO 2014/045254 A2 (UNIV ERASMUS MEDICAL CT [NL]) 27 March 2014 (2014-03-27) * claim 30 * ----- | 1-15 | |
| | -/-- | | |

TECHNICAL FIELDS
SEARCHED (IPC)

C07K
C12Q
G01N
C12N

The present search report has been drawn up for all claims

| Place of search | Date of completion of the search | Examiner |
|---|---|---|
| Munich | 20 October 2020 | Griesinger, Irina |

EPO FORM 1503 03.82 (P04C01)

Europäisches
Patentamt

European
Patent Office

Office européen
des brevets

**EUROPEAN SEARCH REPORT**

Application Number

EP 20 18 0359

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HOFMANN H ET AL: "Susceptibility to SARS coronavirus S protein-driven infection correlates with expression of angiotensin converting enzyme 2 and infection can be blocked by soluble receptor", BIOCHEMICAL AND BIOPHYSICAL RESEARCH COMMUNICATIONS, ELSEVIER, AMSTERDAM, NL, vol. 319, no. 4, 9 July 2004 (2004-07-09), pages 1216-1221, XP004515246, ISSN: 0006-291X, DOI: 10.1016/J.BBRC.2004.05.114 * abstract * ----- | 1-15 | |

TECHNICAL FIELDS
SEARCHED    (IPC)

The present search report has been drawn up for all claims

| Place of search | Date of completion of the search | Examiner |
|---|---|---|
| Munich | 20 October 2020 | Griesinger, Irina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 20 18 0359

This annex lists the patent family members relating to the patent documents cited in the above-mentioned European search report.
The members are as contained in the European Patent Office EDP file on
The European Patent Office is in no way liable for these particulars which are merely given for the purpose of information.

20-10-2020

| Patent document cited in search report | | Publication date | Patent family member(s) | | Publication date |
|---|---|---|---|---|---|
| US 2005282154 | A1 | 22-12-2005 | US 2005282154 A1<br>WO 2005032487 A2 | | 22-12-2005<br>14-04-2005 |
| WO 2005120565 | A2 | 22-12-2005 | AU 2005251738 A1<br>CA 2569142 A1<br>EP 1773388 A2<br>WO 2005120565 A2 | | 22-12-2005<br>22-12-2005<br>18-04-2007<br>22-12-2005 |
| WO 2014045254 | A2 | 27-03-2014 | EP 2898067 A2<br>KR 20150064104 A<br>SG 11201502189U A<br>US 2015275183 A1<br>WO 2014045254 A2 | | 29-07-2015<br>10-06-2015<br>29-04-2015<br>01-10-2015<br>27-03-2014 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FU et al.** *J. Med. Virol.,* 2020
- **NIE et al.** *Emerg. Microbes Infect.,* 2020, vol. 9 (1), 680-686
- **TAN et al.** *Natureresearch,* 2020

- **ALWIS et al.** *EBioMedicine,* 2020, vol. 55, 102768
- **DOROFEEVA et al.** *Sci Rep.,* 2019, vol. 9, 15043
- **WATANABE et al.** *Science,* 2020